(19) **Europäisches Patentamt / European Patent Office / Office européen des brevets**

(11) **EP 3 070 171 B1**

(12) **EUROPEAN PATENT SPECIFICATION**

(45) Date of publication and mention
of the grant of the patent:
**13.06.2018 Bulletin 2018/24**

(51) Int Cl.:
*C12P 7/06* [(2006.01)]       *C12P 19/14* [(2006.01)]
*C12P 19/20* [(2006.01)]      *C12N 9/24* [(2006.01)]
*C12N 9/28* [(2006.01)]       *C12N 9/34* [(2006.01)]

(21) Application number: **16163786.3**

(22) Date of filing: **30.03.2011**

(54) **PROCESS FOR ENHANCING BY-PRODUCTS FROM FERMENTATION PROCESSES**

VERFAHREN ZUR STEIGERUNG DER NEBENPRODUCKTE VON FERMENTATIONSPROZESSEN

PROCEDE POUR AUGMENTER LES PRODUITS SECONDAIRES DE PROCEDES DE FERMENTATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2010 US 318787 P**

(43) Date of publication of application:
**21.09.2016 Bulletin 2016/38**

(62) Document number(s) of the earlier application(s) in
accordance with Art. 76 EPC:
**11715113.4 / 2 553 111**

(73) Proprietors:
• **Novozymes A/S**
  **2880 Bagsvaerd (DK)**
• **Novozymes North America, Inc.**
  **Franklinton, NC 27525 (US)**

(72) Inventors:
• **SAUNDERS, Jeremy**
  **Franklinton, North Carolina 27525 (US)**

• **JUMP, Joseph**
  **Franklinton, North Carolina 27525 (US)**
• **OLSEN, Hans Sejr**
  **2880 Bagsvaerd (DK)**
• **GUICHARD, Amanda**
  **Raleigh, North Carolina 27604 (US)**
• **KREEL, Nathaniel E.**
  **Franklinton, North Carolina 27525 (US)**
• **AKERMAN, Michael**
  **Franklinton, North Carolina 27525 (US)**
• **HJULMAND, Anne Glud**
  **deceased (DK)**

(74) Representative: **Shenker, Paul Dhan**
**Novozymes A/S**
**Patents**
**Krogshoejvej 36**
**2880 Bagsværd (DK)**

(56) References cited:
**WO-A1-02/38786**          **WO-A1-2007/056321**
**WO-A2-2004/031378**      **WO-A2-2004/080923**

SEQUENCE LISTING

<110> Saunders, Jeremy D.
Akerman, Michael
Jump, Joseph M.
Olsen, Hans Sejr
Guichard, Amanda
Kreel, Nathaniel E.
Hjulmand, Anne

<120> METHODS FOR ENHANCING BY PRODUCTS FROM FERMENTATION PROCESSES

<130> 11683-WO-PCT

<150> US 61/318,787
<151> 2010-03-30

<160> 32

<170> PatentIn version 3.5

<210> 1
<211> 326
<212> PRT
<213> Thielavia terrestris

<400> 1

```
Met Lys Ser Phe Thr Ile Ala Ala Leu Ala Ala Leu Trp Ala Gln Glu
1               5                   10                  15

Ala Ala Ala His Ala Thr Phe Gln Asp Leu Trp Ile Asp Gly Val Asp
            20                  25                  30

Tyr Gly Ser Gln Cys Val Arg Leu Pro Ala Ser Asn Ser Pro Val Thr
        35                  40                  45

Asn Val Ala Ser Asp Asp Ile Arg Cys Asn Val Gly Thr Ser Arg Pro
        50                  55                  60

Thr Val Lys Cys Pro Val Lys Ala Gly Ser Thr Val Thr Ile Glu Met
65                  70                  75                  80

His Gln Gln Pro Gly Asp Arg Ser Cys Ala Asn Glu Ala Ile Gly Gly
                85                  90                  95

Asp His Tyr Gly Pro Val Met Val Tyr Met Ser Lys Val Asp Asp Ala
            100                 105                 110

Val Thr Ala Asp Gly Ser Ser Gly Trp Phe Lys Val Phe Gln Asp Ser
            115                 120                 125

Trp Ala Lys Asn Pro Ser Gly Ser Thr Gly Asp Asp Asp Tyr Trp Gly
        130                 135                 140
```

```
Thr Lys Asp Leu Asn Ser Cys Cys Gly Lys Met Asn Val Lys Ile Pro
145                 150                 155                 160

Glu Asp Ile Glu Pro Gly Asp Tyr Leu Leu Arg Ala Glu Val Ile Ala
                165                 170                 175

Leu His Val Ala Ala Ser Ser Gly Gly Ala Gln Phe Tyr Met Ser Cys
                180                 185                 190

Tyr Gln Leu Thr Val Thr Gly Ser Gly Ser Ala Thr Pro Ser Thr Val
            195                 200                 205

Asn Phe Pro Gly Ala Tyr Ser Ala Ser Asp Pro Gly Ile Leu Ile Asn
        210                 215                 220

Ile His Ala Pro Met Ser Thr Tyr Val Val Pro Gly Pro Thr Val Tyr
225                 230                 235                 240

Ala Gly Gly Ser Thr Lys Ser Ala Gly Ser Ser Cys Ser Gly Cys Glu
            245                 250                 255

Ala Thr Cys Thr Val Gly Ser Gly Pro Ser Ala Thr Leu Thr Gln Pro
            260                 265                 270

Thr Ser Thr Ala Thr Ala Thr Ser Ala Pro Gly Gly Gly Gly Ser Gly
        275                 280                 285

Cys Thr Ala Ala Lys Tyr Gln Gln Cys Gly Gly Thr Gly Tyr Thr Gly
290                 295                 300

Cys Thr Thr Cys Ala Ser Gly Ser Thr Cys Ser Ala Val Ser Pro Pro
305                 310                 315                 320

Tyr Tyr Ser Gln Cys Leu
                325
```

<210> 2
<211> 239
<212> PRT
<213> Thielavia terrestris

<400> 2

```
Met Arg Phe Asp Ala Leu Ser Ala Leu Ala Leu Ala Pro Leu Val Ala
1               5                   10                  15

Gly His Gly Ala Val Thr Ser Tyr Ile Ile Gly Gly Lys Thr Tyr Pro
            20                  25                  30
```

```
Gly Tyr Glu Gly Phe Ser Pro Ala Ser Ser Pro Pro Thr Ile Gln Tyr
        35              40              45

Gln Trp Pro Asp Tyr Asn Pro Thr Leu Ser Val Thr Asp Pro Lys Met
        50              55              60

Arg Cys Asn Gly Gly Thr Ser Ala Glu Leu Ser Ala Pro Val Gln Ala
65              70              75              80

Gly Glu Asn Val Thr Ala Val Trp Lys Gln Trp Thr His Gln Gln Gly
                85              90              95

Pro Val Met Val Trp Met Phe Lys Cys Pro Gly Asp Phe Ser Ser Ser
            100             105             110

His Gly Asp Gly Lys Gly Trp Phe Lys Ile Asp Gln Leu Gly Leu Trp
        115             120             125

Gly Asn Asn Leu Asn Ser Asn Asn Trp Gly Thr Ala Ile Val Tyr Lys
    130             135             140

Thr Leu Gln Trp Ser Asn Pro Ile Pro Lys Asn Leu Ala Pro Gly Asn
145             150             155             160

Tyr Leu Ile Arg His Glu Leu Leu Ala Leu His Gln Ala Asn Thr Pro
            165             170             175

Gln Phe Tyr Ala Glu Cys Ala Gln Leu Val Val Ser Gly Ser Gly Ser
            180             185             190

Ala Leu Pro Pro Ser Asp Tyr Leu Tyr Ser Ile Pro Val Tyr Ala Pro
            195             200             205

Gln Asn Asp Pro Gly Ile Thr Val Asp Ile Tyr Asn Gly Gly Leu Thr
    210             215             220

Ser Tyr Thr Pro Pro Gly Gly Pro Val Trp Ser Gly Phe Glu Phe
225             230             235
```

<210> 3
<211> 258
<212> PRT
<213> Thielavia terrestris

<400> 3

```
Met Leu Leu Thr Ser Val Leu Gly Ser Ala Ala Leu Leu Ala Ser Gly
1               5               10              15
```

```
Ala Ala Ala His Gly Ala Val Thr Ser Tyr Ile Ile Ala Gly Lys Asn
        20                  25                  30


Tyr Pro Gly Tyr Gln Gly Phe Ser Pro Ala Asn Ser Pro Asn Val Ile
        35                  40                  45


Gln Trp Gln Trp His Asp Tyr Asn Pro Val Leu Ser Cys Ser Asp Ser
        50                  55                  60


Lys Leu Arg Cys Asn Gly Gly Thr Ser Ala Thr Leu Asn Ala Thr Ala
65                  70                  75                  80


Ala Pro Gly Asp Thr Ile Thr Ala Ile Trp Ala Gln Trp Thr His Ser
                85                  90                  95


Gln Gly Pro Ile Leu Val Trp Met Tyr Lys Cys Pro Gly Ser Phe Ser
                100                 105                 110


Ser Cys Asp Gly Ser Gly Ala Gly Trp Phe Lys Ile Asp Glu Ala Gly
        115                 120                 125


Phe His Gly Asp Gly Val Lys Val Phe Leu Asp Thr Glu Asn Pro Ser
        130                 135                 140


Gly Trp Asp Ile Ala Lys Leu Val Gly Gly Asn Lys Gln Trp Ser Ser
145                 150                 155                 160


Lys Val Pro Glu Gly Leu Ala Pro Gly Asn Tyr Leu Val Arg His Glu
                165                 170                 175


Leu Ile Ala Leu His Gln Ala Asn Asn Pro Gln Phe Tyr Pro Glu Cys
                180                 185                 190


Ala Gln Val Val Ile Thr Gly Ser Gly Thr Ala Gln Pro Asp Ala Ser
        195                 200                 205


Tyr Lys Ala Ala Ile Pro Gly Tyr Cys Asn Gln Asn Asp Pro Asn Ile
210                 215                 220


Lys Val Pro Ile Asn Asp His Ser Ile Pro Gln Thr Tyr Lys Ile Pro
225                 230                 235                 240


Gly Pro Pro Val Phe Lys Gly Thr Ala Ser Lys Lys Ala Arg Asp Phe
                245                 250                 255


Thr Ala
```

<210> 4
<211> 226
<212> PRT
<213> Thielavia terrestris

<400> 4

```
Met Leu Ala Asn Gly Ala Ile Val Phe Leu Ala Ala Ala Leu Gly Val
1               5                   10                  15

Ser Gly His Tyr Thr Trp Pro Arg Val Asn Asp Gly Ala Asp Trp Gln
            20                  25                  30

Gln Val Arg Lys Ala Asp Asn Trp Gln Asp Asn Gly Tyr Val Gly Asp
            35                  40                  45

Val Thr Ser Pro Gln Ile Arg Cys Phe Gln Ala Thr Pro Ser Pro Ala
        50                  55                  60

Pro Ser Val Leu Asn Thr Thr Ala Gly Ser Thr Val Thr Tyr Trp Ala
65                  70                  75                  80

Asn Pro Asp Val Tyr His Pro Gly Pro Val Gln Phe Tyr Met Ala Arg
                85                  90                  95

Val Pro Asp Gly Glu Asp Ile Asn Ser Trp Asn Gly Asp Gly Ala Val
            100                 105                 110

Trp Phe Lys Val Tyr Glu Asp His Pro Thr Phe Gly Ala Gln Leu Thr
            115                 120                 125

Trp Pro Ser Thr Gly Lys Ser Ser Phe Ala Val Pro Ile Pro Pro Cys
            130                 135                 140

Ile Lys Ser Gly Tyr Tyr Leu Leu Arg Ala Glu Gln Ile Gly Leu His
145                 150                 155                 160

Val Ala Gln Ser Val Gly Gly Ala Gln Phe Tyr Ile Ser Cys Ala Gln
                165                 170                 175

Leu Ser Val Thr Gly Gly Gly Ser Thr Glu Pro Pro Asn Lys Val Ala
            180                 185                 190

Phe Pro Gly Ala Tyr Ser Ala Thr Asp Pro Gly Ile Leu Ile Asn Ile
            195                 200                 205

Tyr Tyr Pro Val Pro Thr Ser Tyr Gln Asn Pro Gly Pro Ala Val Phe
            210                 215                 220
```

```
                          Ser Cys
                          225
```

<210> 5
<211> 304
<212> PRT
<213> Thielavia terrestris

<400> 5

```
Met Lys Gly Leu Phe Ser Ala Ala Ala Leu Ser Leu Ala Val Gly Gln
1               5                   10                  15

Ala Ser Ala His Tyr Ile Phe Gln Gln Leu Ser Ile Asn Gly Asn Gln
            20                  25                  30

Phe Pro Val Tyr Gln Tyr Ile Arg Lys Asn Thr Asn Tyr Asn Ser Pro
            35                  40                  45

Val Thr Asp Leu Thr Ser Asp Asp Leu Arg Cys Asn Val Gly Ala Gln
        50                  55                  60

Gly Ala Gly Thr Asp Thr Val Thr Val Lys Ala Gly Asp Gln Phe Thr
65                  70                  75                  80

Phe Thr Leu Asp Thr Pro Val Tyr His Gln Gly Pro Ile Ser Ile Tyr
                85                  90                  95

Met Ser Lys Ala Pro Gly Ala Ala Ser Asp Tyr Asp Gly Ser Gly Gly
            100                 105                 110

Trp Phe Lys Ile Lys Asp Trp Gly Pro Thr Phe Asn Ala Asp Gly Thr
            115                 120                 125

Ala Thr Trp Asp Met Ala Gly Ser Tyr Thr Tyr Asn Ile Pro Thr Cys
        130                 135                 140

Ile Pro Asp Gly Asp Tyr Leu Leu Arg Ile Gln Ser Leu Ala Ile His
145                 150                 155                 160

Asn Pro Trp Pro Ala Gly Ile Pro Gln Phe Tyr Ile Ser Cys Ala Gln
                165                 170                 175

Ile Thr Val Thr Gly Gly Gly Asn Gly Asn Pro Gly Pro Thr Ala Leu
                180                 185                 190

Ile Pro Gly Ala Phe Lys Asp Thr Asp Pro Gly Tyr Thr Val Asn Ile
                195                 200                 205
```

```
Tyr Thr Asn Phe His Asn Tyr Thr Val Pro Gly Pro Glu Val Phe Ser
    210                 215                 220

Cys Asn Gly Gly Gly Ser Asn Pro Pro Pro Val Ser Ser Ser Thr
225                 230                 235                 240

Pro Ala Thr Thr Thr Leu Val Thr Ser Thr Arg Thr Thr Ser Ser Thr
                245                 250                 255

Ser Ser Ala Ser Thr Pro Ala Ser Thr Gly Gly Cys Thr Val Ala Lys
            260                 265                 270

Trp Gly Gln Cys Gly Gly Asn Gly Tyr Thr Gly Cys Thr Thr Cys Ala
        275                 280                 285

Ala Gly Ser Thr Cys Ser Lys Gln Asn Asp Tyr Tyr Ser Gln Cys Leu
    290                 295                 300
```

<210> 6
<211> 317
<212> PRT
<213> Thielavia terrestris

<400> 6

```
Met Lys Gly Leu Ser Leu Leu Ala Ala Ala Ser Ala Ala Thr Ala His
1               5                   10                  15

Thr Ile Phe Val Gln Leu Glu Ser Gly Gly Thr Thr Tyr Pro Val Ser
            20                  25                  30

Tyr Gly Ile Arg Asp Pro Ser Tyr Asp Gly Pro Ile Thr Asp Val Thr
            35                  40                  45

Ser Asp Ser Leu Ala Cys Asn Gly Pro Pro Asn Pro Thr Thr Pro Ser
    50                  55                  60

Pro Tyr Ile Ile Asn Val Thr Ala Gly Thr Thr Val Ala Ala Ile Trp
65                  70                  75                  80

Arg His Thr Leu Thr Ser Gly Pro Asp Asp Val Met Asp Ala Ser His
            85                  90                  95

Lys Gly Pro Thr Leu Ala Tyr Leu Lys Lys Val Asp Asp Ala Leu Thr
            100                 105                 110

Asp Thr Gly Ile Gly Gly Gly Trp Phe Lys Ile Gln Glu Ala Gly Tyr
            115                 120                 125
```

```
Asp Asn Gly Asn Trp Ala Thr Ser Thr Val Ile Thr Asn Gly Gly Phe
    130                 135             140

Gln Tyr Ile Asp Ile Pro Ala Cys Ile Pro Asn Gly Gln Tyr Leu Leu
    145             150             155                 160

Arg Ala Glu Met Ile Ala Leu His Ala Ala Ser Thr Gln Gly Gly Ala
                165             170                 175

Gln Leu Tyr Met Glu Cys Ala Gln Ile Asn Val Val Gly Gly Ser Gly
            180             185                 190

Ser Ala Ser Pro Gln Thr Tyr Ser Ile Pro Gly Ile Tyr Gln Ala Thr
        195             200             205

Asp Pro Gly Leu Leu Ile Asn Ile Tyr Ser Met Thr Pro Ser Ser Gln
    210             215             220

Tyr Thr Ile Pro Gly Pro Pro Leu Phe Thr Cys Ser Gly Ser Gly Asn
225             230             235                 240

Asn Gly Gly Gly Ser Asn Pro Ser Gly Gly Gln Thr Thr Thr Ala Lys
            245             250                 255

Pro Thr Thr Thr Thr Ala Ala Thr Thr Thr Ser Ser Ala Ala Pro Thr
        260             265             270

Ser Ser Gln Gly Gly Ser Ser Gly Cys Thr Val Pro Gln Trp Gln Gln
        275             280             285

Cys Gly Gly Ile Ser Phe Thr Gly Cys Thr Thr Cys Ala Ala Gly Tyr
    290             295             300

Thr Cys Lys Tyr Leu Asn Asp Tyr Tyr Ser Gln Cys Gln
305             310             315
```

<210> 7
<211> 249
<212> PRT
<213> Thermoascus aurantiacus

<400> 7

```
Met Ser Phe Ser Lys Ile Ile Ala Thr Ala Gly Val Leu Ala Ser Ala
1               5               10                  15

Ser Leu Val Ala Gly His Gly Phe Val Gln Asn Ile Val Ile Asp Gly
            20              25                  30
```

46

```
        Lys Tyr Tyr Gly Gly Tyr Leu Val Asn Gln Tyr Pro Tyr Met Ser Asn
                35                  40                  45

        Pro Pro Glu Val Ile Ala Trp Ser Thr Thr Ala Thr Asp Leu Gly Phe
                50                  55                  60

        Val Asp Gly Thr Gly Tyr Gln Thr Pro Asp Ile Ile Cys His Arg Gly
        65                  70                  75                  80

        Ala Lys Pro Gly Ala Leu Thr Ala Pro Val Ser Pro Gly Gly Thr Val
                        85                  90                  95

        Glu Leu Gln Trp Thr Pro Trp Pro Asp Ser His His Gly Pro Val Ile
                    100                 105                 110

        Asn Tyr Leu Ala Pro Cys Asn Gly Asp Cys Ser Thr Val Asp Lys Thr
                    115                 120                 125

        Gln Leu Glu Phe Phe Lys Ile Ala Glu Ser Gly Leu Ile Asn Asp Asp
                    130                 135                 140

        Asn Pro Pro Gly Ile Trp Ala Ser Asp Asn Leu Ile Ala Ala Asn Asn
        145                 150                 155                 160

        Ser Trp Thr Val Thr Ile Pro Thr Thr Ile Ala Pro Gly Asn Tyr Val
                    165                 170                 175

        Leu Arg His Glu Ile Ile Ala Leu His Ser Ala Gln Asn Gln Asp Gly
                    180                 185                 190

        Ala Gln Asn Tyr Pro Gln Cys Ile Asn Leu Gln Val Thr Gly Gly Gly
                    195                 200                 205

        Ser Asp Asn Pro Ala Gly Thr Leu Gly Thr Ala Leu Tyr His Asp Thr
                210                 215                 220

        Asp Pro Gly Ile Leu Ile Asn Ile Tyr Gln Lys Leu Ser Ser Tyr Ile
        225                 230                 235                 240

        Ile Pro Gly Pro Pro Leu Tyr Thr Gly
                        245
```

<210> 8
<211> 249
<212> PRT
<213> Trichoderma reesei

<400> 8

47

```
Met Lys Ser Cys Ala Ile Leu Ala Ala Leu Gly Cys Leu Ala Gly Ser
1               5                   10              15

Val Leu Gly His Gly Gln Val Gln Asn Phe Thr Ile Asn Gly Gln Tyr
            20                  25                  30

Asn Gln Gly Phe Ile Leu Asp Tyr Tyr Gln Lys Gln Asn Thr Gly
        35                  40                  45

His Phe Pro Asn Val Ala Gly Trp Tyr Ala Glu Asp Leu Asp Leu Gly
        50                  55                  60

Phe Ile Ser Pro Asp Gln Tyr Thr Thr Pro Asp Ile Val Cys His Lys
65                  70                  75                  80

Asn Ala Ala Pro Gly Ala Ile Ser Ala Thr Ala Ala Ala Gly Ser Asn
                85                  90                  95

Ile Val Phe Gln Trp Gly Pro Gly Val Trp Pro His Pro Tyr Gly Pro
            100                 105                 110

Ile Val Thr Tyr Val Val Glu Cys Ser Gly Ser Cys Thr Thr Val Asn
            115                 120                 125

Lys Asn Asn Leu Arg Trp Val Lys Ile Gln Glu Ala Gly Ile Asn Tyr
    130                 135                 140

Asn Thr Gln Val Trp Ala Gln Gln Asp Leu Ile Asn Gln Gly Asn Lys
145                 150                 155                 160

Trp Thr Val Lys Ile Pro Ser Ser Leu Arg Pro Gly Asn Tyr Val Phe
            165                 170                 175

Arg His Glu Leu Leu Ala Ala His Gly Ala Ser Ser Ala Asn Gly Met
            180                 185                 190

Gln Asn Tyr Pro Gln Cys Val Asn Ile Ala Val Thr Gly Ser Gly Thr
            195                 200                 205

Lys Ala Leu Pro Ala Gly Thr Pro Ala Thr Gln Leu Tyr Lys Pro Thr
    210                 215                 220

Asp Pro Gly Ile Leu Phe Asn Pro Tyr Thr Thr Ile Thr Ser Tyr Thr
225                 230                 235                 240

Ile Pro Gly Pro Ala Leu Trp Gln Gly
```

245

<210> 9
<211> 232
<212> PRT
<213> Myceliophthora thermophila

<400> 9

```
Met Lys Phe Thr Ser Ser Leu Ala Val Leu Ala Ala Ala Gly Ala Gln
1                5                    10                   15

Ala His Tyr Thr Phe Pro Arg Ala Gly Thr Gly Gly Ser Leu Ser Gly
            20                   25                   30

Glu Trp Glu Val Val Arg Met Thr Glu Asn His Tyr Ser His Gly Pro
            35                   40                   45

Val Thr Asp Val Thr Ser Pro Glu Met Thr Cys Tyr Gln Ser Gly Val
    50                   55                   60

Gln Gly Ala Pro Gln Thr Val Gln Val Lys Ala Gly Ser Gln Phe Thr
65                   70                   75                   80

Phe Ser Val Asp Pro Ser Ile Gly His Pro Gly Pro Leu Gln Phe Tyr
                85                   90                   95

Met Ala Lys Val Pro Ser Gly Gln Thr Ala Ala Thr Phe Asp Gly Thr
            100                  105                  110

Gly Ala Val Trp Phe Lys Ile Tyr Gln Asp Gly Pro Asn Gly Leu Gly
            115                  120                  125

Thr Asp Ser Ile Thr Trp Pro Ser Ala Gly Lys Thr Glu Val Ser Val
    130                  135                  140

Thr Ile Pro Ser Cys Ile Asp Asp Gly Glu Tyr Leu Leu Arg Val Glu
145                  150                  155                  160

His Ile Ala Leu His Ser Ala Ser Ser Val Gly Gly Ala Gln Phe Tyr
                165                  170                  175

Ile Ala Cys Ala Gln Leu Ser Val Thr Gly Gly Ser Gly Thr Leu Asn
            180                  185                  190

Thr Gly Ser Leu Val Ser Leu Pro Gly Ala Tyr Lys Ala Thr Asp Pro
            195                  200                  205

Gly Ile Leu Phe Gln Leu Tyr Trp Pro Ile Pro Thr Glu Tyr Ile Asn
            210                  215                  220

Pro Gly Pro Ala Pro Val Ser Cys
225                  230
```

<210> 10

<211> 235
<212> PRT
<213> Myceliophthora thermophila

<400> 10

```
Met Lys Ala Leu Ser Leu Leu Ala Ala Ala Ser Ala Val Ser Ala His
1               5               10              15

Thr Ile Phe Val Gln Leu Glu Ala Asp Gly Thr Arg Tyr Pro Val Ser
            20              25              30

Tyr Gly Ile Arg Asp Pro Ser Tyr Asp Gly Pro Ile Thr Asp Val Thr
            35              40              45

Ser Asn Asp Val Ala Cys Asn Gly Gly Pro Asn Pro Thr Thr Pro Ser
    50              55              60

Ser Asp Val Ile Thr Val Thr Ala Gly Thr Thr Val Lys Ala Ile Trp
65              70              75              80

Arg His Thr Leu Gln Ser Gly Pro Asp Asp Val Met Asp Ala Ser His
            85              90              95

Lys Gly Pro Thr Leu Ala Tyr Leu Lys Lys Val Gly Asp Ala Thr Lys
            100             105             110

Asp Ser Gly Val Gly Gly Gly Trp Phe Lys Ile Gln Glu Asp Gly Tyr
    115             120             125

Asn Asn Gly Gln Trp Gly Thr Ser Thr Val Ile Ser Asn Gly Gly Glu
    130             135             140

His Tyr Ile Asp Ile Pro Ala Cys Ile Pro Glu Gly Gln Tyr Leu Leu
145             150             155             160

Arg Ala Glu Met Ile Ala Leu His Ala Ala Gly Ser Pro Gly Gly Ala
            165             170             175

Gln Leu Tyr Met Glu Cys Ala Gln Ile Asn Ile Val Gly Gly Ser Gly
            180             185             190

Ser Val Pro Ser Ser Thr Val Ser Phe Pro Gly Ala Tyr Ser Pro Asn
```

```
                195                    200                      205


        Asp Pro Gly Leu Leu Ile Asn Ile Tyr Ser Met Ser Pro Ser Ser Ser
            210               215               220


        Tyr Thr Ile Pro Gly Pro Pro Val Phe Lys Cys
        225               230               235
```

<210> 11
<211> 323
<212> PRT
<213> Myceliophthora thermophila

<400> 11

```
Met Lys Ser Phe Ala Leu Thr Thr Leu Ala Ala Leu Ala Gly Asn Ala
1               5               10              15

Ala Ala His Ala Thr Phe Gln Ala Leu Trp Val Asp Gly Val Asp Tyr
        20              25              30

Gly Ala Gln Cys Ala Arg Leu Pro Ala Ser Asn Ser Pro Val Thr Asp
        35              40              45

Val Thr Ser Asn Ala Ile Arg Cys Asn Ala Asn Pro Ser Pro Ala Arg
    50              55              60

Gly Lys Cys Pro Val Lys Ala Gly Ser Thr Val Thr Val Glu Met His
65              70              75              80

Gln Gln Pro Gly Asp Arg Ser Cys Ser Ser Glu Ala Ile Gly Gly Ala
            85              90              95

His Tyr Gly Pro Val Met Val Tyr Met Ser Lys Val Ser Asp Ala Ala
            100             105             110

Ser Ala Asp Gly Ser Ser Gly Trp Phe Lys Val Phe Glu Asp Gly Trp
        115             120             125

Ala Lys Asn Pro Ser Gly Gly Ser Gly Asp Asp Asp Tyr Trp Gly Thr
    130             135             140

Lys Asp Leu Asn Ser Cys Cys Gly Lys Met Asn Val Lys Ile Pro Ala
145             150             155             160

Asp Leu Pro Ser Gly Asp Tyr Leu Leu Arg Ala Glu Ala Leu Ala Leu
            165             170             175

His Thr Ala Gly Ser Ala Gly Gly Ala Gln Phe Tyr Met Thr Cys Tyr
```

```
                 180                      185                      190


     Gln Leu Thr Val Thr Gly Ser Gly Ser Ala Ser Pro Pro Thr Val Ser
             195                 200                 205


     Phe Pro Gly Ala Tyr Lys Ala Thr Asp Pro Gly Ile Leu Val Asn Ile
         210                 215                 220


     His Ala Pro Leu Ser Gly Tyr Thr Val Pro Gly Pro Ala Val Tyr Ser
     225                 230                 235                 240


     Gly Gly Ser Thr Lys Lys Ala Gly Ser Ala Cys Thr Gly Cys Glu Ser
                 245                 250                 255


     Thr Cys Ala Val Gly Ser Gly Pro Thr Ala Thr Val Ser Gln Ser Pro
                 260                 265                 270


     Gly Ser Thr Ala Thr Ser Ala Pro Gly Gly Gly Gly Gly Cys Thr Val
             275                 280                 285


     Gln Lys Tyr Gln Gln Cys Gly Gly Glu Gly Tyr Thr Gly Cys Thr Asn
         290                 295                 300


     Cys Ala Ser Gly Ser Thr Cys Ser Ala Val Ser Pro Pro Tyr Tyr Ser
     305                 310                 315                 320


     Gln Cys Val
```

<210> 12
<211> 310
<212> PRT
<213> Myceliophthora thermophila

<400> 12

```
Met Lys Pro Phe Ser Leu Val Ala Leu Ala Thr Ala Val Ser Gly His
1               5                   10                  15

Ala Ile Phe Gln Arg Val Ser Val Asn Gly Gln Asp Gln Gly Gln Leu
            20                  25                  30

Lys Gly Val Arg Ala Pro Ser Ser Asn Ser Pro Ile Gln Asn Val Asn
            35                  40                  45

Asp Ala Asn Met Ala Cys Asn Ala Asn Ile Val Tyr His Asp Ser Thr
            50                  55                  60

Ile Ile Lys Val Pro Ala Gly Ala Arg Val Gly Ala Trp Trp Gln His
```

```
                65                      70                      75                      80


                Val Ile Gly Gly Pro Gln Gly Ala Asn Asp Pro Asp Asn Pro Ile Ala
                                85                      90                      95


                Ala Ser His Lys Gly Pro Ile Gln Val Tyr Leu Ala Lys Val Asp Asn
                            100                     105                     110


                Ala Ala Thr Ala Ser Pro Ser Gly Leu Arg Trp Phe Lys Val Ala Glu
                            115                     120                     125


                Arg Gly Leu Asn Asn Gly Val Trp Ala Val Asp Glu Leu Ile Ala Asn
                            130                     135                     140


                Asn Gly Trp His Tyr Phe Asp Leu Pro Ser Cys Val Ala Pro Gly Gln
                145                     150                     155                     160


                Tyr Leu Met Arg Val Glu Leu Leu Ala Leu His Ser Ala Ser Ser Pro
                                165                     170                     175


                Gly Gly Ala Gln Phe Tyr Met Gly Cys Ala Gln Ile Glu Val Thr Gly
                            180                     185                     190


                Ser Gly Thr Asn Ser Gly Ser Asp Phe Val Ser Phe Pro Gly Ala Tyr
                            195                     200                     205


                Ser Ala Asn Asp Pro Gly Ile Leu Leu Ser Ile Tyr Asp Ser Ser Gly
                            210                     215                     220


                Lys Pro Thr Asn Gly Gly Arg Ser Tyr Pro Ile Pro Gly Pro Arg Pro
                225                     230                     235                     240


                Ile Ser Cys Ser Gly Ser Gly Asp Gly Gly Asn Asn Gly Gly Gly Gly
                                245                     250                     255


                Asp Asp Asn Asn Asn Asn Asn Gly Gly Gly Asn Asn Gly Gly Gly Gly
                            260                     265                     270


                Gly Gly Ser Val Pro Leu Tyr Gly Gln Cys Gly Gly Ile Gly Tyr Thr
                            275                     280                     285


                Gly Pro Thr Thr Cys Ala Gln Gly Thr Cys Lys Val Ser Asn Glu Tyr
                            290                     295                     300


                Tyr Ser Gln Cys Leu Pro
                305                     310
```

<210> 13
<211> 246
<212> PRT
<213> Myceliophthora thermophila

<400> 13

```
Met Lys Leu Ser Leu Phe Ser Val Leu Ala Thr Ala Leu Thr Val Glu
1               5                   10                  15

Gly His Ala Ile Phe Gln Lys Val Ser Val Asn Gly Ala Asp Gln Gly
            20                  25                  30

Ser Leu Thr Gly Leu Arg Ala Pro Asn Asn Asn Asn Pro Val Gln Asp
            35                  40                  45

Val Asn Ser Gln Asp Met Ile Cys Gly Gln Ser Gly Ser Thr Ser Asn
            50                  55                  60

Thr Ile Ile Glu Val Lys Ala Gly Asp Arg Ile Gly Ala Trp Tyr Gln
65                  70                  75                  80

His Val Ile Gly Gly Ala Gln Phe Pro Asn Asp Pro Asp Asn Pro Ile
                85                  90                  95

Ala Lys Ser His Lys Gly Pro Val Met Ala Tyr Leu Ala Lys Val Asp
            100                 105                 110

Asn Ala Ala Thr Ala Ser Lys Thr Gly Leu Lys Trp Phe Lys Ile Trp
            115                 120                 125

Glu Asp Thr Phe Asn Pro Ser Thr Lys Thr Trp Gly Val Asp Asn Leu
            130                 135                 140

Ile Asn Asn Asn Gly Trp Val Tyr Phe Asn Leu Pro Gln Cys Ile Ala
145                 150                 155                 160

Asp Gly Asn Tyr Leu Leu Arg Val Glu Val Leu Ala Leu His Ser Ala
                165                 170                 175

Tyr Ser Gln Gly Gln Ala Gln Phe Tyr Gln Ser Cys Ala Gln Ile Asn
            180                 185                 190

Val Ser Gly Gly Gly Ser Phe Thr Pro Pro Ser Thr Val Ser Phe Pro
            195                 200                 205

Gly Ala Tyr Ser Ala Ser Asp Pro Gly Ile Leu Ile Asn Ile Tyr Gly
            210                 215                 220
```

```
        Ala Thr Gly Gln Pro Asp Asn Asn Gly Gln Pro Tyr Thr Ala Pro Gly
        225             230             235             240


        Pro Ala Pro Ile Ser Cys
                        245
```

<210> 14
<211> 354
<212> PRT
<213> Thermoascus aurantiacus

<400> 14

```
        Met Ser Phe Ser Lys Ile Ala Ala Ile Thr Gly Ala Ile Thr Tyr Ala
        1               5               10              15


        Ser Leu Ala Ala Ala His Gly Tyr Val Thr Gly Ile Val Ala Asp Gly
                        20              25              30


        Thr Tyr Tyr Gly Gly Tyr Ile Val Thr Gln Tyr Pro Tyr Met Ser Thr
                        35              40              45


        Pro Pro Asp Val Ile Ala Trp Ser Thr Lys Ala Thr Asp Leu Gly Phe
            50              55              60


        Val Asp Pro Ser Ser Tyr Ala Ser Ser Asp Ile Ile Cys His Lys Gly
        65              70              75              80


        Ala Glu Pro Gly Ala Leu Ser Ala Lys Val Ala Ala Gly Gly Thr Val
                        85              90              95


        Glu Leu Gln Trp Thr Asp Trp Pro Glu Ser His Lys Gly Pro Val Ile
                        100             105             110


        Asp Tyr Leu Ala Ala Cys Asn Gly Asp Cys Ser Thr Val Asp Lys Thr
                115             120             125


        Lys Leu Glu Phe Phe Lys Ile Asp Glu Ser Gly Leu Ile Asp Gly Ser
            130             135             140


        Ser Ala Pro Gly Thr Trp Ala Ser Asp Asn Leu Ile Ala Asn Asn Asn
        145             150             155             160


        Ser Trp Thr Val Thr Ile Pro Ser Thr Ile Ala Pro Gly Asn Tyr Val
                        165             170             175


        Leu Arg His Glu Ile Ile Ala Leu His Ser Ala Gly Asn Thr Asn Gly
                        180             185             190
```

```
Ala Gln Asn Tyr Pro Gln Cys Ile Asn Leu Glu Val Thr Gly Ser Gly
        195                 200                 205

Thr Asp Thr Pro Ala Gly Thr Leu Gly Thr Glu Leu Tyr Lys Ala Thr
        210                 215                 220

Asp Pro Gly Ile Leu Val Asn Ile Tyr Gln Thr Leu Thr Ser Tyr Asp
225                 230                 235                 240

Ile Pro Gly Pro Ala Leu Tyr Thr Gly Gly Ser Ser Gly Ser Ser Gly
                245                 250                 255

Ser Ser Asn Thr Ala Lys Ala Thr Thr Ser Thr Ala Ser Ser Ser Ile
        260                 265                 270

Val Thr Pro Thr Pro Val Asn Asn Pro Thr Val Thr Gln Thr Ala Val
        275                 280                 285

Val Asp Val Thr Gln Thr Val Ser Gln Asn Ala Ala Val Ala Thr Thr
        290                 295                 300

Thr Pro Ala Ser Thr Ala Val Ala Thr Ala Val Pro Thr Gly Thr Thr
305                 310                 315                 320

Phe Ser Phe Asp Ser Met Thr Ser Asp Glu Phe Val Ser Leu Met Arg
                325                 330                 335

Ala Thr Val Asn Trp Leu Leu Ser Asn Lys Lys His Ala Arg Asp Leu
        340                 345                 350

Ser Tyr
```

<210> 15
<211> 250
<212> PRT
<213> Aspergillus fumigatus

<400> 15

```
Met Thr Leu Ser Lys Ile Thr Ser Ile Ala Gly Leu Leu Ala Ser Ala
1               5                   10                  15

Ser Leu Val Ala Gly His Gly Phe Val Ser Gly Ile Val Ala Asp Gly
        20                  25                  30

Lys Tyr Tyr Gly Gly Tyr Leu Val Asn Gln Tyr Pro Tyr Met Ser Asn
        35                  40                  45
```

```
Pro Pro Asp Thr Ile Ala Trp Ser Thr Thr Ala Thr Asp Leu Gly Phe
    50                  55                  60

Val Asp Gly Thr Gly Tyr Gln Ser Pro Asp Ile Ile Cys His Arg Asp
65                  70                  75                  80

Ala Lys Asn Gly Lys Leu Thr Ala Thr Val Ala Ala Gly Ser Gln Ile
                85                  90                  95

Glu Phe Gln Trp Thr Thr Trp Pro Glu Ser His His Gly Pro Leu Ile
            100                 105                 110

Thr Tyr Leu Ala Pro Cys Asn Gly Asp Cys Ala Thr Val Asp Lys Thr
        115                 120                 125

Thr Leu Lys Phe Val Lys Ile Ala Ala Gln Gly Leu Ile Asp Gly Ser
    130                 135                 140

Asn Pro Pro Gly Val Trp Ala Asp Asp Glu Met Ile Ala Asn Asn Asn
145                 150                 155                 160

Thr Ala Thr Val Thr Ile Pro Ala Ser Tyr Ala Pro Gly Asn Tyr Val
                165                 170                 175

Leu Arg His Glu Ile Ile Ala Leu His Ser Ala Gly Asn Leu Asn Gly
            180                 185                 190

Ala Gln Asn Tyr Pro Gln Cys Phe Asn Ile Gln Ile Thr Gly Gly Gly
        195                 200                 205

Ser Ala Gln Gly Ser Gly Thr Ala Gly Thr Ser Leu Tyr Lys Asn Thr
    210                 215                 220

Asp Pro Gly Ile Lys Phe Asp Ile Tyr Ser Asp Leu Ser Gly Gly Tyr
225                 230                 235                 240

Pro Ile Pro Gly Pro Ala Leu Phe Asn Ala
                245                 250
```

<210> 16
<211> 322
<212> PRT
<213> Penicillium pinophilum

<400> 16

```
Met Pro Ser Thr Lys Val Ala Ala Leu Ser Ala Val Leu Ala Leu Ala
1               5                   10                  15
```

```
Ser Thr Val Ala Gly His Gly Phe Val Gln Asn Ile Val Ile Asp Gly
            20              25              30

Lys Ser Tyr Ser Gly Tyr Leu Val Asn Gln Phe Pro Tyr Glu Ser Asn
            35              40              45

Pro Pro Ala Val Ile Gly Trp Ala Thr Thr Ala Thr Asp Leu Gly Phe
        50              55              60

Val Ala Pro Ser Glu Tyr Thr Asn Ala Asp Ile Ile Cys His Lys Asn
65              70              75              80

Ala Thr Pro Gly Ala Leu Ser Ala Pro Val Ala Ala Gly Gly Thr Val
            85              90              95

Glu Leu Gln Trp Thr Thr Trp Pro Asp Ser His His Gly Pro Val Ile
            100             105             110

Ser Tyr Leu Ala Asn Cys Asn Gly Asn Cys Ser Thr Val Asp Lys Thr
            115             120             125

Lys Leu Asp Phe Val Lys Ile Asp Gln Gly Gly Leu Ile Asp Asp Thr
            130             135             140

Thr Pro Pro Gly Thr Trp Ala Ser Asp Lys Leu Ile Ala Ala Asn Asn
145             150             155             160

Ser Trp Thr Val Thr Ile Pro Ser Thr Ile Ala Pro Gly Asn Tyr Val
                165             170             175

Leu Arg His Glu Ile Ile Ala Leu His Ser Ala Gly Asn Ala Asp Gly
            180             185             190

Ala Gln Asn Tyr Pro Gln Cys Ile Asn Leu Glu Ile Thr Gly Ser Gly
            195             200             205

Thr Ala Ala Pro Ser Gly Thr Ala Gly Glu Lys Leu Tyr Thr Ser Thr
    210             215             220

Asp Pro Gly Ile Leu Val Asn Ile Tyr Gln Ser Leu Ser Thr Tyr Val
225             230             235             240

Ile Pro Gly Pro Thr Leu Trp Ser Gly Ala Ala Asn Gly Ala Val Ala
            245             250             255

Thr Gly Ser Ala Thr Ala Val Ala Thr Thr Ala Thr Ala Ser Ala Thr
            260             265             270
```

61

```
Ala Thr Pro Thr Thr Leu Val Thr Ser Val Ala Pro Ala Ser Ser Thr
        275             280             285

Phe Ala Thr Ala Val Val Thr Thr Val Ala Pro Ala Val Thr Asp Val
    290             295             300

Val Thr Val Thr Asp Val Val Thr Val Thr Thr Val Ile Thr Thr Thr
305             310             315             320

Val Leu
```

<210> 17
<211> 444
<212> PRT
<213> Thermoascus sp.

<400> 17

```
Met Leu Ser Phe Ala Ser Ala Lys Ser Ala Val Leu Thr Thr Leu Leu
1               5               10              15

Leu Leu Gly Ser Ala Gln Ala His Thr Leu Met Thr Thr Leu Phe Val
        20              25              30

Asp Gly Val Asn Gln Gly Asp Gly Val Cys Ile Arg Met Asn Asn Asn
        35              40              45

Gly Ser Thr Ala Asn Thr Tyr Ile Gln Pro Val Thr Ser Lys Asp Ile
        50              55              60

Ala Cys Gly Ile Gln Gly Glu Ile Gly Ala Ala Arg Val Cys Pro Ala
65              70              75              80

Lys Ala Ser Ser Thr Leu Thr Phe Gln Phe Arg Glu Gln Pro Ser Asn
            85              90              95

Pro Asn Ser Ala Pro Leu Asp Pro Ser His Lys Gly Pro Ala Ala Val
            100             105             110

Tyr Leu Lys Lys Val Asp Ser Ala Ile Ala Ser Asn Asn Ala Ala Gly
        115             120             125

Asp Gly Trp Phe Lys Ile Trp Glu Ser Val Tyr Asp Glu Ser Thr Gly
        130             135             140

Lys Trp Gly Thr Thr Lys Met Ile Glu Asn Asn Gly His Ile Ser Val
145             150             155             160
```

```
Lys Val Pro Asp Asp Ile Glu Gly Gly Tyr Tyr Leu Ala Arg Thr Glu
            165                 170                 175

Leu Leu Ala Leu His Ala Ala Asn Glu Gly Asp Pro Gln Phe Tyr Val
            180                 185                 190

Gly Cys Ala Gln Leu Phe Ile Asp Ser Ala Gly Thr Ala Lys Pro Pro
            195                 200                 205

Thr Val Ser Ile Gly Glu Gly Thr Tyr Asp Leu Ser Met Pro Ala Met
    210                 215                 220

Thr Tyr Asn Ile Tyr Gln Thr Pro Leu Ala Leu Pro Tyr Pro Met Tyr
225                 230                 235                 240

Gly Pro Pro Val Tyr Thr Pro Gly Ser Gly Ser Gly Ser Gly Ser Gly
            245                 250                 255

Ser Gly Ser Ala Ser Ala Thr Arg Ser Ser Ala Ile Pro Thr Ala Thr
            260                 265                 270

Ala Val Thr Asp Cys Ser Ser Glu Glu Asp Arg Glu Asp Ser Val Met
    275                 280                 285

Ala Thr Gly Val Pro Val Ala Arg Ser Thr Leu Arg Thr Trp Val Asp
    290                 295                 300

Arg Leu Ser Trp His Gly Lys Ala Arg Glu Asn Val Lys Pro Ala Ala
305                 310                 315                 320

Arg Arg Ser Ala Leu Val Gln Thr Glu Gly Leu Lys Pro Glu Gly Cys
            325                 330                 335

Ile Phe Val Asn Gly Asn Trp Cys Gly Phe Glu Val Pro Asp Tyr Asn
            340                 345                 350

Asp Ala Glu Ser Cys Trp Ala Ala Ser Asp Asn Cys Trp Lys Gln Ser
            355                 360                 365

Asp Ser Cys Trp Asn Gln Thr Gln Pro Thr Gly Tyr Asn Asn Cys Gln
    370                 375                 380

Ile Trp Gln Asp Gln Lys Cys Lys Pro Ile Gln Asp Ser Cys Ser Gln
385                 390                 395                 400

Ser Asn Pro Thr Gly Pro Pro Asn Lys Gly Lys Asp Ile Thr Pro Thr
            405                 410                 415
```

```
        Trp Pro Pro Leu Glu Gly Ser Met Lys Thr Phe Thr Lys Arg Thr Val
                420                     425                 430

        Ser Tyr Arg Asp Trp Ile Met Lys Arg Lys Gly Ala
                435             440
```

<210> 18
<211> 253
<212> PRT
<213> Penicillium sp.

<400> 18

```
        Met Leu Ser Ser Thr Thr Arg Thr Leu Ala Phe Thr Gly Leu Ala Gly
        1               5                   10                  15

        Leu Leu Ser Ala Pro Leu Val Lys Ala His Gly Phe Val Gln Gly Ile
                20                  25                  30

        Val Ile Gly Asp Gln Phe Tyr Ser Gly Tyr Ile Val Asn Ser Phe Pro
                35                  40                  45

        Tyr Glu Ser Asn Pro Pro Pro Val Ile Gly Trp Ala Thr Thr Ala Thr
                50                  55                  60

        Asp Leu Gly Phe Val Asp Gly Thr Gly Tyr Gln Gly Pro Asp Ile Ile
        65                  70                  75                  80

        Cys His Arg Asn Ala Thr Pro Ala Pro Leu Thr Ala Pro Val Ala Ala
                        85                  90                  95

        Gly Gly Thr Val Glu Leu Gln Trp Thr Pro Trp Pro Asp Ser His His
                100                 105                 110

        Gly Pro Val Ile Thr Tyr Leu Ala Pro Cys Asn Gly Asn Cys Ser Thr
                115                 120                 125

        Val Asp Lys Thr Thr Leu Glu Phe Phe Lys Ile Asp Gln Gln Gly Leu
                130                 135                 140

        Ile Asp Asp Thr Ser Pro Pro Gly Thr Trp Ala Ser Asp Asn Leu Ile
        145                 150                 155                 160

        Ala Asn Asn Asn Ser Trp Thr Val Thr Ile Pro Asn Ser Val Ala Pro
                        165                 170                 175

        Gly Asn Tyr Val Leu Arg His Glu Ile Ile Ala Leu His Ser Ala Asn
                180                 185                 190
```

| Asn | Lys | Asp | Gly | Ala | Gln | Asn | Tyr | Pro | Gln | Cys | Ile | Asn | Ile | Glu | Val |
|     |     | 195 |     |     |     |     | 200 |     |     |     |     | 205 |     |     |     |

| Thr | Gly | Gly | Gly | Ser | Asp | Ala | Pro | Glu | Gly | Thr | Leu | Gly | Glu | Asp | Leu |
|     | 210 |     |     |     |     | 215 |     |     |     |     | 220 |     |     |     |     |

| Tyr | His | Asp | Thr | Asp | Pro | Gly | Ile | Leu | Val | Asp | Ile | Tyr | Glu | Pro | Ile |
| 225 |     |     |     |     | 230 |     |     |     |     | 235 |     |     |     |     | 240 |

| Ala | Thr | Tyr | Thr | Ile | Pro | Gly | Pro | Pro | Glu | Pro | Thr | Phe |
|     |     |     |     | 245 |     |     |     |     | 250 |     |     |     |

<210> 19
<211> 223
<212> PRT
<213> Thielavia terrestris

<400> 19

| Met | Lys | Leu | Ser | Ser | Gln | Leu | Ala | Ala | Leu | Thr | Leu | Ala | Ala | Ala | Ser |
| 1   |     |     |     | 5   |     |     |     |     | 10  |     |     |     |     | 15  |     |

| Val | Ser | Gly | His | Tyr | Ile | Phe | Glu | Gln | Ile | Ala | His | Gly | Gly | Thr | Lys |
|     |     |     | 20  |     |     |     | 25  |     |     |     |     | 30  |     |     |     |

| Phe | Pro | Pro | Tyr | Glu | Tyr | Ile | Arg | Arg | Asn | Thr | Asn | Tyr | Asn | Ser | Pro |
|     |     | 35  |     |     |     | 40  |     |     |     |     | 45  |     |     |     |     |

| Val | Thr | Ser | Leu | Ser | Ser | Asn | Asp | Leu | Arg | Cys | Asn | Val | Gly | Gly | Glu |
|     | 50  |     |     |     | 55  |     |     |     |     | 60  |     |     |     |     |     |

| Thr | Ala | Gly | Asn | Thr | Thr | Val | Leu | Asp | Val | Lys | Ala | Gly | Asp | Ser | Phe |
| 65  |     |     |     | 70  |     |     |     |     | 75  |     |     |     |     | 80  |     |

| Thr | Phe | Tyr | Ser | Asp | Val | Ala | Val | Tyr | His | Gln | Gly | Pro | Ile | Ser | Leu |
|     |     |     | 85  |     |     |     | 90  |     |     |     |     | 95  |     |     |     |

| Tyr | Met | Ser | Lys | Ala | Pro | Gly | Ser | Val | Val | Asp | Tyr | Asp | Gly | Ser | Gly |
|     |     | 100 |     |     |     | 105 |     |     |     |     | 110 |     |     |     |     |

| Asp | Trp | Phe | Lys | Ile | His | Asp | Trp | Gly | Pro | Thr | Phe | Ser | Asn | Gly | Gln |
|     |     | 115 |     |     |     | 120 |     |     |     |     | 125 |     |     |     |     |

| Ala | Ser | Trp | Pro | Leu | Arg | Asp | Asn | Tyr | Gln | Tyr | Asn | Ile | Pro | Thr | Cys |
|     | 130 |     |     |     |     | 135 |     |     |     |     | 140 |     |     |     |     |

| Ile | Pro | Asn | Gly | Glu | Tyr | Leu | Leu | Arg | Ile | Gln | Ser | Leu | Ala | Ile | His |
| 145 |     |     |     |     | 150 |     |     |     |     | 155 |     |     |     |     | 160 |

```
Asn Pro Gly Ala Thr Pro Gln Phe Tyr Ile Ser Cys Ala Gln Val Arg
            165               170               175

Val Ser Gly Gly Gly Ser Ala Ser Pro Ser Pro Thr Ala Lys Ile Pro
            180               185               190

Gly Ala Phe Lys Ala Thr Asp Pro Gly Tyr Thr Ala Asn Ile Tyr Asn
            195               200               205

Asn Phe His Ser Tyr Thr Val Pro Gly Pro Ala Val Phe Gln Cys
    210               215               220
```

<210> 20
<211> 246
<212> PRT
<213> Thielavia terrestris

<400> 20

```
Met Lys Phe Ser Leu Val Ser Leu Leu Ala Tyr Gly Leu Ser Val Glu
1               5               10              15

Ala His Ser Ile Phe Gln Arg Val Ser Val Asn Gly Gln Asp Gln Gly
            20              25              30

Leu Leu Thr Gly Leu Arg Ala Pro Ser Asn Asn Asn Pro Val Gln Asp
            35              40              45

Val Asn Ser Gln Asn Met Ile Cys Gly Gln Ser Gly Ser Lys Ser Gln
    50              55              60

Thr Val Ile Asn Val Lys Ala Gly Asp Arg Ile Gly Ser Leu Trp Gln
65              70              75              80

His Val Ile Gly Gly Ala Gln Phe Ser Gly Asp Pro Asp Asn Pro Ile
            85              90              95

Ala His Ser His Lys Gly Pro Val Met Ala Tyr Leu Ala Lys Val Asp
            100             105             110

Asn Ala Ala Ser Ala Ser Gln Thr Gly Leu Lys Trp Phe Lys Ile Trp
            115             120             125

Gln Asp Gly Phe Asp Thr Ser Ser Lys Thr Trp Gly Val Asp Asn Leu
    130             135             140

Ile Lys Asn Asn Gly Trp Val Tyr Phe His Leu Pro Gln Cys Leu Ala
145             150             155             160
```

```
Pro Gly Gln Tyr Leu Leu Arg Val Glu Val Leu Ala Leu His Ser Ala
            165             170             175

Tyr Gln Gln Gly Gln Ala Gln Phe Tyr Gln Ser Cys Ala Gln Ile Asn
            180             185             190

Val Ser Gly Ser Gly Ser Phe Ser Pro Ser Gln Thr Val Ser Ile Pro
            195             200             205

Gly Val Tyr Ser Ala Thr Asp Pro Ser Ile Leu Ile Asn Ile Tyr Gly
    210             215             220

Ser Thr Gly Gln Pro Asp Asn Gly Gly Lys Ala Tyr Asn Pro Pro Gly
225             230             235             240

Pro Ala Pro Ile Ser Cys
            245
```

<210> 21
<211> 334
<212> PRT
<213> Thielavia terrestris

<400> 21

```
Met Arg Thr Thr Phe Ala Ala Ala Leu Ala Ala Phe Ala Ala Gln Glu
1               5               10              15

Val Ala Gly His Ala Ile Phe Gln Gln Leu Trp His Gly Ser Ser Cys
            20              25              30

Val Arg Met Pro Leu Ser Asn Ser Pro Val Thr Asn Val Gly Ser Arg
            35              40              45

Asp Met Ile Cys Asn Ala Gly Thr Arg Pro Val Ser Gly Lys Cys Pro
    50              55              60

Val Lys Ala Gly Gly Thr Val Thr Val Glu Met His Gln Gln Pro Gly
65              70              75              80

Asp Arg Ser Cys Asn Asn Glu Ala Ile Gly Gly Ala His Trp Gly Pro
            85              90              95

Val Gln Val Tyr Leu Ser Lys Val Glu Asp Ala Ser Thr Ala Asp Gly
            100             105             110

Ser Thr Gly Trp Phe Lys Ile Phe Ala Asp Thr Trp Ser Lys Lys Ala
            115             120             125
```

```
Gly Ser Ser Val Gly Asp Asp Asp Asn Trp Gly Thr Arg Asp Leu Asn
    130             135             140

Ala Cys Cys Gly Lys Met Gln Val Lys Ile Pro Ala Asp Ile Pro Ser
    145             150             155             160

Gly Asp Tyr Leu Leu Arg Ala Glu Ala Leu Ala Leu His Thr Ala Gly
                165             170             175

Gln Val Gly Gly Ala Gln Phe Tyr Met Ser Cys Tyr Gln Ile Thr Val
            180             185             190

Ser Gly Gly Gly Ser Ala Ser Pro Ala Thr Val Lys Phe Pro Gly Ala
            195             200             205

Tyr Ser Ala Asn Asp Pro Gly Ile His Ile Asn Ile His Ala Ala Val
    210             215             220

Ser Asn Tyr Val Ala Pro Gly Pro Ala Val Tyr Ser Gly Gly Thr Thr
225             230             235             240

Lys Val Ala Gly Ser Gly Cys Gln Gly Cys Glu Asn Thr Cys Lys Val
            245             250             255

Gly Ser Ser Pro Thr Ala Thr Ala Pro Ser Gly Lys Ser Gly Ala Gly
            260             265             270

Ser Asp Gly Gly Ala Gly Thr Asp Gly Gly Ser Ser Ser Ser Ser Pro
            275             280             285

Asp Thr Gly Ser Ala Cys Ser Val Gln Ala Tyr Gly Gln Cys Gly Gly
    290             295             300

Asn Gly Tyr Ser Gly Cys Thr Gln Cys Ala Pro Gly Tyr Thr Cys Lys
305             310             315             320

Ala Val Ser Pro Pro Tyr Tyr Ser Gln Cys Ala Pro Ser Ser
            325             330
```

<210> 22
<211> 227
<212> PRT
<213> Thielavia terrestris

<400> 22

```
Met Lys Leu Ser Val Ala Ile Ala Val Leu Ala Ser Ala Leu Ala Glu
1               5               10              15
```

```
Ala His Tyr Thr Phe Pro Ser Ile Gly Asn Thr Ala Asp Trp Gln Tyr
             20              25              30

Val Arg Ile Thr Thr Asn Tyr Gln Ser Asn Gly Pro Val Thr Asp Val
         35              40              45

Thr Ser Asp Gln Ile Arg Cys Tyr Glu Arg Asn Pro Gly Thr Gly Ala
     50              55              60

Gln Gly Ile Tyr Asn Val Thr Ala Gly Gln Thr Ile Asn Tyr Asn Ala
65              70              75              80

Lys Ala Ser Ile Ser His Pro Gly Pro Met Ser Phe Tyr Ile Ala Lys
             85              90              95

Val Pro Ala Gly Gln Thr Ala Ala Thr Trp Asp Gly Lys Gly Ala Val
         100             105             110

Trp Thr Lys Ile Tyr Gln Asp Met Pro Lys Phe Gly Ser Ser Leu Thr
         115             120             125

Trp Pro Thr Met Gly Ala Lys Ser Val Pro Val Thr Ile Pro Arg Cys
     130             135             140

Leu Gln Asn Gly Asp Tyr Leu Leu Arg Ala Glu His Ile Ala Leu His
145             150             155             160

Ser Ala Ser Ser Val Gly Gly Ala Gln Phe Tyr Leu Ser Cys Ala Gln
             165             170             175

Leu Thr Val Ser Gly Gly Ser Gly Thr Trp Asn Pro Lys Asn Arg Val
         180             185             190

Ser Phe Pro Gly Ala Tyr Lys Ala Thr Asp Pro Gly Ile Leu Ile Asn
         195             200             205

Ile Tyr Tyr Pro Val Pro Thr Ser Tyr Ser Pro Pro Gly Pro Pro Ala
     210             215             220

Glu Thr Cys
225
```

<210> 23
<211> 368
<212> PRT
<213> Thielavia terrestris

<400> 23

```
Met Pro Ser Phe Ala Ser Lys Thr Leu Leu Ser Thr Leu Ala Gly Ala
1               5               10              15

Ala Ser Val Ala Ala His Gly His Val Ser Asn Ile Val Ile Asn Gly
            20              25              30

Val Ser Tyr Gln Gly Tyr Asp Pro Thr Ser Phe Pro Tyr Met Gln Asn
        35              40              45

Pro Pro Ile Val Val Gly Trp Thr Ala Ala Asp Thr Asp Asn Gly Phe
    50              55              60

Val Ala Pro Asp Ala Phe Ala Ser Gly Asp Ile Ile Cys His Lys Asn
65              70              75              80

Ala Thr Asn Ala Lys Gly His Ala Val Val Ala Ala Gly Asp Lys Ile
                85              90              95

Phe Ile Gln Trp Asn Thr Trp Pro Glu Ser His His Gly Pro Val Ile
            100             105             110

Asp Tyr Leu Ala Ser Cys Gly Ser Ala Ser Cys Glu Thr Val Asp Lys
        115             120             125

Thr Lys Leu Glu Phe Phe Lys Ile Asp Glu Val Gly Leu Val Asp Gly
    130             135             140

Ser Ser Ala Pro Gly Val Trp Gly Ser Asp Gln Leu Ile Ala Asn Asn
145             150             155             160

Asn Ser Trp Leu Val Glu Ile Pro Pro Thr Ile Ala Pro Gly Asn Tyr
            165             170             175

Val Leu Arg His Glu Ile Ile Ala Leu His Ser Ala Glu Asn Ala Asp
        180             185             190

Gly Ala Gln Asn Tyr Pro Gln Cys Phe Asn Leu Gln Ile Thr Gly Thr
        195             200             205

Gly Thr Ala Thr Pro Ser Gly Val Pro Gly Thr Ser Leu Tyr Thr Pro
    210             215             220

Thr Asp Pro Gly Ile Leu Val Asn Ile Tyr Ser Ala Pro Ile Thr Tyr
225             230             235             240

Thr Val Pro Gly Pro Ala Leu Ile Ser Gly Ala Val Ser Ile Ala Gln
```

245 250 255

Ser Ser Ser Ala Ile Thr Ala Ser Gly Thr Ala Leu Thr Gly Ser Ala
260 265 270

Thr Ala Pro Ala Ala Ala Ala Ala Thr Thr Thr Ser Thr Thr Asn Ala
275 280 285

Ala Ala Ala Ala Thr Ser Ala Ala Ala Ala Gly Thr Ser Thr Thr
290 295 300

Thr Thr Ser Ala Ala Ala Val Val Gln Thr Ser Ser Ser Ser Ser Ser
305 310 315 320

Ala Pro Ser Ser Ala Ala Ala Ala Ala Thr Thr Thr Ala Ala Ala Ser
325 330 335

Ala Arg Pro Thr Gly Cys Ser Ser Gly Arg Ser Arg Lys Gln Pro Arg
340 345 350

Arg His Ala Arg Asp Met Val Val Ala Arg Gly Ala Glu Glu Ala Asn
355 360 365

<210> 24
<211> 330
<212> PRT
<213> Thielavia terrestris

<400> 24

```
Met Pro Pro Ala Leu Pro Gln Leu Leu Thr Thr Val Leu Thr Ala Leu
1               5                   10              15

Thr Leu Gly Ser Thr Ala Leu Ala His Ser His Leu Ala Tyr Ile Ile
        20              25              30

Val Asn Gly Lys Leu Tyr Gln Gly Phe Asp Pro Arg Pro His Gln Ala
        35              40              45

Asn Tyr Pro Ser Arg Val Gly Trp Ser Thr Gly Ala Val Asp Asp Gly
    50              55              60

Phe Val Thr Pro Ala Asn Tyr Ser Thr Pro Asp Ile Ile Cys His Ile
65              70              75              80

Ala Gly Thr Ser Pro Ala Gly His Ala Pro Val Arg Pro Gly Asp Arg
            85              90              95

Ile His Val Gln Trp Asn Gly Trp Pro Val Gly His Ile Gly Pro Val
```

```
              100                    105                    110

    Leu Ser Tyr Leu Ala Arg Cys Glu Ser Asp Thr Gly Cys Thr Gly Gln
            115             120             125

    Asn Lys Thr Ala Leu Arg Trp Thr Lys Ile Asp Asp Ser Ser Pro Thr
            130             135             140

    Met Gln Asn Val Ala Gly Ala Gly Thr Gln Gly Glu Gly Thr Pro Gly
    145                 150             155                 160

    Lys Arg Trp Ala Thr Asp Val Leu Ile Ala Ala Asn Asn Ser Trp Gln
                    165             170             175

    Val Ala Val Pro Ala Gly Leu Pro Thr Gly Ala Tyr Val Leu Arg Asn
                180             185             190

    Glu Ile Ile Ala Leu His Tyr Ala Ala Arg Lys Asn Gly Ala Gln Asn
            195             200             205

    Tyr Pro Leu Cys Met Asn Leu Trp Val Asp Ala Ser Gly Asp Asn Ser
        210             215             220

    Ser Val Ala Ala Thr Thr Ala Ala Val Thr Ala Gly Gly Leu Gln Met
    225             230             235             240

    Asp Ala Tyr Asp Ala Arg Gly Phe Tyr Lys Glu Asn Asp Pro Gly Val
                245             250             255

    Leu Val Asn Val Thr Ala Ala Leu Ser Ser Tyr Val Val Pro Gly Pro
                260             265             270

    Thr Val Ala Ala Gly Ala Thr Pro Val Pro Tyr Ala Gln Gln Ser Pro
                275             280             285

    Ser Val Ser Thr Ala Ala Gly Thr Pro Val Val Val Thr Arg Thr Ser
            290             295             300

    Glu Thr Ala Pro Tyr Thr Gly Ala Met Thr Pro Thr Val Ala Ala Arg
    305             310             315             320

    Met Lys Gly Arg Gly Tyr Asp Arg Arg Gly
                325             330
```

<210> 25
<211> 236
<212> PRT

<213> Thielavia terrestris

<400> 25

```
Met Lys Thr Phe Thr Ala Leu Leu Ala Ala Ala Gly Leu Val Ala Gly
1               5               10              15

His Gly Tyr Val Asp Asn Ala Thr Ile Gly Gly Gln Phe Tyr Gln Asn
            20              25              30

Pro Ala Val Leu Thr Phe Phe Gln Pro Asp Arg Val Ser Arg Ser Ile
            35              40              45

Pro Gly Asn Gly Pro Val Thr Asp Val Thr Leu Ile Asp Leu Gln Cys
    50              55              60

Asn Ala Asn Ser Thr Pro Ala Lys Leu His Ala Thr Ala Ala Ala Gly
65              70              75              80

Ser Asp Val Ile Leu Arg Trp Thr Leu Trp Pro Glu Ser His Val Gly
            85              90              95

Pro Val Ile Thr Tyr Met Ala Arg Cys Pro Asp Thr Gly Cys Gln Asp
            100             105             110

Trp Met Pro Gly Thr Ser Ala Val Trp Phe Lys Ile Lys Glu Gly Gly
    115             120             125

Arg Asp Gly Thr Ser Asn Thr Trp Ala Asp Thr Pro Leu Met Thr Ala
    130             135             140

Pro Thr Ser Tyr Thr Tyr Thr Ile Pro Ser Cys Leu Lys Lys Gly Tyr
145             150             155             160

Tyr Leu Val Arg His Glu Ile Ile Ala Leu His Ala Ala Tyr Thr Tyr
            165             170             175

Pro Gly Ala Gln Phe Tyr Pro Gly Cys His Gln Leu Asn Val Thr Gly
            180             185             190

Gly Gly Ser Thr Val Pro Ser Ser Gly Leu Val Ala Phe Pro Gly Ala
    195             200             205

Tyr Lys Gly Ser Asp Pro Gly Ile Thr Tyr Asp Ala Tyr Lys Ala Gln
    210             215             220

Thr Tyr Gln Ile Pro Gly Pro Ala Val Phe Thr Cys
225             230             235
```

<210> 26
<211> 250

<212> PRT
<213> Thielavia terrestris

<400> 26

```
Met Ala Leu Leu Leu Leu Ala Gly Leu Ala Ile Leu Ala Gly Pro Ala
1               5               10              15

His Ala His Gly Gly Leu Ala Asn Tyr Thr Val Gly Asn Thr Trp Tyr
            20              25              30

Arg Gly Tyr Asp Pro Phe Thr Pro Ala Ala Asp Gln Ile Gly Gln Pro
            35              40              45

Trp Met Ile Gln Arg Ala Trp Asp Ser Ile Asp Pro Ile Phe Ser Val
    50              55              60

Asn Asp Lys Ala Leu Ala Cys Asn Thr Pro Ala Thr Ala Pro Thr Ser
65              70              75              80

Tyr Ile Pro Ile Arg Ala Gly Glu Asn Ile Thr Ala Val Tyr Trp Tyr
            85              90              95

Trp Leu His Pro Val Gly Pro Met Thr Ala Trp Leu Ala Arg Cys Asp
            100             105             110

Gly Asp Cys Arg Asp Ala Asp Val Asn Glu Ala Arg Trp Phe Lys Ile
            115             120             125

Trp Glu Ala Gly Leu Leu Ser Gly Pro Asn Leu Ala Glu Gly Met Trp
    130             135             140

Tyr Gln Lys Ala Phe Gln Asn Trp Asp Gly Ser Pro Asp Leu Trp Pro
145             150             155             160

Val Thr Ile Pro Ala Gly Leu Lys Ser Gly Leu Tyr Met Ile Arg His
            165             170             175

Glu Ile Leu Ser Ile His Val Glu Asp Lys Pro Gln Phe Tyr Pro Glu
            180             185             190

Cys Ala His Leu Asn Val Thr Gly Gly Gly Asp Leu Leu Pro Pro Asp
            195             200             205

Glu Phe Leu Val Lys Phe Pro Gly Ala Tyr Lys Glu Asp Asn Pro Ser
    210             215             220
```

```
        Ile Lys Ile Asn Ile Tyr Ser Asp Gln Tyr Ala Asn Thr Thr Asn Tyr
        225             230             235                     240


        Thr Ile Pro Gly Gly Pro Ile Trp Asp Gly
                        245             250
```

<210> 27
<211> 478
<212> PRT
<213> Thielavia terrestris

<400> 27

```
        Met Met Pro Ser Leu Val Arg Phe Ser Met Gly Leu Ala Thr Ala Phe
        1               5               10                  15


        Ala Ser Leu Ser Thr Ala His Thr Val Phe Thr Thr Leu Phe Ile Asn
                    20              25                  30


        Gly Val Asp Gln Gly Asp Gly Thr Cys Ile Arg Met Ala Lys Lys Gly
                    35              40              45


        Ser Val Cys Thr His Pro Ile Ala Gly Gly Leu Asp Ser Pro Asp Met
            50              55              60


        Ala Cys Gly Arg Asp Gly Gln Gln Ala Val Ala Phe Thr Cys Pro Ala
        65              70              75                  80


        Pro Ala Gly Ser Lys Leu Ser Phe Glu Phe Arg Met Trp Ala Asp Ala
                        85              90                  95


        Ser Gln Pro Gly Ser Ile Asp Pro Ser His Leu Gly Ser Thr Ala Ile
                    100             105             110


        Tyr Leu Lys Gln Val Ser Asn Ile Ser Ser Asp Ser Ala Ala Gly Pro
                    115             120             125


        Gly Trp Phe Lys Ile Tyr Ala Glu Gly Tyr Asp Thr Ala Ala Lys Lys
            130             135             140


        Trp Ala Thr Glu Lys Leu Ile Asp Asn Gly Gly Leu Leu Ser Ile Glu
        145             150             155             160


        Leu Pro Pro Thr Leu Pro Ala Gly Tyr Tyr Leu Ala Arg Ser Glu Ile
                        165             170             175


        Val Thr Ile Gln Asn Val Thr Asn Asp His Val Asp Pro Gln Phe Tyr
                    180             185             190
```

Val Gly Cys Ala Gln Leu Phe Val Gln Gly Pro Pro Thr Thr Pro Thr
195 200 205

Val Pro Pro Asp Arg Leu Val Ser Ile Pro Gly His Val His Ala Ser
210 215 220

Asp Pro Gly Leu Thr Phe Asn Ile Trp Arg Asp Asp Pro Ser Lys Thr
225 230 235 240

Ala Tyr Thr Val Val Gly Pro Ala Pro Phe Ser Pro Thr Ala Ala Pro
245 250 255

Thr Pro Thr Ser Thr Asn Thr Asn Gly Gln Gln Gln Gln Gln Gln Gln
260 265 270

Gln Ala Ile Lys Gln Thr Asp Gly Val Ile Pro Ala Asp Cys Gln Leu
275 280 285

Lys Asn Ala Asn Trp Cys Gly Ala Glu Val Pro Ala Tyr Ala Asp Glu
290 295 300

Ala Gly Cys Trp Ala Ser Ser Ala Asp Cys Phe Ala Gln Leu Asp Ala
305 310 315 320

Cys Tyr Thr Ser Ala Pro Pro Thr Gly Ser Arg Gly Cys Arg Leu Trp
325 330 335

Glu Asp Trp Cys Thr Gly Ile Gln Gln Gly Cys Arg Ala Gly Arg Trp
340 345 350

Arg Gly Pro Pro Pro Phe His Gly Glu Gly Ala Ala Ala Glu Thr Ala
355 360 365

Ser Ala Gly Arg Gly Gly Ala Arg Ile Ala Ala Val Ala Gly Cys Gly
370 375 380

Gly Gly Thr Gly Asp Met Val Glu Glu Val Phe Leu Phe Tyr Trp Asp
385 390 395 400

Ala Cys Ser Gly Trp Arg Arg Ser Arg Gly Gly Gly Ser Ile Leu Ala
405 410 415

Arg Leu Ile Leu His Val Leu Leu Pro Leu Leu Arg Pro Arg Arg Ala
420 425 430

Pro Arg Val His Leu Leu Leu Phe His Leu Tyr Leu Asn Phe Cys Tyr
435 440 445

78

```
        Pro Gly Thr Ser Gly Phe Tyr Asn Arg Leu Ser Ile Lys Leu Gly Ile
            450                 455                 460

        Trp Pro Ser Lys Met Ser Pro Asp Val Ala His Tyr Val Lys
            465                 470                 475
```

<210> 28
<211> 230
<212> PRT
<213> Thielavia terrestris

<400> 28

```
        Met Gln Leu Leu Val Gly Leu Leu Leu Ala Ala Val Ala Ala Arg Ala
        1                5                   10                  15

        His Tyr Thr Phe Pro Arg Leu Val Val Asn Gly Gln Pro Glu Asp Lys
                        20                  25                  30

        Asp Trp Ser Val Thr Arg Met Thr Lys Asn Ala Gln Ser Lys Gln Gly
                35                  40                  45

        Val Gln Asp Pro Thr Ser Pro Asp Ile Arg Cys Tyr Thr Ser Gln Thr
            50                  55                  60

        Ala Pro Asn Val Ala Thr Val Pro Ala Gly Ala Thr Val His Tyr Ile
        65                  70                  75                  80

        Ser Thr Gln Gln Ile Asn His Pro Gly Pro Thr Gln Tyr Tyr Leu Ala
                        85                  90                  95

        Lys Val Pro Ala Gly Ser Ser Ala Lys Thr Trp Asp Gly Ser Gly Ala
                        100                 105                 110

        Val Trp Phe Lys Ile Ser Thr Thr Met Pro Tyr Leu Asp Asn Asn Lys
                        115                 120                 125

        Gln Leu Val Trp Pro Asn Gln Asn Thr Tyr Thr Thr Val Asn Thr Thr
            130                 135                 140

        Ile Pro Ala Asp Thr Pro Ser Gly Glu Tyr Leu Leu Arg Val Glu Gln
        145                 150                 155                 160

        Ile Ala Leu His Leu Ala Ser Gln Pro Asn Gly Ala Gln Phe Tyr Leu
                        165                 170                 175

        Ala Cys Ser Gln Ile Gln Ile Thr Gly Gly Gly Asn Gly Thr Pro Gly
                        180                 185                 190
```

```
        Pro Leu Val Ala Leu Pro Gly Ala Tyr Lys Ser Asn Asp Pro Gly Ile
                195                 200             205
```

```
        Leu Val Asn Ile Tyr Ser Met Gln Pro Gly Asp Tyr Lys Pro Pro Gly
                210                 215             220
```

```
        Pro Pro Val Trp Ser Gly
        225                 230
```

<210> 29
<211> 257
<212> PRT
<213> Thielavia terrestris

<400> 29

```
        Met Lys Leu Tyr Leu Ala Ala Phe Leu Gly Ala Val Ala Thr Pro Gly
        1               5                   10                  15
```

```
        Ala Phe Ala His Gln Ile His Gly Ile Leu Leu Val Asn Gly Thr Glu
                20                  25                  30
```

```
        Thr Pro Glu Trp Lys Tyr Val Arg Asp Val Ala Trp Glu Gly Ala Tyr
                35                  40                  45
```

```
        Glu Pro Glu Lys Tyr Pro Asn Thr Glu Phe Phe Lys Thr Pro Pro Gln
                50                  55                  60
```

```
        Thr Asp Ile Asn Asn Pro Asn Ile Thr Cys Gly Arg Asn Ala Phe Asp
        65                  70                  75                  80
```

```
        Ser Ala Ser Lys Thr Glu Thr Ala Asp Ile Leu Ala Gly Ser Glu Val
                        85                  90                  95
```

```
        Gly Phe Arg Val Ser Trp Asp Gly Asn Gly Lys Tyr Gly Val Phe Trp
                    100                 105                 110
```

```
        His Pro Gly Pro Gly Gln Ile Tyr Leu Ser Arg Ala Pro Asn Asp Asp
                    115                 120                 125
```

```
        Leu Glu Asp Tyr Arg Gly Asp Gly Asp Trp Phe Lys Ile Ala Thr Gly
                    130                 135                 140
```

```
        Ala Ala Val Ser Asn Thr Glu Trp Leu Leu Trp Asn Lys His Asp Phe
        145                 150                 155                 160
```

```
        Asn Phe Thr Ile Pro Lys Thr Thr Pro Pro Gly Lys Tyr Leu Met Arg
                        165                 170                 175
```

Ile Glu Gln Phe Met Pro Ser Thr Val Glu Tyr Ser Gln Trp Tyr Val
                180                 185                 190

Asn Cys Ala His Val Asn Ile Ile Gly Pro Gly Gly Gly Thr Pro Thr
                195                 200                 205

Gly Phe Ala Arg Phe Pro Gly Thr Tyr Thr Val Asp Asp Pro Gly Ile
        210                 215                 220

Lys Val Pro Leu Asn Gln Ile Val Asn Ser Gly Glu Leu Pro Gln Asp
225                 230                 235                 240

Gln Leu Arg Leu Leu Glu Tyr Lys Pro Pro Gly Pro Ala Leu Trp Thr
                245                 250                 255

Gly

<210> 30
<211> 251
<212> PRT
<213> Thermoascus crustaceus

<400> 30

Met Ala Phe Ser Gln Ile Met Ala Ile Thr Gly Val Phe Leu Ala Ser
1                 5                 10                  15

Ala Ser Leu Val Ala Gly His Gly Phe Val Gln Asn Ile Val Ile Asp
                20                 25                  30

Gly Lys Ser Tyr Gly Gly Tyr Ile Val Asn Gln Tyr Pro Tyr Met Ser
                35                 40                  45

Asp Pro Pro Glu Val Val Gly Trp Ser Thr Thr Ala Thr Asp Leu Gly
        50                 55                 60

Phe Val Asp Gly Thr Gly Tyr Gln Gly Pro Asp Ile Ile Cys His Arg
65                 70                 75                  80

Gly Ala Lys Pro Ala Ala Leu Thr Ala Gln Val Ala Ala Gly Gly Thr
                85                 90                  95

Val Lys Leu Glu Trp Thr Pro Trp Pro Asp Ser His His Gly Pro Val
                100                 105                 110

Ile Asn Tyr Leu Ala Pro Cys Asn Gly Asp Cys Ser Thr Val Asp Lys
                115                 120                 125

```
Thr Gln Leu Lys Phe Phe Lys Ile Ala Gln Ala Gly Leu Ile Asp Asp
    130             135             140

Asn Ser Pro Pro Gly Ile Trp Ala Ser Asp Asn Leu Ile Ala Ala Asn
145             150             155             160

Asn Ser Trp Thr Val Thr Ile Pro Thr Thr Thr Ala Pro Gly Asn Tyr
                165             170             175

Val Leu Arg His Glu Ile Ile Ala Leu His Ser Ala Gly Asn Lys Asp
            180             185             190

Gly Ala Gln Asn Tyr Pro Gln Cys Ile Asn Leu Lys Val Thr Gly Asn
        195             200             205

Gly Ser Gly Asn Pro Pro Ala Gly Ala Leu Gly Thr Ala Leu Tyr Lys
    210             215             220

Asp Thr Asp Pro Gly Ile Leu Ile Asn Ile Tyr Gln Lys Leu Ser Ser
225             230             235             240

Tyr Val Ile Pro Gly Pro Ala Leu Tyr Thr Gly
                245             250
```

<210> 31
<211> 349
<212> PRT
<213> Thermoascus crustaceus

<400> 31

```
Met Ser Phe Ser Lys Ile Leu Ala Ile Ala Gly Ala Ile Thr Tyr Ala
1               5               10              15

Ser Ser Ala Ala Ala His Gly Tyr Val Gln Gly Ile Val Val Asp Gly
        20              25              30

Ser Tyr Tyr Gly Gly Tyr Met Val Thr Gln Tyr Pro Tyr Thr Ala Gln
        35              40              45

Pro Pro Glu Leu Ile Ala Trp Ser Thr Lys Ala Thr Asp Leu Gly Phe
    50              55              60

Val Asp Gly Ser Gly Tyr Thr Ser Pro Asp Ile Ile Cys His Lys Gly
65              70              75              80

Ala Glu Pro Gly Ala Gln Ser Ala Lys Val Ala Ala Gly Gly Thr Val
            85              90              95
```

Glu Leu Gln Trp Thr Ala Trp Pro Glu Ser His Lys Gly Pro Val Ile
                100                 105                 110

Asp Tyr Leu Ala Ala Cys Asp Gly Asp Cys Ser Ser Val Asp Lys Thr
            115                 120                 125

Ala Leu Lys Phe Phe Lys Ile Asp Glu Ser Gly Leu Ile Asp Gly Asn
        130                 135                 140

Gly Ala Gly Thr Trp Ala Ser Asp Thr Leu Ile Lys Asn Asn Asn Ser
145                 150                 155                 160

Trp Thr Val Thr Ile Pro Ser Thr Ile Ala Ser Gly Asn Tyr Val Leu
                165                 170                 175

Arg His Glu Ile Ile Ala Leu His Ser Ala Gly Asn Lys Asp Gly Ala
            180                 185                 190

Gln Asn Tyr Pro Gln Cys Ile Asn Leu Glu Val Thr Gly Ser Gly Thr
            195                 200                 205

Glu Asn Pro Ala Gly Thr Leu Gly Thr Ala Leu Tyr Thr Asp Thr Asp
    210                 215                 220

Pro Gly Leu Leu Val Asn Ile Tyr Gln Gly Leu Ser Asn Tyr Ser Ile
225                 230                 235                 240

Pro Gly Pro Ala Leu Tyr Ser Gly Asn Ser Asp Asn Ala Gly Ser Leu
                245                 250                 255

Asn Pro Thr Thr Thr Pro Ser Ile Gln Asn Ala Ala Ala Ala Pro Ser
            260                 265                 270

Thr Ser Thr Ala Ser Val Val Thr Asp Ser Ser Ser Ala Thr Gln Thr
        275                 280                 285

Ala Ser Val Ala Ala Thr Thr Pro Ala Ser Thr Ser Ala Val Thr Ala
    290                 295                 300

Ser Pro Ala Pro Asp Thr Gly Ser Asp Val Thr Lys Tyr Leu Asp Ser
305                 310                 315                 320

Met Ser Ser Asp Glu Val Leu Thr Leu Val Arg Gly Thr Leu Ser Trp
                325                 330                 335

Leu Val Ser Asn Lys Lys His Ala Arg Asp Leu Ser His
            340                 345

<210> 32
<211> 436
<212> PRT
<213> Thermoascus crustaceus

<400> 32

```
Met Leu Ser Phe Ile Pro Thr Lys Ser Ala Ala Leu Thr Thr Leu Leu
1               5                   10                  15

Leu Leu Gly Thr Ala His Ala His Thr Leu Met Thr Thr Met Phe Val
            20                  25                  30

Asp Gly Val Asn Gln Gly Asp Gly Val Cys Ile Arg Met Asn Asn Asp
            35                  40                  45

Gly Gly Thr Ala Asn Thr Tyr Ile Gln Pro Ile Thr Ser Lys Asp Ile
        50                  55                  60

Ala Cys Gly Ile Gln Gly Glu Ile Gly Ala Ser Arg Val Cys Pro Val
65                  70                  75                  80

Lys Ala Ser Ser Thr Leu Thr Phe Gln Phe Arg Glu Gln Pro Asn Asn
                85                  90                  95

Pro Asn Ser Ser Pro Leu Asp Pro Ser His Lys Gly Pro Ala Ala Val
            100                 105                 110

Tyr Leu Lys Lys Val Asp Ser Ala Ile Ala Ser Asn Asn Ala Ala Gly
            115                 120                 125

Asp Ser Trp Phe Lys Ile Trp Glu Ser Val Tyr Asp Glu Ser Thr Gly
            130                 135                 140

Lys Trp Gly Thr Thr Lys Met Ile Glu Asn Asn Gly His Ile Ser Val
145                 150                 155                 160

Lys Val Pro Asp Asp Ile Glu Gly Gly Tyr Tyr Leu Ala Arg Thr Glu
                165                 170                 175

Leu Leu Ala Leu His Ser Ala Asp Gln Gly Asp Pro Gln Phe Tyr Val
            180                 185                 190

Gly Cys Ala Gln Leu Phe Ile Asp Ser Asp Gly Thr Ala Lys Pro Pro
            195                 200                 205

Thr Val Ser Ile Gly Glu Gly Thr Tyr Asp Leu Ser Met Pro Ala Met
            210                 215                 220
```

```
Thr Tyr Asn Ile Trp Glu Thr Pro Leu Ala Leu Pro Tyr Pro Met Tyr
225             230             235             240

Gly Pro Pro Val Tyr Thr Pro Gly Ser Gly Ser Gly Ser Val Arg Ala
            245             250             255

Thr Ser Ser Ser Ala Val Pro Thr Ala Thr Glu Ser Ser Phe Val Glu
        260             265             270

Glu Arg Ala Asn Pro Val Thr Ala Asn Ser Val Tyr Ser Ala Arg Gly
        275             280             285

Lys Phe Lys Thr Trp Ile Asp Lys Leu Ser Trp Arg Gly Lys Val Arg
    290             295             300

Glu Asn Val Arg Gln Ala Ala Gly Arg Arg Ser Thr Leu Val Gln Thr
305             310             315             320

Val Gly Leu Lys Pro Lys Gly Cys Ile Phe Val Asn Gly Asn Trp Cys
            325             330             335

Gly Phe Glu Val Pro Asp Tyr Asn Asp Ala Glu Ser Cys Trp Ala Ala
        340             345             350

Ser Asp Asn Cys Trp Lys Gln Ser Asp Ala Cys Trp Asn Lys Thr Gln
        355             360             365

Pro Thr Gly Tyr Asn Asn Cys Gln Ile Trp Gln Asp Lys Lys Cys Lys
    370             375             380

Val Ile Gln Asp Ser Cys Ser Gly Pro Asn Pro His Gly Pro Pro Asn
385             390             395             400

Lys Gly Lys Asp Leu Thr Pro Glu Trp Pro Pro Leu Lys Gly Ser Met
            405             410             415

Asp Thr Phe Ser Lys Arg Thr Ile Gly Tyr Arg Asp Trp Ile Val Arg
        420             425             430

Arg Arg Gly Ala
        435
```

**Description**

**FIELD OF THE INVENTION**

The present invention relates to processes of dewatering whole stillage.

**BACKGROUND OF THE INVENTION**

Due to the limited reserves of fossil fuels and worries about emission of greenhouse gases there is an increasing focus on using renewable energy sources.

Processes for producing fermentation products, such as ethanol, from a starch or lignocellulose containing material are well known in the art. The preparation of the starch-containing material such as corn for utilization in such fermentation processes typically begins with grinding the corn in a dry-grind or wet-milling process. Wet-milling processes involve fractionating the corn into different components where only the starch fraction enters into the fermentation process. Dry-grind processes involve grinding the corn kernels into meal and mixing the meal with water and enzymes. Generally, two different kinds of dry-grind processes are used. The most commonly used process, often referred to as a "conventional process," includes grinding the starch-containing material and then liquefying gelatinized starch at a high temperature using typically a bacterial alpha-amylase, followed by simultaneous saccharification and fermentation (SSF) carried out in the presence of a glucoamylase and a fermentation organism. Another well known process, often referred to as a "raw starch hydrolysis" process (RSH process), includes grinding the starch-containing material and then simultaneously saccharifying and fermenting granular starch below the initial gelatinization temperature typically in the presence of an acid fungal alpha-amylase and a glucoamylase.

In a process for producing ethanol from corn, following SSF or the RSH process the ethanol is distilled from the whole mash after fermentation. The resulting ethanol-free slurry, usually referred to as whole stillage, is separated into solid and liquid fractions (*i.e.,* wet cake and thin stillage containing about 35 and 7% solids, respectively). The thin stillage is often condensed by evaporation into a thick stillage or syrup and recombined with the wet cake and further dried into distillers' dried grains with solubles distillers' dried grain with solubles (DDGS) for use in animal feed. The oil content of DDGS is sometimes higher than desired and methods of recovering more oil as a separate by-product for use in biodiesel production or other biorenewable products are sought.

Much of the work in oil recovery from fermentation processes has focused on improving the extractability of the oil from the whole stillage. Effective removal of oil is often accomplished by hexane extraction. However, the utilization of hexane extraction has not seen widespread application due to the high capital investment required. Therefore, other methods that improve oil extraction from fermentation processes have been explored.

U.S. Patent No. 6,433,146 discloses extracting oil and zein from corn or corn processing by-products using ethanol.

U.S. Patent No. 7,601,858 discloses a method for recovering oil from a concentrated byproduct, such as thin stillage formed during a dry milling process used for producing ethanol. The method includes forming a concentrate from the byproduct, e.g., by evaporating the by-product, and recovering oil from the concentrate.

U.S. Patent No. 7,608,729 discloses a method of freeing the bound oil present in whole stillage and thin stillage by heating the stillage to a temperature sufficient to at least partially separate, or bind, the oil from the stillage.

U.S. Application Publication No. 2010/0058649 discloses a method of separating an oil fraction from a fermentation product, adjusting the pH of the oil fraction, and recovering the oil from the oil fraction.

Wang (2008, Lipid Technology 20(9): 203-207) and Wang et al. (2009, J. Agric. Food Chem. 57: 2302-2307) have investigated the use of enzymes and different grind and extrusion methods to enhance oil partitioning.

Both whole stillage and thin stillage consist of a large amount of water. Methods for dewatering stillage are known in the art.

WO 2007/056321-A1 discloses a method of dewatering whole stillage derived from a process for producing ethanol from a starch-containing material by subjecting the whole stillage to an enzyme, such as a hemicellulase and then separating the whole stillage into a solid fraction and a liquid fraction.

It is an object of the present invention to provide improved processes of dewatering whole stillage.

**SUMMARY OF THE INVENTION**

The present invention relates to processes of dewatering whole stillage, comprising

> (a) converting a starch-containing material into dextrins with an alpha-amylase;
> (b) saccharifying the dextrins using a carbohydrate source generating enzyme to form a sugar;
> (c) fermenting the sugar in a fermentation medium into a fermentation product using a fermenting organism, wherein the fermentation medium comprises a hemicellulase(s), an endoglucanase(s), and a GH61 polypeptide(s);

(d) distilling the fermentation product to form the whole stillage; and

(e) separating the whole stillage into thin stillage and wet cake, whereby more liquid phase is transferred to the thin stillage.

## DETAILED DESCRIPTION OF THE INVENTION

### Definitions

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and *p*-nitrophenyl acetate. For purposes of the present invention, acetylxylan esterase activity is determined using 0.5 mM *p*-nitrophenylacetate as substrate in 50 mM sodium acetate pH 5.0 containing 0.01% TWEEN™ 20. One unit of acetylxylan esterase is defined as the amount of enzyme capable of releasing 1 micromole of *p*-nitrophenolate anion per minute at pH 5, 25°C.

**Alpha-Amylases:** The term "alpha-amylase" means an alpha-1,4-glucan-4-glucanohydrolase (E.C. 3.2.1.1) that catalyzes the hydrolysis of starch and other linear and branched 1,4-glucosidic oligo- and polysaccharides.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present invention, alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 microliters for 30 minutes at 40°C followed by arabinose analysis by AMINEX® HPX-87H column chromatography (Bio-Rad Laboratories, Inc., Hercules, CA, USA).

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. For purposes of the present invention, alpha-glucuronidase activity is determined according to de Vries, 1998, J. Bacteriol. 180: 243-249. One unit of alpha-glucuronidase equals the amount of enzyme capable of releasing 1 micromole of glucuronic or 4-O-methylglucuronic acid per minute at pH 5, 40°C.

**Beta-glucosidase:** The term "beta-glucosidase" means a beta-D-glucoside glucohydrolase (E.C. 3.2.1.21), which catalyzes the hydrolysis of terminal non-reducing beta-D-glucose residues with the release of beta-D-glucose. For purposes of the present invention, beta-glucosidase activity is determined according to the basic procedure described by Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 micromole of *p*-nitrophenolate anion produced per minute at 25°C, pH 4.8 from 1 mM p-nitrophenyl-beta-D-glucopyranoside as substrate in 50 mM sodium citrate containing 0.01% TWEEN® 20.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from the non-reducing termini. For purposes of the present invention, one unit of beta-xylosidase is defined as 1.0 micromole of *p*-nitrophenolate anion produced per minute at 40°C, pH 5 from 1 mM *p*-nitrophenyl-beta-D-xyloside as substrate in 100 mM sodium citrate containing 0.01% TWEEN® 20.

**Cellobiohydrolase:** The term "cellobiohydrolase" means a 1,4-beta-D-glucan cellobiohydrolase (E.C. 3.2.1.91) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). Cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Tomme *et al.* method can be used to determine cellobiohydrolase activity.

**Cellulosic material:** The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, herbaceous material (including energy crops), agricultural residue, wood (including forestry residue), municipal solid waste, waste paper, and pulp and paper mill residue (see, for example, Wiselogel et al., 1995, in Handbook on Bioethanol (Charles E. Wyman, editor), pp. 105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp.23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is herbaceous material (including energy crops). In another aspect, the cellulosic material is agricultural residue. In another aspect, the cellulosic material is wood (including forestry residue). In another aspect, the cellulosic material is municipal solid waste. In another aspect, the cellulosic material is waste paper. In another aspect, the cellulosic material is pulp and paper mill residue.

In another aspect, the cellulosic material is corn stover. In another aspect, the cellulosic material is wheat straw. In another aspect, the cellulosic material is bagasse. In another aspect, the cellulosic material is corn cob. In another aspect, the cellulosic material is switchgrass. In another aspect, the cellulosic material is corn fiber. In another aspect, the cellulosic material is rice straw. In another aspect, the cellulosic material is miscanthus. In another aspect, the cellulosic material is orange peel. In another aspect, the cellulosic material is poplar. In another aspect, the cellulosic material is pine. In another aspect, the cellulosic material is willow. In another aspect, the cellulosic material is eucalyptus.

In another aspect, the cellulosic material is microcrystalline cellulose. In another aspect, the cellulosic material is bacterial cellulose. In another aspect, the cellulosic material is algal cellulose. In another aspect, the cellulosic material is cotton linter. In another aspect, the cellulosic material is amorphous phosphoric-acid treated cellulose. In another aspect, the cellulosic material is filter paper.

In another aspect, the cellulosic material is an aquatic biomass. As used herein the term "aquatic biomass" means biomass produced in an aquatic environment by a photosynthesis process. The aquatic biomass can be algae; submerged plants; emergent plants; and floating-leaf plants.

The cellulosic material may be used as is or may be subjected to pretreatment, using conventional methods known in the art, as described herein. In a preferred aspect, the cellulosic material is pretreated.

**Cellulolytic enzyme or cellulase:** The term "cellulolytic enzyme" or "cellulase" means one or more (*e.g.,* several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, etc. The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present invention, cellulolytic enzyme activity is determined by measuring the increase in hydrolysis of a cellulosic material by cellulolytic enzyme(s) under the following conditions: 1-20 mg of cellulolytic enzyme protein/g of cellulose in PCS for 3-7 days at a suitable temperature, *e.g.,* 50°C, 55°C, or 60°C, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, or 60°C, 72 hours, sugar analysis by AMINEX® HPX-87H column (Bio-Rad Laboratories, Inc., Hercules, CA, USA).

**Coding sequence:** The term "coding sequence" means a polynucleotide, which directly specifies the amino acid sequence of its polypeptide product. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

**Control sequence:** The term "control sequence" means all components necessary for the expression of a polynucleotide encoding an alpha-amylase of the present invention. Each control sequence may be native or foreign to the polynucleotide encoding the alpha-amylase or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding an alpha-amylase.

**Endoglucanase:** The term "endoglucanase" means an endo-1,4-(1,3;1,4)-beta-D-glucan 4-glucanohydrolase (E.C. 3.2.1.4), which catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). For purposes of the present invention, endoglucanase activity is determined using carboxymethyl cellulose (CMC) as substrate according to the procedure of Ghose, 1987, Pure and Appl. Chem. 59: 257-268, at pH 5, 40°C.

**Expression:** The term "expression" includes any step involved in the production of the polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to additional nucleotides that provide for its expression.

**Family 61 glycoside hydrolase:** The term "Family 61 glycoside hydrolase" or "Family GH61" or "GH61" means a polypeptide falling into the glycoside hydrolase Family 61 according to Henrissat, 1991, A classification of glycosyl hydrolases based on amino-acid sequence similarities, Biochem. J. 280: 309-316, and Henrissat and Bairoch, 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are certainly non-canonical and they cannot be considered as bona fide glycosidases. However, they are kept in the CAZy classification on the basis of their capacity to enhance the breakdown of lignocellulose when used in conjunction with a cellulase or a mixture of cellulases.

**Ferulic acid esterase or feruloyl esterase:** The term "ferulic acid esterase" or "feruloyl esterase" means a 4-hydroxy-3-methoxycinnamoyl-sugar hydrolase (EC 3.1.1.73) that catalyzes the hydrolysis of the 4-hydroxy-3-methoxycinnamoyl (feruloyl) group from an esterified sugar, which is usually arabinose in "natural" substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II. For purposes of the present invention, feruloyl esterase activity is determined using 0.5 mM *p*-nitrophenylferulate as substrate in 50 mM sodium acetate pH 5.0. One unit of feruloyl esterase equals the amount of enzyme capable of releasing 1 micromole of *p*-nitrophenolate anion per minute at pH 5, 25°C.

**Hemicellulolytic enzyme or hemicellulase:** The term "hemicellulolytic enzyme" or "hemicellulase" means one or more (*e.g.,* several) enzymes that hydrolyze a hemicellulosic material. See, for example, Shallom and Shoham, 2003, Microbial hemicellulases, Current Opinion In Microbiology 6(3): 219-228. Hemicellulases are key components in the degradation of plant biomass. Examples of hemicellulases include, but are not limited to, an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families marked by numbers. Some families, with overall similar fold, can be further grouped into clans, marked alphabetically (*e.g.,* GH-A). A most informative and updated classification of these and other carbohydrate active enzymes is available on the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature, e.g., 50°C, 55°C, or 60°C.

**Host cell:** The term "host cell" means any cell type that is susceptible to transformation, transfection, transduction, and the like with a nucleic acid construct or expression vector comprising a polynucleotide. The term "host cell" encompasses any progeny of a parent cell that is not identical to the parent cell due to mutations that occur during replication.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence.

**Operably linked:** The term "operably linked" means a configuration in which a control sequence is placed at an appropriate position relative to the coding sequence of the polynucleotide sequence such that the control sequence directs the expression of the coding sequence of a polypeptide.

**Polypeptide fragment:** The term "polypeptide fragment" means a polypeptide having one or more (several) amino acids deleted from the amino and/or carboxyl terminus of a mature polypeptide; wherein the fragment has enzyme activity.

**Polypeptide having cellulolytic enhancing activity:** The term "polypeptide having cellulolytic enhancing activity" means a GH61 polypeptide that catalyzes the enhancement of the hydrolysis of a cellulosic material by enzyme having cellulolytic activity. For purposes of the present invention, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS, wherein total protein is comprised of 50-99.5% w/w cellulolytic enzyme protein and 0.5-50% w/w protein of a GH61 polypeptide having cellulolytic enhancing activity for 1-7 days at a suitable temperature, *e.g.,* 50°C, 55°C, or 60°C, compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsværd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 2002/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, more preferably at least 1.05-fold, more preferably at least 1.10-fold, more preferably at least 1.25-fold, more preferably at least 1.5-fold, more preferably at least 2-fold, more preferably at least 3-fold, more preferably at least 4-fold, more preferably at least 5-fold, even more preferably at least 10-fold, and most preferably at least 20-fold.

**Pretreated corn stover:** The term "PCS" or "Pretreated Corn Stover" means a cellulosic material derived from corn stover by treatment with heat and dilute sulfuric acid.

**Sequence Identity:** The relatedness between two amino acid sequences or between two nucleotide sequences is described by the parameter "sequence identity".

For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\text{(Identical Residues x 100)/(Length of Alignment – Total Number of Gaps in Alignment)}$$

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice *et al.,* 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\text{(Identical Deoxyribonucleotides x 100)/(Length of Alignment – Total Number of Gaps in Alignment)}$$

**Xylan-containing material:** The term "xylan-containing material" means any material comprising a plant cell wall polysaccharide containing a backbone of beta-(1-4)-linked xylose residues. Xylans of terrestrial plants are heteropolymers possessing a beta-(1-4)-D-xylopyranose backbone, which is branched by short carbohydrate chains. They comprise D-glucuronic acid or its 4-*O*-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67.

In the methods of the present invention, any material containing xylan may be used. In a preferred aspect, the xylan-containing material is lignocellulose.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.,* acetylxylan esterases, alpha-glucuronyl esterases, alpha-glucuronidases, arabinofuranosidases, beta-xylosidases, endoxylanases, and feruloyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann et al., 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% TRITON® X-100 and 200 mM sodium phosphate buffer pH 6 at 37°C. One unit of xylanase activity is defined as 1.0 micromole of azurine produced per minute at 37°C, pH 6 from 0.2% AZCL-arabinoxylan as substrate in 200 mM sodium phosphate pH 6 buffer.

For purposes of the present invention, xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, A new reaction for colorimetric determination of carbohydrates, Anal. Biochem 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present invention, xylanase activity is determined with 0.2% AZCL-arabinoxylan as substrate in 0.01% TRITON® X-100 and 200 mM sodium phosphate buffer pH 6 at 37°C. One unit of xylanase activity is defined as 1.0 micromole of azurine produced per minute at 37°C, pH 6 from 0.2% AZCL-arabinoxylan as substrate in 200 mM sodium phosphate pH 6 buffer.

Processes of the Present Invention

The present invention relates to processes of dewatering whole stillage, comprising

(a) converting a starch-containing material into dextrins with an alpha-amylase;
(b) saccharifying the dextrins using a carbohydrate source generating enzyme to form a sugar;
(c) fermenting the sugar in a fermentation medium into a fermentation product using a fermenting organism, wherein the fermentation medium comprises a hemicellulase(s), an endoglucanase(s), and a GH61 polypeptide(s);
(d) distilling the fermentation product to form the whole stillage; and
(e) separating the whole stillage into thin stillage and wet cake, whereby more liquid phase is transferred to the thin stillage.

In an embodiment, the endoglucanase(s) is added in an amount of 0.01-1.0, *e.g.,* 0.02-0.08, 0.025-0.06, 0.025-0.05, or 0.03-0.04 EGU/g dry solids.

In an embodiment, the endoglucanase(s) is added in an amount of 1-30, *e.g.,* 5-30 7-25, 10-20, 10-17, or 12-15 micrograms/g dry solids.

In an embodiment, the hemicellulase(s) is added in an amount of 0.01-1.0, *e.g.,* 0.015-0.08, 0.015-0.06, 0.015-0.04, or 0.02-0.03 FXU/g dry solids.

In an embodiment, the hemicellulase(s) is added in an amount of 1-30, *e.g.,* 5-30, 7-25, 10-20, 10-17, or 12-15 micrograms/g dry solids.

Processes for producing fermentation products from a gelatinized starch-containing material

Disclosed is a process for producing a fermentation product comprising:

(a) liquefying a starch-containing material in the presence of an alpha-amylase;
(b) saccharifying the liquefied material obtained in step (a) using a carbohydrate-source generating enzyme; and
(c) fermenting using a fermenting organism in the presence of a hemicellulase(s).

The process for producing a fermentation product comprising the steps of:

(a) liquefying the starch-containing material in the presence of an alpha-amylase;
(b) saccharifying the liquefied material obtained in step (a) using a carbohydrate-source generating enzyme; and
(c) fermenting using a fermenting organism in the presence of an endoglucanase(s) and a hemicellulase(s), wherein the endoglucanase(s) is present in an amount of 0.01-1.0 EGU/g dry solids and in an amount of 1-30 micrograms/g dry solids and the hemicellulase(s) is present in an amount of 0.01-1.0 FXU/g dry solids and in an amount of 1-30 micrograms/g dry solids.

The saccharification and fermentation steps may be carried out either sequentially or simultaneously. The hemicellulase(s) and/or endoglucanase(s) may be added during saccharification and/or fermentation when the process is carried out as a sequential saccharification and fermentation process and before or during fermentation when steps (b) and (c) are carried out simultaneously (SSF process).

The liquefaction is preferably carried out in the presence of an alpha-amylase, preferably a bacterial alpha-amylase or acid fungal alpha-amylase. In an embodiment, a pullulanase, isoamylase, and and/or phytase is added during liquefaction. The fermenting organism is preferably a yeast, *e.g.,* a strain of *Saccharomyces cerevisiae.* Suitable fermenting organisms are listed in the "Fermenting Organisms" section below.

Liquefaction may be carried out as a three-step hot slurry process. The slurry is heated to between 60-95°C, preferably 80-85°C, and an alpha-amylase is added to initiate liquefaction (thinning). Then the slurry may be jet-cooked at a temperature between 95-140°C, preferably 105-125°C, for about 1-15 minutes, preferably for about 3-10 minutes, especially around about 5 minutes. The slurry is cooled to 60-95°C and more alpha-amylase is added to complete the hydrolysis (secondary liquefaction). The liquefaction process is usually carried out at a pH of 4.0 to 6.5, in particular at a pH of 4.5 to 6.

Saccharification may be carried out using conditions well known in the art with a saccharifying enzyme, *e.g.,* beta-amylase, glucoamylase or maltogenic amylase, and optionally a debranching enzyme, such as an isoamylase or a pullulanase. For instance, a full saccharification process may last up to from about 24 to about 72 hours, however, it is common to do a pre-saccharification for typically 40-90 minutes at a temperature between 30-65°C, typically about 60°C, followed by complete saccharification during fermentation in a simultaneous saccharification and fermentation process (SSF process). Saccharification is typically carried out at a temperature from 20-75°C, preferably from 40-70°C, typically around 60°C, and at a pH between 4 and 5, normally at about pH 4.5.

The most widely used process to produce a fermentation product, especially ethanol, is the simultaneous saccharification and fermentation (SSF) process, in which there is no holding stage for the saccharification, meaning that a fermenting organism, such as a yeast, and enzyme(s), including the hemicellulase(s) and/or endoglucanase(s), may be added together. SSF is typically carried out at a temperature from 25°C to 40°C, such as from 28°C to 35°C, from 30°C to 34°C, preferably around about 32°C. In an embodiment, fermentation is ongoing for 6 to 120 hours, in particular 24 to 96 hours.

In a particular embodiment, the process described wherein further comprises, prior to step (a), the steps of:

x) reducing the particle size of the starch-containing material, preferably by milling; and
y) forming a slurry comprising the starch-containing material and water.

The aqueous slurry may contain from 10-55 w/w % dry solids (DS), preferably 25-45 w/w % dry solids (DS), more preferably 30-40 w/w % dry solids (DS) of the starch-containing material. The slurry is heated to above the gelatinization temperature and an alpha-amylase, preferably a bacterial and/or acid fungal alpha-amylase, may be added to initiate liquefaction (thinning). The slurry may be jet-cooked to further gelatinize the slurry before being subjected to an alpha-amylase in step (a).

Processes for producing fermentation products from an ungelatinized starch-containing material

Disclosed are processes for producing a fermentation product from a starch-containing material without gelatinization (*i.e.,* without cooking) of the starch-containing material in the presence of a hemicellulase(s).

Disclosed are also processes for producing a fermentation product from a starch-containing material without gelatinization (*i.e.,* without cooking) of the starch-containing material wherein an endoglucanase(s) and a hemicellulase(s) are present during the raw starch hydrolysis process, wherein the endoglucanase(s) is present in an amount of 0.01-1.0 EGU/g dry solids and in an amount of 1-30 micrograms/g dry solids, the hemicellulase(s) is present in an amount of 0.01-1.0 FXU/g dry solids and in an amount of 1-30 micrograms/g dry solids.

In these embodiments, the desired fermentation product, such as ethanol, is produced from an ungelatinized starch containing material. The process comprises simultaneously saccharifying and fermenting a starch-containing material, *e.g.,* granular starch, using an alpha-amylase, a carbohydrate-source generating enzyme, and a fermenting organism at a temperature below the initial gelatinization temperature of the starch-containing material, in the presence of the endogluanase(s) and/or hemicellulase(s).

As used herein, the term "initial gelatinization temperature" means the lowest temperature at which starch gelatinization commences. In general, starch heated in water begins to gelatinize between about 50°C and 75°C; the exact temperature of gelatinization depends on the specific starch and can readily be determined by the skilled artisan. Thus, the initial gelatinization temperature may vary according to the plant species, to the particular variety of the plant species as well as with the growth conditions. In the context of this invention the initial gelatinization temperature of a given starch-containing material may be determined as the temperature at which birefringence is lost in 5% of the starch granules using the method described by Gorinstein and Lii, 1992, Starch/Starke 44(12): 461-466.

An RSH process is conducted at a temperature below the initial gelatinization temperature, which means that the temperature typically lies in the range between 30-75°C, preferably between 45-60°C. In an embodiment, the process is carried out at a temperature from 25°C to 40°C, such as from 28°C to 35°C, such as from 30°C to 34°C, preferably around 32°C.

A skilled person in the art can easily determine which doses/quantities of enzyme and fermenting organism to use.

In one embodiment, a slurry of a starch-containing material, such as granular starch, having 10-55 w/w % dry solids (DS), preferably 25-45 w/w % dry solids, more preferably 30-40 w/w % dry solids of the starch-containing material may be prepared. The slurry may include water and/or process waters, such as stillage (backset), scrubber water, evaporator condensate or distillate, side-stripper water from distillation, or process water from other fermentation product plants. Because the RSH process of the invention is carried out below the initial gelatinization temperature, and thus no significant viscosity increase takes place, high levels of stillage may be used if desired. In an embodiment the aqueous slurry contains from about 1 to about 70 vol. %, preferably 15-60 vol. %, especially from about 30 to 50 vol. % water and/or process waters, such as stillage (backset), scrubber water, evaporator condensate or distillate, side-stripper water from distillation, or process water from other fermentation product plants, or combinations thereof, or the like.

The starch-containing material may be prepared by reducing the particle size, preferably by dry or wet milling, to 0.05 to 3.0 mm, preferably 0.1-0.5 mm. After being subjected to a process of the invention at least 85%, at least 86%, at least 87%, at least 88%, at least 89%, at least 90%, at least 91%, at least 92%, at least 93%, at least 94%, at least 95%, at least 96%, at least 97%, at least 98%, or preferably at least 99% of the dry solids in the starch-containing material are converted into a soluble starch hydrolyzate.

Processes for producing fermentation products from a lignocellulose-containing material

Disclosed are processes of producing a fermentation product, comprising the steps of:

(a) pre-treating a lignocellulose-containing material;
(b) hydrolyzing the pre-treated lignocellulose-containing material; and
(c) fermenting using a fermenting organism in the presence of a hemicellulase(s).

Disclosed are processes of producing a fermentation product, comprising the steps of:

(a) pre-treating a lignocellulose-containing material;
(b) hydrolyzing the pre-treated lignocellulose-containing material; and
(c) fermenting using a fermenting organism in the presence of an endoglucanase(s) and a hemicellulase(s), wherein the endoglucanase(s) is present in an amount of 0.01-1.0 EGU/g dry solids and in an amount of 1-30 micrograms/g dry solids, the hemicellulase(s) is present in an amount of 0.01-1.0 FXU/g dry solids and in an amount of 1-30 micrograms/g dry solids.

Steps (b) and (c) may be performed separately or simultaneously. Alternatively, step (b) may be initiated followed by the continuation of step (b) with step (c), otherwise known as a hybrid hydrolysis and fermentation process.

The lignocellulose-containing material may be pre-treated before being hydrolyzed and fermented. The goal of pre-treatment is to separate and/or release cellulose, hemicellulose and/or lignin and this way improve the rate of enzymatic hydrolysis.

During the pre-treatment, the lignocellulose-containing material may be present in an amount between 10-80 wt. %, *e.g.,* between 20-50 wt. %.

The lignocellulose-containing material may be chemically, mechanically and/or biologically pre-treated before hydrolysis and/or fermentation. Mechanical treatment (often referred to as a physical pre-treatment) may be used alone or in combination with subsequent or simultaneous hydrolysis, especially enzymatic hydrolysis, to promote the separation and/or release of cellulose, hemicellulose and/or lignin.

Preferably, the chemical, mechanical and/or biological pre-treatment is carried out prior to the hydrolysis and/or fermentation. Alternatively, the chemical, mechanical and/or biological pre-treatment is carried out simultaneously with hydrolysis, such as simultaneously with addition of one or more cellulolytic enzymes, or other enzyme activities mentioned below, to release fermentable sugars, such as glucose and/or maltose.

Chemical pre-treatment refers to any chemical treatment which promotes the separation and/or release of cellulose, hemicellulose and/or lignin. Examples of suitable chemical pre-treatment steps include treatment with, for example, dilute acid, lime, alkaline, organic solvent, ammonia, sulphur dioxide, carbon dioxide. Further, wet oxidation and pH-controlled hydrothermolysis are also contemplated chemical pre-treatments.

Preferably, the chemical pre-treatment is acid treatment, more preferably, a continuous dilute and/or mild acid treatment, such as, treatment with sulfuric acid, or another organic acid, such as acetic acid, citric acid, tartaric acid, succinic acid, or a mixture thereof. Other acids may also be used. Mild acid treatment means in the context of the present invention that the treatment pH lies in the range from 1-5, preferably from pH 1-3. In a specific embodiment the acid concentration is in the range from 0.1 to 2.0 wt. % acid, preferably sulphuric acid. The acid may be mixed or contacted with the material to be fermented according to the invention and the mixture may be held at a temperature in the range of 160-220°C, such as 165-195°C, for periods ranging from minutes to seconds, *e.g.,* 1-60 minutes, such as 2-30 minutes or 3-12 minutes. Addition of strong acids, such as sulphuric acid, may be applied to remove hemicellulose. This enhances the digestibility of cellulose.

Cellulose solvent treatment has been shown to convert about 90% of cellulose to glucose. It has also been shown that enzymatic hydrolysis could be greatly enhanced when the lignocellulosic structure is disrupted. Alkaline $H_2O_2$, ozone, organosolv (uses Lewis acids, $FeCl_3$, $(Al)_2SO_4$ in aqueous alcohols), glycerol, dioxane, phenol, or ethylene glycol are among solvents known to disrupt cellulose structure and promote hydrolysis (Mosier et al., 2005, Bioresource Technology 96: 673-686).

The pre-treatment may also be an alkaline chemical pre-treatment with base, *e.g.,* NaOH, $Na_2CO_3$ and/or ammonia or the like. Pre-treatment methods using ammonia are described in, *e.g.,* WO 2006/110891, WO 2006/110899, WO 2006/110900, WO 2006/110901.

Wet oxidation techniques involve use of oxidizing agents, such as: sulphite based oxidizing agents or the like. Examples of solvent pre-treatments include treatment with DMSO (dimethyl sulfoxide) or the like. Chemical pre-treatment is generally carried out for 1 to 60 minutes, such as from 5 to 30 minutes, but may be carried out for shorter or longer periods of time dependent on the material to be pre-treated.

Other examples of suitable pre-treatment methods are described by Schell et al., 2003, Appl. Biochem and Biotechn. 105-108: 69-85, and Mosier et al., 2005, Bioresource Technology 96: 673-686, and U.S. application publication no. 2002/0164730.

Mechanical pre-treatment refers to any mechanical or physical pre-treatment which promotes the separation and/or release of cellulose, hemicellulose and/or lignin from lignocellulose-containing material. For example, mechanical pre-treatment includes various types of milling, irradiation, steaming/steam explosion, and hydrothermolysis.

Mechanical pre-treatment includes comminution (mechanical reduction of the particle size). Comminution includes dry milling, wet milling and vibratory ball milling. Mechanical pre-treatment may involve high pressure and/or high temperature (steam explosion). In an embodiment high pressure means pressure in the range from 300 to 600 psi, preferably 400 to 500 psi, such as around 450 psi. In an embodiment of the invention high temperature means temperatures in the range from about 100 to 300°C, preferably from about 140 to 235°C. In a preferred embodiment mechanical pre-treatment is a batch-process, steam gun hydrolyzer system which uses high pressure and high temperature as defined above. A Sunds Hydrolyzer (available from Sunds Defibrator AB (Sweden) may be used for this.

In an embodiment both chemical and mechanical pre-treatments are carried out involving, for example, both dilute or mild acid pretreatment and high temperature and pressure treatment. The chemical and mechanical pretreatment may be carried out sequentially or simultaneously, as desired.

Accordingly, in a preferred embodiment, the lignocellulose-containing material is subjected to both chemical and mechanical pre-treatment to promote the separation and/or release of cellulose, hemicellulose and/or lignin.

In a preferred embodiment the pre-treatment is carried out as a dilute and/or mild acid steam explosion step. In another preferred embodiment pre-treatment is carried out as an ammonia fiber explosion step (or AFEX pretreatment step).

Biological pre-treatment refers to any biological pre-treatment which promotes the separation and/or release of cellulose, hemicellulose, and/or lignin from the lignocellulose-containing material. Biological pre-treatment techniques can involve applying lignin-solubilizing microorganisms (see, for example, Hsu, T.A., 1996, Pretreatment of biomass, in Handbook on Bioethanol: Production and Utilization, Wyman, C. E., ed., Taylor & Francis, Washington, DC, 179-212; Ghosh and Singh, 1993, Physicochemical and biological treatments for enzymatic/microbial conversion of lignocellulosic biomass, Adv. Appl. Microbiol. 39: 295-333; McMillan, 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Fermentation of lignocellulosic hydrolysates for ethanol production, Enz. Microb. Tech. 18: 312-331; and Vallander and Eriksson, 1990, Production of ethanol from lignocellulosic materials: State of the art, Adv. Biochem. Eng./Biotechnol. 42: 63-95).

The pre-treated material is hydrolyzed, preferably enzymatically, before and/or during fermentation. The pre-treated lignocellulose-containing material may be hydrolyzed in order to break the lignin seal and disrupt the crystalline structure of cellulose. In a preferred embodiment hydrolysis is carried out enzymatically by one or more hydrolases (class E.C. 3 according to Enzyme Nomenclature), preferably one or more carbohydrases including cellulolytic enzymes and hemicellulolytic enzymes, or a combination thereof. In addition, alpha-amylase, glucoamylase, protease, and/or the like may be present during hydrolysis and/or fermentation as the lignocellulose-containing material may include some, *e.g.,* starchy and/or proteinaceous material.

The enzyme(s) used for hydrolysis are capable of directly or indirectly converting carbohydrate polymers into fermentable sugars, such as glucose and/or maltose, which can be fermented into a desired fermentation product, such as ethanol.

In an embodiment, the carbohydrase(s) has(have) cellulolytic (cellobiohydrolase, endoglucanase and beta-glucosidase) and/or hemicellulolytic (*e.g.,* xylanase) activity.

In an embodiment hydrolysis is carried out using a cellulolytic enzyme preparation further comprising one or more polypeptides having cellulolytic enhancing activity. In a preferred embodiment the polypeptide(s) having cellulolytic enhancing activity is(are) of family GH61A origin. Examples of suitable and preferred cellulolytic enzyme preparations and polypeptides having cellulolytic enhancing activity are described in the "Cellulolytic Enzymes" section and "Cellulolytic Enhancing Polypeptides" sections below.

Suitable enzymes are described in the "Enzymes" section below.

Hemicellulose polymers can be broken down by hemicellullolytic enzymes and/or acid hydrolysis to release its five and six carbon sugar components. The six carbon sugars (hexoses), such as arabinose, galactose, glucose, and mannose, can readily be fermented to fermentation products such as acetone, butanol, citric acid, ethanol, fumaric acid, glycerol, etc. by suitable fermenting organisms including yeast.

Yeast is the preferred fermenting organism for ethanol fermentation. Preferred are strains of *Saccharomyces,* especially strains of *Saccharomyces cerevisiae,* preferably strains which are resistant towards high levels of ethanol, *i.e.,* up to, *e.g.,* about 10, 12, 15 or 20 vol. % or more ethanol.

Enzymatic hydrolysis is preferably carried out in a suitable aqueous environment under conditions which can readily be determined by one skilled in the art. In a preferred embodiment hydrolysis is carried out at suitable, preferably optimal, conditions for the enzyme(s) in question.

Suitable process time, temperature and pH conditions can readily be determined by one skilled in the art. Preferably, hydrolysis is carried out at a temperature between 25 and 70°C, *e.g.,* between 40 and 60°C, especially around 50°C. The step is preferably carried out at a pH in the range from 3-8, *e.g.,* pH 4-6. Hydrolysis is typically carried out for between 12 and 96 hours, *e.g.,* between 16 to 72 hours, in particular between 24 and 48 hours.

In an embodiment, the pre-treated lignocellulose-containing material is washed and/or detoxified before or after hydrolysis step (b). This may improve the fermentability of, *e.g.,* dilute-acid hydrolyzed lignocellulose-containing material, such as corn stover. Detoxification may be carried out in any suitable way, *e.g.,* by steam stripping, evaporation, ion exchange, resin or charcoal treatment of the liquid fraction or by washing the pre-treated material.

Fermentation Medium

"Fermentation media" or "fermentation medium" refers to the environment in which fermentation is carried out and which includes the fermentation substrate, that is, the carbohydrate source that is metabolized by the fermenting organism.

The fermentation medium may comprise nutrients and growth stimulator(s) for the fermenting organism(s). Nutrient and growth stimulators are widely used in the art of fermentation and include nitrogen sources, such as ammonia; urea, vitamins and minerals, or combinations thereof. The fermentation medium may also include enzymes such as amylases and/or other carbohydrate source generating enzymes, especially wherein the process of the invention is carried out as a simultaneous saccharification and fermentation process or an RSH process.

Fermenting Organisms

The phrase "fermenting organism" refers to any organism, including bacterial and fungal organisms, suitable for use in a fermentation process and capable of producing a desired fermentation product. The fermenting organism may be a C6 or C5 fermenting organism, or a combination thereof. Both C5 and C6 fermenting organisms are well known in the art.

Suitable fermenting organisms are able to ferment, *i.e.*, convert, fermentable sugars, such as arabinose, fructose, galactose, glucose, maltose, mannose, and/or xylose, directly or indirectly into the desired fermentation product.

Examples of fermenting organisms include fungal organisms such as yeast. Preferred yeast includes strains of *Saccharomyces,* in particular strains of *Saccharomyces cerevisiae* or *Saccharomyces uvarum*; a strain of *Pichia,* preferably *Pichia stipitis* such as *Pichia stipitis* CBS 5773 or *Pichia pastoris*; a strain of *Candida*, in particular a strain of *Candida utilis, Candida arabinofermentans, Candida diddensii, Candida sonorensis, Candida shehatae, Candida tropicalis,* or *Candida boidinii.* Other fermenting organisms include strains of *Hansenula,* in particular *Hansenula polymorpha* or *Hansenula anomala*; *Kluyveromyces,* in particular *Kluyveromyces fragilis* or *Kluyveromyces marxianus;* and *Schizosaccharomyces,* in particular *Schizosaccharomyces pombe.*

Commercially available yeast includes, *e.g.,* RED STAR™ and ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

Preferred bacterial fermenting organisms include strains of *Escherichia,* in particular *Escherichia coli*, strains of *Zymomonas*, in particular *Zymomonas mobilis,* strains of *Zymobacter,* in particular *Zymobactor palmae*, strains of *Klebsiella* in particular *Klebsiella oxytoca,* strains of *Leuconostoc*, in particular *Leuconostoc mesenteroides*, strains of *Clostridium*, in particular *Clostridium butyricum,* strains of *Enterobacter,* in particular *Enterobacter aerogenes* and strains of *Thermoanaerobacter,* in particular *Thermoanaerobacter* BG1L1 (Appl. Microbiol. Biotech. 77: 61-86) and *Thermoanarobacter ethanolicus, Thermoanaerobacter thermosaccharolyticum,* or *Thermoanaerobacter mathranii.* Strains of *Lactobacillus* are also envisioned as are strains of *Corynebacterium glutamicum R, Bacillus thermoglucosidaisus,* and *Geobacillus thermoglucosidasius.*

In an embodiment the fermenting organism is a C6 sugar fermenting organism, such as a strain of, *e.g., Saccharomyces cerevisiae.*

In connection with fermentation of lignocellulose derived materials, C5 sugar fermenting organisms may be used. Most C5 sugar fermenting organisms also ferment C6 sugars. Examples of C5 sugar fermenting organisms include strains of *Pichia,* such as of the species *Pichia stipitis.* C5 sugar fermenting bacteria are also known. Also some *Saccharomyces cerevisae* strains ferment C5 (and C6) sugars. Examples are genetically modified strains of *Saccharomyces spp.* that are capable of fermenting C5 sugars include the ones disclosed in, *e.g.,* Ho et al., 1998, Applied and Environmental Microbiology 64: 1852-1859 and Karhumaa et al., 2006, Microbial Cell Factories 5:18, and Kuyper et al., 2005, FEMS Yeast Research 5: 925-934.

The fermentative performance of certain fermenting organisms may be inhibited by the presence of inhibitors in the fermentation media and thus reduce ethanol production capacity. Compounds in biomass hydrozylates and high concentrations of ethanol are known to inhibit the fermentative capacity of certain yeast cells. Pre-adaptation or adaptation methods may reduce this inhibitory effect. Typically pre-adaptation or adaptation of yeast cells involves sequentially growing yeast cells, prior to fermentation, to increase the fermentative performance of the yeast and increase ethanol production. Methods of yeast pre-adaptation and adaptation are known in the art. Such methods may include, for example, growing the yeast cells in the presence of crude biomass hydrolyzates; growing yeast cells in the presence of inhibitors such as phenolic compounds, furaldehydes and organic acids; growing yeast cells in the presence of non-inhibiting amounts of ethanol; and supplementing the yeast cultures with acetaldehyde. In one embodiment, the fermenting organism is a yeast strain subject to one or more pre-adaptation or adaptation methods prior to fermentation.

According to the invention the fermenting organism is preferably grown under precise conditions at a particular growth rate. When the fermenting organism is introduced into/added to the fermentation medium the inoculated fermenting organism pass through a number of stages. Initially growth does not occur. This period is referred to as the "lag phase" and may be considered a period of adaptation. During the next phase referred to as the "exponential phase" the growth rate gradually increases. After a period of maximum growth, the rate ceases and the fermenting organism enters "stationary phase". After a further period of time the fermenting organism enters the "death phase" where the number of viable cells declines.

In one embodiment the fermenting organism is added to the fermentation medium so that the viable fermenting organism count per mL of fermentation medium is in the range from $10^5$ to $10^{12}$, preferably from $10^7$ to $10^{10}$, especially about $5 \times 10^7$.

Starch-Containing Materials

Any suitable starch-containing material may be used in the present invention. The starting material is generally selected based on the desired fermentation product. Examples of starch-containing materials include whole grains, barley, beans, cassava, corn, milo, peas, rice, rye, sago, sorghum, sweet potatoes, tapioca, wheat, or mixtures thereof or starches derived therefrom, or cereals. Contemplated are also waxy and non-waxy types of corn and barley.

The term "granular starch" means raw uncooked starch, *i.e.,* starch in its natural form found in cereal, tubers or grains. Starch is formed within plant cells as tiny granules insoluble in water. When put in cold water, the starch granules may absorb a small amount of the liquid and swell. At temperatures up to 50°C to 75°C the swelling may be reversible. However, at higher temperatures an irreversible swelling called "gelatinization" begins. Granular starch to be processed may be a highly refined starch quality, preferably at least 90%, at least 95%, at least 97% or at least 99.5% pure or it may be a more crude starch-containing material comprising (*e.g.,* milled) whole grains including non-starch fractions such as germ residues and fibers. The raw material, such as whole grains, may be reduced in particle size, *e.g.,* by milling, in order to open up the structure and allow for further processing. Two processes are preferred according to the invention: wet and dry milling. In dry milling whole kernels are milled and used. Wet milling gives a good separation of germ and meal (starch granules and protein) and is often applied at locations where the starch hydrolyzate is used in production of, *e.g.,* syrups. Dry and wet milling are known in the art of starch processing and are equally contemplated for a process of the invention. In an embodiment the particle size is reduced to between 0.05 to 3.0 mm, preferably 0.1-0.5 mm, or so that at least 30%, preferably at least 50%, more preferably at least 70%, even more preferably at least 90% of the starch-containing material fits through a sieve with a 0.05 to 3.0 mm screen, preferably 0.1-0.5 mm screen.

Lignocellulose-Containing Material (Biomass)

Any suitable lignocellulose-containing material may be used in the methods described herein. Lignocellulose-containing material may be any material containing lignocellulose. In a preferred embodiment the lignocellulose-containing material contains at least 50 wt. %, preferably at least 70 wt. %, more preferably at least 90 wt. % lignocellulose. It is to be understood that the lignocellulose-containing material may also comprise other constituents such as cellulosic material, such as cellulose, hemicellulose and may also comprise constituents such as sugars, such as fermentable sugars and/or un-fermentable sugars.

Lignocellulose-containing material is generally found, for example, in the stems, leaves, hulls, husks, and cobs of plants or leaves, branches, and wood of trees. Lignocellulosic material can also be, but is not limited to, herbaceous material, agricultural residues, forestry residues, municipal solid wastes, waste paper, and pulp and paper mill residues. It is understood herein that lignocellulose-containing material may be in the form of plant cell wall material containing lignin, cellulose, and hemi-cellulose in a mixed matrix.

In an embodiment the lignocellulose-containing material is selected from one or more of corn fiber, rice straw, pine wood, wood chips, poplar, bagasse, and paper and pulp processing waste.

Other examples of suitable lignocellulose-containing material include corn stover, corn cobs, hard wood such as poplar and birch, soft wood, cereal straw such as wheat straw, switch grass, Miscanthus, rice hulls, municipal solid waste (MSW), industrial organic waste, office paper, or mixtures thereof.

In one embodiment the lignocellulose-containing material is corn stover or corn cobs. In another embodiment, the lignocellulose-containing material is corn fiber. In another embodiment, the lignocellulose-containing material is switch grass. In another embodiment, the lignocellulose-containing material is bagasse.

Fermentation Products

The term "fermentation product" means a product produced by a process including a fermentation step using a fermenting organism. Fermentation products include alcohols (*e.g.,* arabinitol, n-butanol, isobutanol, ethanol, glycerol, methanol, ethylene glycol, 1,3-propanediol [propylene glycol], butanediol, glycerin, sorbitol, and xylitol); organic acids (*e.g.,* acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); ketones (*e.g.,* acetone); amino acids (*e.g.,* aspartic acid, glutamic acid, glycine, lysine, serine, and threonine); an alkane (*e.g.,* pentane, hexane, heptane, octane, nonane, decane, undecane, and dodecane), a cycloalkane (*e.g.,* cyclopentane, cyclohexane, cycloheptane, and cyclooctane), an alkene (*e.g.* pentene, hexene, heptene, and octene); isoprene; polyketide; gases (*e.g.,* methane, hydrogen ($H_2$), carbon dioxide ($CO_2$), and carbon monoxide (CO)); antibiotics (*e.g.,* penicillin and tetracycline); enzymes; vitamins (*e.g.,* riboflavin, $B_{12}$, beta-carotene); and hormones. In a preferred embodiment the fermentation product is ethanol, *e.g.,* fuel ethanol; drinking ethanol, *i.e.,* potable neutral spirits; or industrial ethanol or products used in the consumable alcohol industry (*e.g.,* beer and wine), dairy industry (*e.g.,* fermented dairy products), leather industry and tobacco industry. Preferred beer types comprise ales, stouts, porters, lagers, bitters, malt liquors, happoushu, high-alcohol beer, low-alcohol beer, low-calorie beer or light beer. Preferred fermentation processes include alcohol fermentation processes. In an embodiment, the fermentation product is ethanol, which may be used as fuel ethanol or as potable ethanol.

Distillation

Subsequent to fermentation the fermentation product may be separated from the fermentation medium. The slurry may be distilled to extract the desired fermentation product or the desired fermentation product from the fermentation medium by micro or membrane filtration techniques. Alternatively, the fermentation product may be recovered by stripping. Methods for recovering fermentation products are well known in the art. Typically, the fermentation product, *e.g.,* ethanol, with a purity of up to, *e.g.,* about 96 vol. % ethanol is obtained.

Following the completion of the fermentation process, the material remaining is considered the whole stillage. As used herein, the term "whole stillage" includes the material that remains at the end of the fermentation process both before and after recovery of the fermentation product, *e.g.,* ethanol. The fermentation product can optionally be recovered by any method known in the art. In one embodiment, the whole stillage is separated or partitioned into a solid and liquid phase by one or more methods for separating the thin stillage from the wet cake. Such methods include, for example, centrifugation and decanting. The fermentation product can be optionally recovered before or after the whole stillage is separated into a solid and liquid phase.

Thus, in one embodiment, the method of the invention further comprises distillation to obtain the fermentation product, *e.g.,* ethanol. The fermentation and the distillation may be carried out simultaneously and/or separately/sequentially; optionally followed by one or more process steps for further refinement of the fermentation product.

In an embodiment of the invention, the aqueous by-product (whole stillage) from the distillation process is separated into two fractions, *e.g.,* by centrifugation: wet grain (solid phase), and thin stillage (supernatant).

In another embodiment, the method of the invention further comprises separation of the whole stillage produced by distillation into wet grain and thin stillage; and recycling thin stillage to the starch containing material prior to liquefaction.

In one embodiment, the thin stillage is recycled to the milled whole grain slurry.

The wet grain fraction may be dried, typically in a drum dryer. The dried product is referred to as distillers dried grains, and can be used, *e.g.,* as animal feed.

The thin stillage fraction may be evaporated providing two fractions (see Fig. 1):

(i) a condensate fraction of 4-6% DS (mainly of starch, proteins, and cell wall components), and
(ii) a syrup fraction, mainly consisting of limit dextrins and non fermentable sugars, which may be introduced into a dryer together with the wet grains (from the whole stillage separation step) to provide a product referred to as distillers dried grain with solubles, which also can be used as animal feed.

Thin stillage is the term used for the supernatant of the centrifugation of the whole stillage. Typically, the thin stillage contains 4-6% DS (mainly starch and proteins) and has a temperature of about 60-90°C.

In another embodiment, the thin stillage is not recycled, but the condensate stream of evaporated thin stillage is recycled to the slurry containing the milled whole grain to be jet cooked.

Distillers' Dried Grains with Solubles

As explained above, stillage is the product which remains after the mash has been converted to sugar, fermented and distilled into ethanol. Stillage can be separated into two fractions, such as, by centrifugation or screening: (1) wet cake (solid phase) and (2) the thin stillage (supernatant). The solid fraction or distillers' wet grain (DWG) can be pressed to remove excess moisture and then dried to produce distillers' dried grains (DDG). After ethanol has been removed from the liquid fraction, the remaining liquid can be evaporated to concentrate the soluble material into condensed distillers' solubles (DS) or dried and ground to create distillers' dried solubles (DDS). DDS is often mixed with DDG to form distillers' dried grain with solubles (DDGS). DDG, DDGS, and DWG are collectively referred to as distillers' grain(s).

DDGS following an ethanol production process from corn typically contains about 13% oil, 31% protein and 56% carbohydrates and other components. Removal of some of the oil from the DDGS will improve the quality of the DDGS for the feed market as many feed producers prefer less oil and fat in the DDGS to make high quality feed.

Oil Extraction and Dewatering

Methods for dewatering stillage and for extracting oil from a fermentation product are known in the art. These methods include decanting or otherwise separating the whole stillage into wet cake and thin stillage. *See, e.g.,* U.S.

Patent Nos. 6,433,146, 7,601,858, and 7,608,729, and U.S. Application Publication No. 2010/0058649. Furthermore, the thin stillage can be evaporated or condensed into syrup or thick stillage from which the oil can be extracted utilizing centrifugation, filtering, heat, high temperature, increased pressure, or a combination of the same. Another way to extract oil is to lower the pH of the thin stillage or syrup. The use of surfactants to break emulsions also enhances oil extraction. Presses can also be used for dewatering.

The presence of hemicellulase(s) and/or endoglucanase(s) during fermentation in the processes of the invention increases the amount of oil in the thin stillage and further the syrup or thick stillage as compared to the amount of oil in the thin stillage, syrup or thick stillage when a hemicellulase and/or an endoglucanase are not added to the fermentation process.

Recovery

The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol.% can be obtained, which can be used as, for example, fuel ethanol, drinking ethanol, *i.e.*, potable neutral spirits, or industrial ethanol.

Hemicellulases

Hemicellulose can be broken down by hemicellulases and/or acid hydrolysis to release its five and six carbon sugar components.

Any hemicellulase suitable for use in hydrolyzing hemicellulose, preferably into xylose, may be used. Preferred hemicellulases include acetylxylan esterases, endo-arabinases, exo-arabinases, arabinofuranosidases, feruloyl esterase, endo-galactanases, exo-galactanases, glucuronidases, mannases, xylanases, and mixtures of two or more thereof. Preferably, the hemicellulase for use in the present invention is an exo-acting hemicellulase, and more preferably, the hemicellulase is an exo-acting hemicellulase which has the ability to hydrolyze hemicellulose under acidic conditions of below pH 7, preferably pH 3-7.

In one aspect, the hemicellulase(s) comprises a commercial hemicellulolytic enzyme preparation. Examples of commercial hemicellulolytic enzyme preparations suitable for use in the present invention include, for example, SHEARZYME™ (Novozymes A/S), CELLIC™ HTec (Novozymes A/S), CELLIC™ HTec2 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

In an embodiment the hemicellulase is a xylanase. In an embodiment the xylanase may preferably be of microbial origin, such as of fungal origin (*e.g., Aspergillus, Fusarium, Humicola, Meripilus, Trichoderma*) or from a bacterium (*e.g., Bacillus*). In a preferred embodiment the xylanase is derived from a filamentous fungus, preferably derived from a strain of *Aspergillus,* such as *Aspergillus aculeatus*; or a strain of *Humicola,* preferably *Humicola lanuginosa.* Examples of xylanases useful in the methods of the present invention include, but are not limited to, *Aspergillus aculeatus* xylanase (GeneSeqP:AAR63790; WO 94/21785), *Aspergillus fumigatus* xylanases (WO 2006/078256), and *Thielavia terrestris* NRRL 8126 xylanases (WO 2009/079210). The xylanase may preferably be an endo-1,4-beta-xylanase, more preferably an endo-1,4-beta-xylanase of GH 10 or GH 11. Examples of commercial xylanases include SHEARZYME™, BIOFEED WHEAT™, HTec and HTec2 from Novozymes A/S, Denmark.

Examples of beta-xylosidases useful in the methods of the present invention include, but are not limited to, *Trichoderma reesei* beta-xylosidase (UniProtKB/TrEMBL accession number Q92458), *Talaromyces emersonii* (SwissProt accession number Q8X212), and *Neurospora crassa* (SwissProt accession number Q7SOW4).

Examples of acetylxylan esterases useful in the methods of the present invention include, but are not limited to, *Hypocrea jecorina* acetylxylan esterase (WO 2005/001036), *Neurospora crassa* acetylxylan esterase (UniProt accession number q7s259), *Thielavia terrestris* NRRL 8126 acetylxylan esterase (WO 2009/042846), *Chaetomium globosum* acetylxylan esterase (Uniprot accession number Q2GWX4), *Chaetomium gracile* acetylxylan esterase (GeneSeqP accession number AAB82124), *Phaeosphaeria nodorum* acetylxylan esterase (Uniprot accession number Q0UHJ1), and *Humicola insolens* DSM 1800 acetylxylan esterase (WO 2009/073709).

Examples of ferulic acid esterases useful in the methods of the present invention include, but are not limited to, *Humicola insolens* DSM 1800 feruloyl esterase (WO 2009/076122), *Neurospora crassa* feruloyl esterase (UniProt accession number Q9HGR3), and *Neosartorya fischeri* feruloyl esterase (UniProt Accession number A1D9T4).

Examples of arabinofuranosidases useful in the methods of the present invention include, but are not limited to, *Humicola insolens* DSM 1800 arabinofuranosidase (WO 2009/073383) and *Aspergillus niger* arabinofuranosidase (GeneSeqP accession number AAR94170).

Examples of alpha-glucuronidases useful in the methods of the present invention include, but are not limited to, *Aspergillus clavatus* alpha-glucuronidase (UniProt accession number alcc12), *Trichoderma reesei* alpha-glucuronidase (Uniprot accession number Q99024), *Talaromyces emersonii* alpha-glucuronidase (UniProt accession number Q8X211), *Aspergillus niger* alpha-glucuronidase (Uniprot accession number Q96WX9), *Aspergillus terreus* alpha-glucuronidase (SwissProt accession number Q0CJP9), and *Aspergillus fumigatus* alpha-glucuronidase (SwissProt accession number Q4WW45).

Endoglucanases (EG)

The term "endoglucanase" means an endo-1,4-(1,3;1,4)-beta-D-glucan 4-glucanohydrolase (E.C. No. 3.2.1.4), which catalyzes endo-hydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity may be determined using carboxymethyl cellulose (CMC) hydrolysis according to the procedure of Ghose, 1987, Pure and Appl. Chem. 59: 257-268.

In a preferred embodiment endoglucanase(s) may be derived from a strain of *Trichoderma,* such as a strain of *Trichoderma reesei*; a strain of *Humicola,* such as a strain of *Humicola insolens*; or a strain of *Chrysosporium,* such as a strain of *Chrysosporium lucknowense.*

Enzymes for Hydrolysis of a Lignocellulose-Containing Material

Hemicellulases

Any of the hemicellulases described above can be used for hydrolyzing a lignocelluloses-containing material.

The hemicellulase may be added in an amount effective to hydrolyze hemicellulose, such as, in an amount from about 0.001 to 0.5 wt. % of total solids (TS), more preferably from about 0.05 to 0.5 wt. % of TS.

Xylanases may be added in an amount of 0.001-1.0 g/kg DM (dry matter) substrate, preferably in the amount of 0.005-0.5 g/kg DM substrate, and most preferably from 0.05-0.10 g/kg DM substrate.

Cellulolytic Activity

The phrase "cellulolytic activity" as used herein includes enzymes having cellobiohydrolase activity (EC 3.2.1.91), *e.g.,* cellobiohydrolase I and cellobiohydrolase II, endoglucanase activity (EC 3.2.1.4) and/or beta-glucosidase activity (EC 3.2.1.21).

At least three categories of enzymes are important for converting cellulose into fermentable sugars: endoglucanases (EC 3.2.1.4) that cut the cellulose chains at random; cellobiohydrolases (EC 3.2.1.91) which cleave cellobiosyl units from the cellulose chain ends and beta-glucosidases (EC 3.2.1.21) that convert cellobiose and soluble cellodextrins into glucose. Among these three categories of enzymes involved in the biodegradation of cellulose, cellobiohydrolases seems to be the key enzymes for degrading native crystalline cellulose.

The cellulolytic activity may, in a preferred embodiment, be in the form of a preparation of enzymes of fungal origin, such as from a strain of *Trichoderma*, preferably a strain of *Trichoderma reesei*; a strain of *Humicola,* such as a strain of *Humicola insolens*; or a strain of *Chrysosporium*, preferably a strain of *Chrysosporium lucknowense.*

In preferred embodiment the cellulolytic enzyme preparation contains one or more of the following activities: cellulase, hemicellulase, cellulolytic enzyme enhancing activity, beta-glucosidase, endoglucanase, cellubiohydrolase, or xylose isomerase.

In a preferred embodiment the cellulase may be a composition as defined in PCT/US2008/065417. In a preferred embodiment the cellulolytic enzyme preparation comprises a polypeptide having cellulolytic enhancing activity, preferably a family GH61A polypeptide, preferably the one disclosed in WO 2005/074656 (Novozymes). The cellulolytic enzyme preparation may further comprise a beta-glucosidase, such as a beta-glucosidase derived from a strain of *Aspergillus, Penicillium,* or *Trichoderma,* including the fusion protein having beta-glucosidase activity disclosed in WO 2008/057637. In a preferred embodiment the cellulolytic enzyme preparation may also comprises a CBH II enzyme, preferably *Thielavia terrestris* cellobiohydrolase II CEL6A. In another preferred embodiment the cellulolytic enzyme preparation may also comprise cellulolytic enzymes, preferably derived from *Trichoderma reesei* or *Humicola insolens.*

The cellulolytic enzyme preparation may also comprise a polypeptide having cellulolytic enhancing activity (GH61A) disclosed in WO 2005/074656; a beta-glucosidase (fusion protein disclosed in WO 2008/057637); and cellulolytic enzymes derived from *Trichoderma reesei.*

In an embodiment the cellulolytic enzyme is the commercially available product CELLUCLAST® 1.5L, CELLUZYME™, CTEC or CTEC2 available from Novozymes A/S, Denmark or ACCELERASE™ 1000 (from Genencor Inc., USA).

A cellulolytic enzyme may be added during fermentation. The cellulolytic enzyme may be dosed in the range from 0.1-100 FPU per gram total solids (TS), preferably 0.5-50 FPU per gram TS, especially 1-20 FPU per gram TS. In another embodiment at least 0.1 mg cellulolytic enzyme per gram total solids (TS), preferably at least 3 mg cellulolytic enzyme per gram TS, such as between 5 and 10 mg cellulolytic enzyme(s) per gram TS is(are) used for hydrolysis.

Endoglucanases (EG)

Any endoglucanase described above can be used for hydrolyzing a lignocellulosic material. Cellobiohydrolases (CBH)

The term "cellobiohydrolase" means a 1,4-beta-D-glucan cellobiohydrolase (E.C. 3.2.1.91), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain.

Examples of cellobiohydroloses include CBH I and CBH II from *Trichoderma reseei; Humicola insolens* and CBH II from *Thielavia terrestris* cellobiohydrolase (CELL6A).

Cellobiohydrolase activity may be determined according to the procedures described by Lever et al., 1972, Anal. Biochem. 47: 273-279 and by van Tilbeurgh et al., 1982, FEBS Letters 149: 152-156; van Tilbeurgh and Claeyssens, 1985, FEBS Letters 187: 283-288. The Lever *et al.* method is suitable for assessing hydrolysis of cellulose in corn stover and the method of van Tilbeurgh *et al.* is suitable for determining the cellobiohydrolase activity on a fluorescent disaccharide derivative.

Beta-glucosidases

The term "beta-glucosidase" means a beta-D-glucoside glucohydrolase (E.C. 3.2.1.21), which catalyzes the hydrolysis of terminal non-reducing beta-D-glucose residues with the release of beta-D-glucose. For purposes of the present invention, beta-glucosidase activity is determined according to the basic procedure described by Venturi et al., 2002, J. Basic Microbiol. 42: 55-66, except different conditions were employed as described herein. One unit of beta-glucosidase activity is defined as 1.0 micro-mole of p-nitrophenol produced per minute at 50°C, pH 5 from 4 mM p-nitrophenyl-beta-D-glucopyranoside as substrate in 100 mM sodium citrate, 0.01% TWEEN® 20.

In a preferred embodiment the beta-glucosidase is of fungal origin, such as a strain of *Aspergillus, Penicillium,* or *Trichoderma.* In a preferred embodiment the beta-glucosidase is a derived from *Trichoderma reesei*, such as the beta-glucosidase encoded by the *bgl1* gene (see Fig. 1 of EP 562003). In another preferred embodiment the beta-glucosidase is derived from *Aspergillus oryzae* (recombinantly produced in *Aspergillus oryzae* according to WO 2002/095014), *Aspergillus fumigatus* (recombinantly produced in *Aspergillus oryzae* according to Example 22 of WO 2002/095014) or *Aspergillus niger* (1981, J. Appl. 3: 157-163).

Xylose Isomerases

Xylose isomerases (D-xylose ketoisomerase) (E.C. 5.3.1.5) are enzymes that catalyze the reversible isomerization reaction of D-xylose to D-xylulose. Some xylose isomerases also convert the reversible isomerization of D-glucose to D-fructose. Therefore, xylose isomarase is sometimes referred to as "glucose isomerase."

A xylose isomerase used in a method or process of the invention may be any enzyme having xylose isomerase activity and may be derived from any source, preferably bacterial or fungal origin, such as filamentous fungi or yeast. Examples of bacterial xylose isomerases include the ones belonging to *Actinoplanes, Bacillus, Flavobacterium, Streptomyces,* and *Thermotoga,* including *T. neapolitana* (Vieille et al., 1995, Appl. Environ. Microbiol. 61(5): 1867-1875) and *T. maritime.*

Examples of fungal xylose isomerases are derived from *Basidiomycetes.*

A preferred xylose isomerase is derived from a strain of *Candida*, preferably a strain of *Candida boidinii,* especially the *Candida boidinii* xylose isomerase disclosed by, e.g., Vongsuvanlert et al., 1988, Agric. Biol. Chem. 52(7): 1817-1824. The xylose isomerase may be derived from a strain of *Candida boidinii* (*Kloeckera 2201*), deposited as DSM 70034 and ATCC 48180, disclosed in Ogata et al., Agric. Biol. Chem. 33: 1519-1520 or Vongsuvanlert et al., 1988, Agric. Biol. Chem. 52(2): 1519-1520.

In one embodiment the xylose isomerase is derived from a strain of *Streptomyces, e.g.,* derived from a strain of *Streptomyces murinus* (U.S. Patent No. 4,687,742); *S. flavovirens, S. albus, S. achromogenus, S. echinatus, S. wedmorensis* all disclosed in U.S. Patent No. 3,616,221. Other xylose isomerases are disclosed in U.S. Patent No. 3,622,463, U.S. Patent No. 4,351,903, U.S. Patent No. 4,137,126, U.S. Patent No. 3,625,828, HU patent no. 12,415, DE patent no. 2,417,642, JP patent no. 69,28,473, and WO 2004/044129.

The xylose isomerase may be either in immobilized or liquid form. Liquid form is preferred.

An example of a commercially available xylose isomerase is SWEETZYME™ T from Novozymes A/S, Denmark.

The xylose isomerase is added to provide an activity level in the range from 0.01-100 IGIU per gram total solids.

GH61 Polypeptides

In the processes of the present invention, any GH61 polypeptide can be used.

In a first aspect, the GH61 polypeptide comprises the following motifs:

[ILMV]-P-X(4,5)-G-X-Y-[ILMV]-X-R-X-[EQ]-X(4)-[HNQ] and [FW]-[TF]-K-[AIV],

wherein X is any amino acid, X(4,5) is any four or five contiguous amino acids, and X(4) is any four contiguous amino acids.

The GH61 polypeptide comprising the above-noted motifs may further comprise:

H-X(1,2)-G-P-X(3)-[YW]-[AILMV],
[EQ]-X-Y-X(2)-C-X-[EHQN]-[FILV]-X-[ILV], or
H-X(1,2)-G-P-X(3)-[YW]-[AILMV] and [EQ]-X-Y-X(2)-C-X-[EHQN]-[FILV]-X-[ILV],

wherein X is any amino acid, X(1,2) is any one or two contiguous amino acids, X(3) is any three contiguous amino acids, and X(2) is any two contiguous amino acids.

In a preferred aspect, the GH61 polypeptide further comprises H-X(1,2)-G-P-X(3)-[YW]-[AILMV]. In another preferred aspect, the GH61 polypeptide further comprises [EQ]-X-Y-X(2)-C-X-[EHQN]-[FILV]-X-[ILV]. In another preferred aspect, the GH61 polypeptide further comprises H-X(1,2)-G-P-X(3)-[YW]-[AILMV] and [EQ]-X-Y-X(2)-C-X-[EHQN]-[FILV]-X-[ILV].

In a second aspect, the GH61 polypeptide comprises the following motif:

[ILMV]-P-x(4,5)-G-x-Y-[ILMV]-x-R-x-[EQ]-x(3)-A-[HNQ],

wherein x is any amino acid, x(4,5) is any 4 or 5 contiguous amino acids, and x(3) is any 3 contiguous amino acids. In the above motif, the accepted IUPAC single letter amino acid abbreviation is employed.

In a third aspect, the GH61 polypeptide comprises an amino acid sequence that has a degree of identity to the mature polypeptide of SEQ ID NO: 1 (*Thielavia terrestris*), SEQ ID NO: 2 (*Thielavia terrestris*), SEQ ID NO: 3 (*Thielavia terrestris*), SEQ ID NO: 4 (*Thielavia terrestris*), SEQ ID NO: 5 (*Thielavia terrestris*), SEQ ID NO: 6 (*Thielavia terrestris*), SEQ ID NO: 7 (*Thermoascus aurantiacus*), SEQ ID NO: 8 (*Trichoderma reesei*), SEQ ID NO: 9 (*Myceliophthora thermophila*), SEQ ID NO: 10 (*Myceliophthora thermophila*), SEQ ID NO: 11 (*Myceliophthora thermophila),* SEQ ID NO: 12 (*Myceliophthora thermophila*), SEQ ID NO: 13 (*Myceliophthora thermophila),* SEQ ID NO: 14 *(Thermoascus aurantiacus*), SEQ ID NO: 15 (*Aspergillus fumigatus*), SEQ ID NO: 16 (*Penicillium pinophilum*), SEQ ID NO: 17 (*Thermoascus sp.*), SEQ ID NO: 18 (*Penicillium sp.*), SEQ ID NO: 19 (*Thielavia terrestris*), SEQ ID NO: 20 (*Thielavia terrestris*), SEQ ID NO: 21 (*Thielavia terrestris*), SEQ ID NO: 22 (*Thielavia terrestris*), SEQ ID NO: 23 (*Thielavia terrestris*), SEQ ID NO: 24 (*Thielavia terrestris*), SEQ ID NO: 25 (*Thielavia terrestris*), SEQ ID NO: 26 (*Thielavia terrestris*), SEQ ID NO: 27 (*Thielavia terrestris*), SEQ ID NO: 28 (*Thielavia terrestris*), SEQ ID NO: 29 (*Thielavia terrestris*), SEQ ID NO: 30 (*Thermoascus crustaceus*), SEQ ID NO: 31 (*Thermoascus crustaceus*), SEQ ID NO: 32 (*Thermoascus crustaceus*), SEQ ID NO: 33 (*Aurantiporus alborubescens*), or SEQ ID NO: 34 (*Aurantiporus alborubescens*) of at least 60%, e.g., at least 65%, at least 70%, at least 75%, at least 80%, at least 85%, at least 90%, at least 91%, at least 92%, at least 93%, at least 94%, or at least 95%, at least 96%, at least 97%, at least 98%, at least 99%, or at least 100%.

In a sixth aspect, the GH61 polypeptide is an artificial variant comprising a substitution, deletion, and/or insertion of one or more (or several) amino acids of the mature polypeptide of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32; or a homologous sequence thereof.

Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for cellulolytic enhancing activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identities of essential amino acids can also be inferred from analysis of identities with polypeptides that are related to the parent polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The total number of amino acid substitutions, deletions and/or insertions of the mature GH61 polypeptide of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32 is not more than 4, *e.g.,* 1, 2, 3, or 4.

In one aspect, the GH61 polypeptide is used in the presence of a soluble activating divalent metal cation described in WO 2008/151043, e.g., manganese sulfate.

In one aspect, the GH61 polypeptide is used in the presence of a dioxy compound, a bicyclic compound, a heterocyclic compound, a nitrogen-containing compound, or a sulfur-containing compound.

The dioxy compound may include any suitable compound containing two or more oxygen atoms. In some aspects, the dioxy compounds contain a substituted aryl moiety as described herein. The dioxy compounds may comprise one or more (several) hydroxyl and/or hydroxyl derivatives, but also include substituted aryl moieties lacking hydroxyl and hydroxyl derivatives. Non-limiting examples of dioxy compounds include pyrocatechol or catechol; caffeic acid; 3,4-dihydroxybenzoic acid; 4-tert-butyl-5-methoxy-1,2-benzenediol; pyrogallol; gallic acid; methyl-3,4,5-trihydroxybenzoate; 2,3,4-trihydroxybenzophenone; 2,6-dimethoxyphenol; sinapinic acid; 3,5-dihydroxybenzoic acid; 4-chloro-1,2-benzenediol; 4-nitro-1,2-benzenediol; tannic acid; ethyl gallate; methyl glycolate; dihydroxyfumaric acid; 2-butyne-1,4-diol; (croconic acid; 1,3-propanediol; tartaric acid; 2,4-pentanediol; 3-ethyoxy-1,2-propanediol; 2,4,4'-trihydroxybenzophenone; cis-2-butene-1,4-diol; 3,4-dihydroxy-3-cyclobutene-1,2-dione; dihydroxyacetone; acrolein acetal; methyl-4-hydroxybenzoate; 4-hydroxybenzoic acid; and methyl-3,5-dimethoxy-4-hydroxybenzoate; or a salt or solvate thereof.

The bicyclic compound may include any suitable substituted fused ring system as described herein. The compounds may comprise one or more (several) additional rings, and are not limited to a specific number of rings unless otherwise stated. In one aspect, the bicyclic compound is a flavonoid. In another aspect, the bicyclic compound is an optionally subsituted isoflavonoid. In another aspect, the bicyclic compound is an optionally substituted flavylium ion, such as an optionally substituted anthocyanidin or optionally substituted anthocyanin, or derivative thereof. Non-limiting examples of bicyclic compounds include epicatechin; quercetin; myricetin; taxifolin; kaempferol; morin; acacetin; naringenin; isorhamnetin; apigenin; cyanidin; cyanin; kuromanin; (keracyanin; or a salt or solvate thereof.

The heterocyclic compound may be any suitable compound, such as an optionally substituted aromatic or non-aromatic ring comprising a heteroatom, as described herein. In one aspect, the heterocyclic is a compound comprising an optionally substituted heterocycloalkyl moiety or an optionally substituted heteroaryl moiety. In another aspect, the optionally substituted heterocycloalkyl moiety or optionally substituted heteroaryl moiety is an optionally substituted 5-membered heterocycloalkyl or an optionally substituted 5-membered heteroaryl moiety. In another aspect, the optionally substituted heterocycloalkyl or optionally substituted heteroaryl moiety is an optionally substituted moiety selected from pyrazolyl, furanyl, imidazolyl, isoxazolyl, oxadiazolyl, oxazolyl, pyrrolyl, pyridyl, pyrimidyl, pyridazinyl, thiazolyl, triazolyl, thienyl, dihydrothieno-pyrazolyl, thianaphthenyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, benzoisazolyl, dimethylhydantoin, pyrazinyl, tetrahydrofuranyl, pyrrolinyl, pyrrolidinyl, morpholinyl, indolyl, diazepinyl, azepinyl, thiepinyl, piperidinyl, and oxepinyl. In another aspect, the optionally substituted heterocycloalkyl moiety or optionally substituted heteroaryl moiety is an optionally substituted furanyl. Non-limiting examples of heterocyclic compounds include (1,2-dihydroxyethyl)-3,4-dihydroxyfuran-2(5H)-one; 4-hydroxy-5-methyl-3-furanone; 5-hydroxy-2(5H)-furanone; [1,2-dihydroxyethyl]furan-2,3,4(5H)-trione; $\alpha$-hydroxy-$\gamma$-butyrolactone; ribonic $\gamma$-lactone; aldohexuronicaldohexuronic acid $\gamma$-lactone; gluconic acid $\delta$-lactone; 4-hydroxycoumarin; dihydrobenzofuran; 5-(hydroxymethyl)furfural; furoin; 2(5H)-furanone; 5,6-dihydro-2H-pyran-2-one; and 5,6-dihydro-4-hydroxy-6-methyl-2H-pyran-2-one; or a salt or solvate thereof.

The nitrogen-containing compound may be any suitable compound with one or more nitrogen atoms. In one aspect, the nitrogen-containing compound comprises an amine, imine, hydroxylamine, or nitroxide moiety. Non-limiting examples of nitrogen-containing compounds include acetone oxime; violuric acid; pyridine-2-aldoxime; 2-aminophenol; 1,2-benzenediamine; 2,2,6,6-tetramethyl-1-piperidinyloxy; 5,6,7,8-tetrahydrobiopterin; 6,7-dimethyl-5,6,7,8-tetrahydropterine; and maleamic acid; or a salt or solvate thereof.

The quinone compound may be any suitable compound comprising a quinone moiety as described herein. Non-limiting examples of quinone compounds include 1,4-benzoquinone; 1,4-naphthoquinone; 2-hydroxy-1,4-naphthoquinone; 2,3-dimethoxy-5-methyl-1,4-benzoquinone or coenzyme $Q_0$; 2,3,5,6-tetramethyl-1,4-benzoquinone or duroquinone; 1,4-dihydroxyanthraquinone; 3-hydroxy-1-methyl-5,6-indolinedione or adrenochrome; 4-tert-butyl-5-methoxy-1,2-benzoquinone; pyrroloquinoline quinone; or a salt or solvate thereof.

The sulfur-containing compound may be any suitable compound comprising one or more sulfur atoms. In one aspect, the sulfur-containing comprises a moiety selected from thionyl, thioether, sulfinyl, sulfonyl, sulfamide, sulfonamide, sulfonic acid, and sulfonic ester. Non-limiting examples of sulfur-containing compounds include ethanethiol; 2-propanethiol; 2-propene-1-thiol; 2-mercaptoethanesulfonic acid; benzenethiol; benzene-1,2-dithiol; cysteine; methionine; glutathione; cystine; or a salt or solvate thereof.

In an embodiment, the GH61 polypeptide is present in the amount of 2-1000 micrograms/g dry solids (DS), *e.g.,* 5-100, 10-40, or 20-40 micrograms/g DS.

## Enzymes for use in a Conventional Process and Raw Starch Hydrolysis Process

### Alpha-Amylases

Any alpha-amylase may be used to convert a starch-containing material to dextrins. Preferred alpha-amylases are of microbial, such as bacterial or fungal origin. The most suitable alpha-amylase depends on the process conditions but can easily be determined by one skilled in the art.

In one embodiment the preferred alpha-amylase is an acid alpha-amylase, *e.g.,* fungal acid alpha-amylase or bacterial acid alpha-amylase. The phrase "acid alpha-amylase" means an alpha-amylase (E.C. 3.2.1.1) which has optimum activity at a pH in the range of 3 to 7, preferably from 3.5 to 6, or more preferably from 4-5.

### Bacterial Alpha-Amylases

In another preferred embodiment the alpha-amylase is of *Bacillus* origin. The *Bacillus* alpha-amylase may preferably be derived from a strain of *B. amyloliquefaciens, B. licheniformis*, *B. stearothermophilus,* or *B. subtilis*, but may also be derived from other *Bacillus* sp. Specific examples of contemplated alpha-amylases include the *Bacillus licheniformis* alpha-amylase shown in SEQ ID NO: 4 in WO 99/19467, the *Bacillus amyloliquefaciens* alpha-amylase SEQ ID NO: 5 in WO 99/19467 and the *Bacillus stearothermophilus* alpha-amylase shown in SEQ ID NO: 3 in WO 99/19467. In an embodiment of the invention the alpha-amylase may be an enzyme having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NO: 3, 4 or 5, respectively, in WO 99/19467.

The *Bacillus* alpha-amylase may also be a variant and/or hybrid, especially one described in any of WO 96/23873, WO 96/23874, WO 97/41213, WO 99/19467, WO 00/60059, and WO 02/10355. Specifically contemplated alpha-amylase variants are disclosed in U.S. Patent No. 6,093,562, 6,297,038 or 6,187,576 and include *Bacillus stearothermophilus* alpha-amylase (BSG alpha-amylase) variants having a deletion of one or two amino acids in positions R179 to G182, preferably a double deletion disclosed in WO 96/23873 - see *e.g.,* page 20, lines 1-10, preferably corresponding to delta(181-182) compared to the wild-type BSG alpha-amylase amino acid sequence set forth in SEQ ID NO: 3 disclosed in WO 99/19467 or deletion of amino acids R179 and G180 using SEQ ID NO: 3 in WO 99/19467 for numbering. Even more preferred are *Bacillus* alpha-amylases, especially *Bacillus stearothermophilus* alpha-amylase, which have a double deletion corresponding to delta(181-182) and further comprise a N193F substitution (also denoted 1181* + G182* + N193F) compared to the wild-type BSG alpha-amylase amino acid sequence set forth in SEQ ID NO: 3 disclosed in WO 99/19467.

Bacterial Hybrid Alpha-Amylases

A hybrid alpha-amylase specifically contemplated comprises 445 C-terminal amino acid residues of the *Bacillus licheniformis* alpha-amylase (shown in SEQ ID NO: 4 of WO 1999/19467) and the 37 N-terminal amino acid residues of the alpha-amylase derived from *Bacillus amyloliquefaciens* (shown in SEQ ID NO: 5 of WO 99/19467), with one or more, especially all, of the following substitutions: G48A+T49I+G107A+H156Y+A181T+N190F+1201F+A209V+Q264S (using the *Bacillus licheniformis* numbering in SEQ ID NO: 4 of WO 99/19467). Also preferred are variants having one or more of the following mutations (or corresponding mutations in other *Bacillus* alpha-amylase backbones): H154Y, A181T, N190F, A209V and Q264S and/or a deletion of two residues between positions 176 and 179, preferably a deletion of E178 and G179 (using the SEQ ID NO: 5 numbering of WO 99/19467).

Fungal Alpha-Amylases

Fungal alpha-amylases include alpha-amylases derived from a strain of *Aspergillus,* such as, *Aspergillis kawachii, Aspergillus niger*, and *Aspergillus oryzae.*

A preferred acidic fungal alpha-amylase is a Fungamyl-like alpha-amylase which is derived from a strain of *Aspergillus oryzae.* According to the present invention, the phrase "Fungamyl-like alpha-amylase" indicates an alpha-amylase which exhibits a high identity, *i.e.*, more than 70%, more than 75%, more than 80%, more than 85% more than 90%, more than 95%, more than 96%, more than 97%, more than 98%, more than 99% or even 100% identity to the mature part of the amino acid sequence shown in SEQ ID NO: 10 in WO 96/23874.

Another preferred acidic alpha-amylase is derived from a strain of *Aspergillus niger.* In a preferred embodiment the acid fungal alpha-amylase is the one from *A. niger* disclosed as "AMYA_ASPNG" in the Swiss-prot/TeEMBL database under the primary accession no. P56271 and described in WO 89/01969 (Example 3). A commercially available acid fungal alpha-amylase derived from *Aspergillus niger* is SP288 (available from Novozymes A/S, Denmark).

Other contemplated wild-type alpha-amylases include those derived from a strain of the genera *Rhizomucor* and *Meripilus,* preferably a strain of *Rhizomucor pusillus* (WO 2004/055178) or *Meripilus giganteus.*

In a preferred embodiment the alpha-amylase is derived from *Aspergillus kawachii* and disclosed by Kaneko et al., 1996, J. Ferment. Bioeng. 81: 292-298, "Molecular-cloning and determination of the nucleotide-sequence of a gene encoding an acid-stable alpha-amylase from *Aspergillus kawachii*"; and further as EMBL:#AB008370.

The fungal alpha-amylase may also be a wild-type enzyme comprising a starch-binding domain (SBO) and an alpha-amylase catalytic domain *(i.e.,* non-hybrid), or a variant thereof. In an embodiment the wild-type alpha-amylase is derived from a strain of *Aspergillus kawachii.*

Fungal Hybrid Alpha-Amylases

In a preferred embodiment the fungal acid alpha-amylase is a hybrid alpha-amylase. Preferred examples of fungal hybrid alpha-amylases include the ones disclosed in WO 2005/003311 or U.S. Application Publication No. 2005/0054071 (Novozymes) or U.S. application no. 60/638,614 (Novozymes). A hybrid alpha-amylase may comprise an alpha-amylase catalytic domain (CO) and a carbohydrate-binding domain/module (CBM), such as a starch binding domain, and optionally a linker.

Specific examples of contemplated hybrid alpha-amylases include those disclosed in Tables 1 to 5 of the examples in US patent application no. 60/638,614, including Fungamyl variant with catalytic domain JA118 and the *Athelia rolfsii* SBD (SEQ ID NO: 100 in U.S. application no. 60/638,614), *Rhizomucor pusillus* alpha-amylase with *Athelia rolfsii* AMG linker and SBD (SEQ ID NO: 101 in U.S. Application No. 60/638,614), *Rhizomucor pusillus* alpha-amylase with *Aspergillus niger* glucoamylase linker and SBD (which is disclosed in Table 5 as a combination of amino acid sequences SEQ ID NO: 20, SEQ ID NO: 72 and SEQ ID NO: 96 in U.S. application no. 11/316,535) or as V039 in Table 5 in WO 2006/069290, and *Meripilus giganteus* alpha-amylase with *Athelia rolfsii* glucoamylase linker and SBD (SEQ ID NO: 102 in U.S. application no. 60/638,614). Other specifically contemplated hybrid alpha-amylases are any of the ones listed in Tables 3, 4, 5, and 6 in Example 4 in U.S. application no. 11/316,535 and WO 2006/069290.

Other specific examples of contemplated hybrid alpha-amylases include those disclosed in U.S. application publication no. 2005/0054071, including those disclosed in Table 3 on page 15, such as *Aspergillus niger* alpha-amylase with *Aspergillus kawachii* linker and starch binding domain.

Contemplated are also alpha-amylases which exhibit a high identity to any of the above-mentioned alpha-amylases, *i.e.,* more than 70%, more than 75%, more than 80%, more than 85%, more than 90%, more than 95%, more than 96%, more than 97%, more than 98%, more than 99% or even 100% identity to the mature enzyme sequences.

An acid alpha-amylases may according to the invention be added in an amount of 0.1 to 10 AFAU/g DS, preferably 0.10 to 5 AFAU/g DS, especially 0.3 to 2 AFAU/g DS.

Commercial Alpha-Amylase Products

Preferred commercial compositions comprising alpha-amylase include MYCOLASE from DSM, BAN™, TERMAMYL™ SC, FUNGAMYL™, LIQUOZYME™ X, SAN™ SUPER, and SAN™ EXTRA L (Novozymes A/S) and CLARASE™ L-40,000, DEX-LO™, SPEZYME™ FRED, SPEZYME™ AA, SPEZYME™, DELTA AA GC358 and Clearflow AA (Genencor Int.), and the acid fungal alpha-amylase sold under the trade name SP288 (available from Novozymes A/S, Denmark).

Phytases

Any phytase may be used in a process of the present invention. Phytases are enzymes that degrade phytates and/or phytic acid by specifically hydrolyzing the ester link between inositol and phosphorus. Phytase activity is credited with phosphorus and ion availability in many ingredients. In some embodiments, the phytase is capable of liberating at least one inorganic phosphate from an inositol hexaphosphate (*e.g.,* phytic acid). Phytases can be grouped according to their preference for a specific position of the phosphate ester group on the phytate molecule at which hydrolysis is initiated (*e.g.,* 3-phytase (EC 3.1.3.8) or 6-phytase (EC 3.1.3.26)). An example of phytase is myo-inositol-hexakiphosphate-3-phosphohydrolase.

Phytases can be obtained from microorganisms such as fungal and bacterial organisms. For example, the phytase may be obtained from filamentous fungi such as *Aspergillus* (*e.g., A. ficuum, A. fumigatus, A. niger,* and *A. terreus*), *Cladospirum, Mucor* (*e.g., Mucor piriformis*), *Myceliophthora* (*e.g., M. thermophila*), *Penicillium* (*e.g., P. hordei* (ATCC No. 22053)), *P. piceum* (ATCC No. 10519), or *P. brevi-compactum* (ATCC No. 48944), *Talaromyces* (*e.g., T. thermophilus*), *Thermomyces* (WO 99/49740), and *Trichoderma spp.* (*e.g., T. reesei*).

In an embodiment, the phytate-degrading enzyme is obtained from yeast (*e.g., Arxula adeninivorans, Pichia anomala, Schwanniomyces occidentalis*), gram-negative bacteria (*e.g., Escherichia coli, Klebsiella spp., Pseudomonas spp.*), and gram-positive bacteria (*e.g., Bacillus spp.* such as *Bacillus subtilis*).

The phytase also may be obtained from *Citrobacter, Enterbacter,* or *Peniophora.*

In an embodiment, the phytase is derived from *Buttiauxiella spp.* such as *B. agrestis, B. brennerae, B. ferragutiase, B. gaviniae, B. izardii, B. noackiae,* and *B. warmboldiae.* In some embodiments, the phytase is a phytase disclosed in WO 2006/043178 or U.S. application no. 11/714,487.

In one preferred embodiment, the phytase has at least 75%, at least 80%, at least 85%, at least 88%, at least 90%, at least 93%, at least 95%, at least 96%, at least 97%, at least 98% and at least 99% sequence identity to the amino acid sequence set forth in SEQ ID NO: 31 of U.S. Application No. 12/263,886.

Commercially-available phytases are NATUPHOS (BASF), RONOZYME P (Novozymes A/S), PHZYME (Danisco A/S, Diversa) and FINASE (AB Enzymes). The method for determining microbial phytase activity and the definition of a phytase unit is disclosed in Engelen et al., 1994, Journal of AOAC International 77: 760-764. The phytase may be a wild-type phytase, an active variant or active fragment thereof.

Pullulanases

Any pullulanase may be used in a process of the present invention. In an embodiment, the pullulanase is a GH57 pullulanase, *e.g.,* a pullulanase obtained from a strain of *Thermococcus,* including *Thermococcus* sp. AM4, *Thermococcus* sp. HJ21, *Thermococcus barophilus, Thermococcus gammatolerans, Thermococcus hydrothermalis; Thermococcus kodakarensis, Thermococcus litoralis,* and *Thermococcus onnurineus*; or from a strain of *Pyrococcus,* such as *Pyrococcus abyssi* and *Pyrococcus furiosus.*

Carbohydrate-Source Generating Enzymes

The phrase "carbohydrate-source generating enzyme" includes glucoamylase (a glucose generator), beta-amylase and maltogenic amylase (both maltose generators) and also pullulanase and alpha-glucosidase. A carbohydrate-source generating enzyme is capable of producing a carbohydrate that can be used as an energy-source by the fermenting organism(s) in question, for instance, when used in a process for producing a fermentation product such as ethanol. The generated carbohydrate may be converted directly or indirectly to the desired fermentation product, preferably ethanol. According to the invention a mixture of carbohydrate-source generating enzymes may be present. Especially contemplated mixtures are mixtures of at least a glucoamylase and an alpha-amylase, especially an acid amylase, even more preferred an acid fungal alpha-amylase. The ratio between acidic fungal alpha-amylase activity (AFAU) per glucoamylase activity (AGU) (AFAU per AGU) may be at least 0.1, in particular at least 0.16, such as in the range from 0.12 to 0.50 or greater.

Glucoamylases

A glucoamylase may be derived from any suitable source, *e.g.,* derived from a microorganism or a plant. Preferred glucoamylases are of fungal or bacterial origin selected from the group consisting of *Aspergillus* glucoamylases, in particular *A. niger* G1 or G2 glucoamylase (Boel et al., 1984, EMBO J. 3(5): 1097-1102), and variants thereof, such as those disclosed in WO 92/00381, WO 2000/104136 and WO 2001/04273 (from Novozymes, Denmark); the *A. awamori* glucoamylase disclosed in WO 84/02921, *A. oryzae* glucoamylase (Agric. Biol. Chem. 55(4): 941-949 (1991)), and variants or fragments thereof. Other *Aspergillus* glucoamylase variants include variants with enhanced thermal stability: G137A and G139A (Chen et al., 1996, Prot. Eng. 9: 499-505); D257E and D293E/Q (Chen et al., 1995, Prot. Eng. 8: 575-582); N182 (Chen et al., 1994, Biochem. J. 301: 275-281); disulphide bonds, A246C (Fierobe et al., 1996, Biochemistry 35: 8698-8704; and introduction of Pro residues in positions A435 and S436 (Li et al., 1997, Protein Eng. 10: 1199-1204).

Other glucoamylases include *Athelia rolfsii* (previously denoted *Corticium rolfsii*) glucoamylase (see U.S. Patent No. 4,727,026 and Nagasaka et al., 1998, "Purification and properties of the raw-starch-degrading glucoamylases from Corticium rolfsii, Appl. Microbiol. Biotechnol. 50:323-330), *Talaromyces* glucoamylases, in particular derived from *Talaromyces emersonii* (WO 99/28448), *Talaromyces leycettanus* (U.S. Patent No. Re. 32,153), *Talaromyces duponti*, and *Talaromyces thermophilus* (U.S. Patent No. 4,587,215).

Bacterial glucoamylases include glucoamylases from *Clostridium,* in particular *C. thermoamylolyticum* (EP 135,138), and *C. thermohydrosulfuricum* (WO 86/01831) and *Trametes cingulata* disclosed in WO 2006/069289.

Hybrid glucoamylases may also be used in a process of the invention. Examples of hybrid glucoamylases are disclosed in WO 2005/045018. Specific examples include the hybrid glucoamylase disclosed in Tables 1 and 4 of Example 1 of WO 2005/045018.

Contemplated are also glucoamylases which exhibit a high identity to any of above-mentioned glucoamylases, *i.e.,* more than 70%, more than 75%, more than 80%, more than 85%, more than 90%, more than 95%, more than 96%, more than 97%, more than 98%, more than 99% or even 100% identity to the mature enzymes sequences.

Commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U and AMG™ E (from Novozymes A/S); OPTIDEX™ 300 (from Genencor Int.); AMIGASE™ and AMIGASE™ PLUS (from DSM); G-ZYME™ G900, G-ZYME™, GC480, GC148, GC019 and G990 ZR (from Genencor Int.).

Glucoamylases may be added in an amount of 0.02-20 AGU/g DS, preferably 0.1-10 AGU/g DS, especially between 1-5 AGU/g DS, such as 0.5 AGU/g DS.

Beta-amylases

The term "beta-amylase" (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (Fogarty and Kelly, 1979, Progress in Industrial Microbiology 15: 112-115). These beta-amylases are characterized by having optimum temperatures in the range from 40°C to 65°C and optimum pH in the range from 4.5 to 7. A commercially available beta-amylase from barley is NOVOZYM™ WBA from Novozymes A/S, Denmark and SPEZYME™ BBA 1500 from Genencor Int., USA.

Maltogenic amylases

The amylase may also be a maltogenic alpha-amylase. A maltogenic alpha-amylase (glucan 1,4-alpha-maltohydrolase, E.C. 3.2.1.133) is able to hydrolyze amylose and amylopectin to maltose in the alpha-configuration. A maltogenic amylase from *Bacillus stearothermophilus* strain NCIB 11837 is commercially available from Novozymes A/S. Maltogenic alpha-amylases are described in U.S. Patent Nos. 4,598,048, 4,604,355 and 6,162,628.

The maltogenic amylase may be added in an amount of 0.05-5 mg total protein/gram DS or 0.05-5 MANU/g DS.

Proteases

A protease may be added during saccharification, fermentation, simultaneous saccharification and fermentation. The protease may be added to deflocculate the fermenting organism, especially yeast, during fermentation. The protease may be any protease. In a preferred embodiment the protease is an acid protease of microbial origin, preferably of fungal or bacterial origin. An acid fungal protease is preferred, but also other proteases can be used.

Suitable proteases include microbial proteases, such as fungal and bacterial proteases. Preferred proteases are acidic proteases, i.e., proteases characterized by the ability to hydrolyze proteins under acidic conditions below pH 7.

Contemplated acid fungal proteases include fungal proteases derived from *Aspergillus, Candida, Coriolus, Endothia, Enthomophtra, Irpex, Mucor, Penicillium, Rhizopus, Sclerotium* and *Torulopsis.* Especially contemplated are proteases derived from *Aspergillus niger* (see, *e.g.,* Koaze et al., 1964, Agr. Biol. Chem. Japan 28: 216), *Aspergillus saitoi* (see, *e.g.,* Yoshida, 1954, J. Agr. Chem. Soc. Japan 28: 66), *Aspergillus awamori* (Hayashida et al., 1977, Agric. Biol. Chem. 42(5): 927-933), *Aspergillus aculeatus* (WO 95/02044), or *Aspergillus oryzae,* such as the pepA protease; and acidic proteases from *Mucor pusillus* or *Mucor miehei.*

Contemplated are also neutral or alkaline proteases, such as a protease derived from a strain of *Bacillus.* A particular protease contemplated for the invention is derived from *Bacillus amyloliquefaciens* and has the sequence obtainable at Swissprot as Accession No. P06832.

Also contemplated are the proteases having at least 90% identity to the amino acid sequence obtainable at Swissprot as Accession No. P06832 such as at least 92%, at least 95%, at least 96%, at least 97%, at least 98%, or particularly at least 99% identity.

Further contemplated are the proteases having at least 90% identity to amino acid sequence disclosed as SEQ ID NO:1 in WO 2003/048353 such as at 92%, at least 95%, at least 96%, at least 97%, at least 98%, or particularly at least 99% identity.

Also contemplated are papain-like proteases such as proteases within E.C. 3.4.22.* (cysteine protease), such as EC 3.4.22.2 (papain), EC 3.4.22.6 (chymopapain), EC 3.4.22.7 (asclepain), EC 3.4.22.14 (actinidain), EC 3.4.22.15 (cathepsin L), EC 3.4.22.25 (glycyl endopeptidase) and EC 3.4.22.30 (caricain).

In an embodiment the protease is a protease preparation derived from a strain of *Aspergillus,* such as *Aspergillus oryzae.* In another embodiment the protease is derived from a strain of *Rhizomucor,* preferably *Rhizomucormeihei.* In another contemplated embodiment the protease is a protease preparation, preferably a mixture of a proteolytic preparation derived from a strain of *Aspergillus,* such as *Aspergillus oryzae*, and a protease derived from a strain of *Rhizomucor,* preferably *Rhizomucor meihei.*

Aspartic acid proteases are described in, for example, Handbook of Proteolytic Enzymes, Edited by A.J. Barrett, N.D. Rawlings and J.F. Woessner, Academic Press, San Diego, 1998, Chapter 270). Suitable examples of aspartic acid protease include, e.g., those disclosed in Berka et al., 1990, Gene 96: 313; Berka et al., 1993, Gene 125: 195-198; and Gomi et al., 1993, Biosci. Biotech. Biochem. 57: 1095-1100.

Commercially available products include ALCALASE®, ESPERASE™, FLAVOURZYME™, PROMIX™, NEUTRASE®, RENNILASE®, NOVOZYM™ FM 2.0L, and NOVOZYM™ 50006 (available from Novozymes A/S, Denmark) and GC106T™ and SPEZYME™ FAN from Genencor Int., Inc., USA.

The protease may be present in an amount of 0.0001-1 mg enzyme protein per 9 DS, preferably 0.001 to 0.1 mg enzyme protein per 9 DS. Alternatively, the protease may be present in an amount of 0.0001 to 1 LAPU/g DS, preferably 0.001 to 0.1 LAPU/g DS and/or 0.0001 to 1 mAU-RH/g DS, preferably 0.001 to 0.1 mAU-RH/g DS.

The invention described and claimed herein is not to be limited in scope by the specific embodiments herein disclosed, since these embodiments are intended as illustrations of several aspects of the invention. Any equivalent embodiments are intended to be within the scope of this invention as well as combinations of one or more of the embodiments. Various modifications of the invention in addition to those shown and described herein will become apparent to those skilled in the art from the foregoing description. Such modifications are also intended to fall within the scope of the appended claims.

The present invention is further described by the following examples which should not be construed as limiting the scope of the invention.

**MATERIALS & METHODS**

**Methods**

**Measurement of cellulase activity using filter paper assay (FPU assay)**

1. Source of Method

1.1 The method is disclosed in "Measurement of Cellulase Activities" by Adney and Baker, 1996, Laboratory Analytical Procedure, LAP-006, National Renewable Energy Laboratory (NREL), which is based on the IUPAC method for measuring cellulase activity (Ghose, 1987, Measurement of Cellulase Activities, Pure & Appl. Chem. 59: 257-268.

2. Procedure

2.1 The method is carried out as described by Adney and Baker, 1996, *supra,* except for the use of a 96 well plate to read the absorbance values after color development, as described below.
*2.2 Enzyme Assay Tubes:*

2.2.1 A rolled filter paper strip (#1 Whatman; 1 x 6 cm; 50 mg) is added to the bottom of a test tube (13 X 100 mm).
2.2.2 To the tube is added 1.0 ml of 0.05 M sodium citrate buffer (pH 4.80).
2.2.3 The tubes containing filter paper and buffer are incubated 5 minutes at 50°C ($\pm$ 0.1 °C) in a circulating water bath.
2.2.4 Following incubation, 0.5 ml of enzyme dilution in citrate buffer is added to the tube. Enzyme dilutions are designed to produce values slightly above and below the target value of 2.0 mg glucose.
2.2.5 The tube contents are mixed by gently vortexing for 3 seconds.
2.2.6 After vortexing, the tubes are incubated for 60 minutes at 50°C ($\pm$ 0.1 °C) in a circulating water bath.
2.2.7 Immediately following the 60 minute incubation, the tubes are removed from the water bath, and 3.0 ml of dinitrosalicylic acid (DNS) reagent is added to each tube to stop the reaction. The tubes are vortexed 3 seconds to mix.

*2.3 Blank and Controls*

2.3.1 A reagent blank is prepared by adding 1.5 ml of citrate buffer to a test tube.
2.3.2 A substrate control is prepared by placing a rolled filter paper strip into the bottom of a test tube, and adding 1.5 ml of citrate buffer.
2.3.3 Enzyme controls are prepared for each enzyme dilution by mixing 1.0 ml of citrate buffer with 0.5 ml of the appropriate enzyme dilution.
2.3.4 The reagent blank, substrate control, and enzyme controls are assayed in the same manner as the enzyme assay tubes, and done along with them.

*2.4 Glucose Standards*

2.4.1 A 100 ml stock solution of glucose (10.0 mg/ml) is prepared, and 5 ml aliquots are frozen. Prior to use, aliquots are thawed and vortexed to mix.
2.4.2 Dilutions of the stock solution are made in citrate buffer as follows:

G1 = 1.0 ml stock + 0.5 ml buffer = 6.7 mg/ml =3.3 mg/0.5 ml

G2 = 0.75 ml stock + 0.75 ml buffer = 5.0 mg/ml =2.5 mg/0.5 ml

G3 = 0.5 ml stock + 1.0 ml buffer = 3.3 mg/ml =1.7 mg/0.5 ml

$$G4 = 0.2 \text{ ml stock} + 0.8 \text{ ml buffer} = 2.0 \text{ mg/ml} = 1.0 \text{ mg/0.5 ml}$$

2.4.3 Glucose standard tubes are prepared by adding 0.5 ml of each dilution to 1.0 ml of citrate buffer. 2.4.4 The glucose standard tubes are assayed in the same manner as the enzyme assay tubes, and done along with them.

*2.5 Color Development*

2.5.1 Following the 60 minute incubation and addition of DNS, the tubes are all boiled together for 5 minutes in a water bath.

2.5.2 After boiling, they are immediately cooled in an ice/water bath.

2.5.3 When cool, the tubes are briefly vortexed, and the pulp is allowed to settle. Then each tube is diluted by adding 50 microliters from the tube to 200 microliters of double distilled water in a 96-well plate. Each well is mixed, and the absorbance is read at 540 nm.

*2.6 Calculations (examples are given in the NREL document)*

2.6.1 A glucose standard curve is prepared by graphing glucose concentration (mg/0.5 ml) for the four standards (G1-G4) vs. absorbance at 540 nm. This is fitted using a linear regression (Prism Software), and the equation for the line is used to determine the glucose produced for each of the enzyme assay tubes.

2.6.2 A plot of glucose produced (mg/0.5 ml) vs. total enzyme dilution is prepared, with the Y-axis (enzyme dilution) being on a log scale.

2.6.3 A line is drawn between the enzyme dilution that produced just above 2.0 mg glucose and the dilution that produced just below that. From this line, it is determined the enzyme dilution that would have produced exactly 2.0 mg of glucose.

2.6.4 The Filter Paper Units/ml (FPU/ml) are calculated as follows:

$$FPU/mL = 0.37 \text{ enzyme dilution producing } 2.0 \text{ mg glucose.}$$

**EGU assay**

Ten liters of a 0.1 M phosphate pH 6.0 buffer is prepared and is subsequently used to prepare 1 liter of 35 g/l, pH 6.0 carboxymethyl cellulose (CMC, Aqualon 7LF) reagent. An EGU standard is allowed to stand 15 minutes at room temperature before it is weighed out. Approximately 1 g of this standard is weighed out into a 50 ml volumetric flask and filled to the mark with phosphate buffer. This stock solution is then stirred for 15 minutes. Working standards are prepared as described in the table below:

| Standard no. | Dilution ratio | Obtain dilution ratio by dilution as: | | Concentration (EGU/mL) |
| --- | --- | --- | --- | --- |
| | | Enzyme stock solution | Diluent | |
| 1 | 0 | 0 | 1000 | 0.000 |
| 2 | 20 | 50 | 950 | 0.270 |
| 3 | 14 | 71 | 928 | 0.386 |
| 4 | 10 | 100 | 900 | 0.540 |
| 5 | 8 | 125 | 875 | 0.675 |
| 6 | 7 | 142 | 857 | 0.772 |
| 7 | 5 | 200 | 800 | 1.080 |

The sample is prepared for the assay by allowing it to warm to room temperature. The sample is weighed out, diluted with 0.1 M phosphate pH 6.0 buffer and stirred for a minimum of 15 minutes with a maximum stir time of 30 minutes. The enzyme samples are diluted so that the activity of the final dilution is approximately in the middle of the curve. All weights and dilutions are recorded. Samples can be analyzed robotically using a ZYMATE II ROBOT (Zymark, USA), or manually according to the following specifications: A 0.375 ml volume of phosphate buffer is pipetted into a test tube. Next, 0.125 ml of the standard or sample is pipetted into the test tube with the phosphate buffer. It is important that the solutions must be freshly stirred when they are pipetted out. Four ml of CFC substrate is preheated to 25°C, and mixed on Whirl mixer for 25-30 seconds. The sample in the test tube is incubated in a 40°C water bath for 30 minutes. After wiping the test tube it is placed in the viscometer's test tube holder so the vibrating spindle is in the center of the test tube, and left for 20 seconds. The mV signal is read and recorded. The measurements of the enzyme standards are used to plot a standard curve, with enzyme activity as the x-axis and the corresponding mV signal as the y-axis. The standard curve is fitted by regression to a 3$^{rd}$ degree polynomial. The mV values for the different dilutions of the samples are then used to read off the corresponding enzyme activity values from the standard curve. The activity of each sample (EGU/g) is then computed as follows:

$$Sample\ Activity = (C \times Flask \times Dilution) / (Weight)$$

wherein:

C = enzyme activity calculated via line of regression (EGU/mL)
Flask = volume of solution flask (mL)
Dilution = further dilution of the sample

### FXU assay

Two liters of 15% (w/v) BRIJ 35 working solution is prepared from a 30% (w/v) BRIJ 35 (Sigma B4184-1 L) stock solution by diluting with demineralized water. Five liters of sample diluents (50 mM ACES buffer with 225 mg BRIJ/L, pH 6.0) is prepared by weighing out 45.55 g ± 0.005 g ACES and transferring the salt to a 5000 mL graduated flask. Approximately 4550 ml demineralized water is added and mixed with a magnetic stirrer for minimum 20 minutes or until dissolved. 7.50 ml of Brij 35 stock solution 15% (w/v) is pipetted into the solution. The pH is adjusted with sodium hydroxide to 6.00 ± 0.03 at room temperature and the volume is adjusted to 5.0 l.

250 ml of 50 mM XYL-BUF/S: MES buffer, pH 6.0 is prepared by weighing out 2.441 g MES ± 0.005 g and transferred to a 250 mL graduated flask. Approximately 240 ml demineralized water is added and mixed with a magnetic stirrer for minimum 20 minutes or until dissolved. The pH is adjusted with sodium hydroxide to 6.00 ± 0.03 at room temperature and the volume is adjusted to 250 ml. 50 ml of XYL-SUB/S: Substrate wheat arabinoxylan 5.60 g/l in MES buffer 50 mM, pH 6.0 is prepared by weighing out of 0.2800 g ± 0.001 g of wheat arabinoxylan in a 100 ml beaker. The solution is stirred with heat in the beaker to approximately 55°C for 30 minutes or until dissolved. After cooling, the pH is adjusted with sodium hydroxide to 6.00 ± 0.03 at room temperature and the volume is adjusted to 50 ml. 50 ml of 500 mM NaOH is prepared by weighing 4 beads (pellets: fresh, not caked together) of NaOH (*e.g.,* Merck 1.06498) to a 50 ml volumetric flask. The actual weight is noted of the 4 beads (approximately 0.8 g) = W. Multiply W, weight with 50 (= approximately 40 g), and the result is noted. The flask is filled up to 50*W+/- 0.001 g weight with demineralized water and stirred with a magnetic stirrer for 5 minutes. 25 mL of p-hydroxy benzoic acid hydrazide (PAHBAH) PAHBAH/S: PAHBAH/Bismuth/Tartrate solution is prepared by weighing out 0.1380 ± 0.0001 g of Bismuth (III)-acetate (*e.g.,* Aldrich 401587). Then, 0.5000 ± 0.001 g of PAHBAH is weighed out (Sigma H-9882, 152.2 g/mol, 4 kg aliquoted in 2.5 g glass containers). A 1.2500 ± 0,001 g quantity of potassium sodium tartrate, tetrahydrate is weighed out (Merck 1.08087, 282.2 g/mol). The powders are transferred to a 25 ml graduated flask. The flask is filled up to the mark with 500 mM NaOH and wrapped immediately in aluminium foil to protect from light or poured into a brown bottle. The sample is stirred for 15 minutes with a magnetic stirrer.

An FXU standard is prepared by weighing out 0.4520 g (± 0.0005 g) and dissolved in sample diluent in a 250 ml volumetric flask and stirred for 15 minutes. A standard curve is prepared with a 7 point curve with a factor 4.0 between lowest and highest standard point, the recommended volume 1200 microliters. The standard solutions are prepared by further diluting the stock solution with sample diluent on a diluter into the sample cups, according to the table below:

| Standard no. | Example | | Dilution Ratio | Concentration (FXU/mL) |
|---|---|---|---|---|
| | Stock solution (microliters) | Diluent (microliters) | | |
| 1 | 30 | 1170 | 40 | 0.1563 |
| 2 | 40 | 1160 | 30 | 0.2084 |
| 3 | 50 | 1150 | 24 | 0.2605 |

(continued)

| Standard no. | Example | | Dilution Ratio | Concentration (FXU/mL) |
| | Stock solution (microliters) | Diluent (microliters) | | |
|---|---|---|---|---|
| 4 | 60 | 1140 | 20 | 0.3126 |
| 5 | 80 | 1120 | 15 | 0.4168 |
| 6 | 100 | 1100 | 12 | 0.5210 |
| 7 | 120 | 1080 | 10 | 0.6252 |

A sample is weighed out and dissolved in sample diluent. The stock solution is stirred for 15 minutes. The working solution is diluted with the stock solution to lie within the range of the standard curve with sample diluent. The activity assay is carried out using a Konelab 30 analyzer (ThermoFischer Scientific). All reagents, standards and samples are placed in the analyzer. The enzyme activity of the diluted samples is read from the standard curve. The results can be calculated automatically. Calculation of activity of a sample in FXU/g is performed according to the formula:

$$FXU/g = (S \times V \times F) / W$$

wherein

S = Reading from the standard curve in FXU/mL
V = Volume of the measuring flask used in mL
F = Dilution factor for second dilution
W = Weight of sample (g)

Glucoamylase activity

Glucoamylase activity may be measured in AGI units or in Glucoamylase Units (AGU).

Glucoamylase activity (AGI)

Glucoamylase (equivalent to amyloglucosidase) converts starch into glucose. The amount of glucose is determined by the glucose oxidase method for the activity determination. The method described in the section 76-11 Starch-Glucoamylase Method with Subsequent Measurement of Glucose with Glucose Oxidase in "Approved methods of the American Association of Cereal Chemists". Vol. 1-2 MCC, from American Association of Cereal Chemists, 2000; ISBN: 1-891127-12-8.
One glucoamylase unit (AGI) is the quantity of enzyme which will form 1 micromole of glucose per minute under the standard conditions of the method.

| Standard conditions/reaction conditions: | |
|---|---|
| Substrate | Soluble starch, concentration approx. 16 g dry matter/L |
| Buffer | Acetate, approx. 0.04 M, pH=4.3 |
| pH | 4.3 |
| Incubation Temperature | 60°C |
| Reaction Time | 15 minutes |
| Termination of the Reaction | NaOH to a concentration of approximately 0.2 g/L (about pH 9) |
| Enzyme Concentration | 0.15-0.55 AAU/mL |

The starch should be Lintner starch, which is a thin-boiling starch used in the laboratory as a colorimetric indicator. Lintner starch is obtained by dilute hydrochloric acid treatment of native starch so that it retains the ability to color blue with iodine.

Glucoamylase activity (AGU)

The Novo Glucoamylase Unit (AGU) is defined as the amount of enzyme, which hydrolyzes 1 micromole maltose per minute under the standard conditions 37°C, pH 4.3, substrate: 23.2 mM maltose, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | 23.2 mM maltose |
| Buffer: | 0.1 M acetate |
| pH | 4.30 |
| Incubation temperature: | 37°C $\pm$ 1 |
| Reaction time: | 5 minutes |
| Enzyme working range: | 0.5-4.0 AGU/mL |

| Color reaction: | |
|---|---|
| GlucDH: | 430 U/L |
| Mutarotase: | 9 U/L |
| NAD | 0.21 mM |
| Buffer: | 0.12 M phosphate; 0.15 M NaCl |
| pH: | 7.60 $\pm$ 0.05 |
| Incubation temperature: | 37°C $\pm$ 1 |
| Reaction time: | 5 minutes |
| Wavelength: | 340 nm |

Alpha-amylase activity

Alpha-amylase activity (KNU)

The alpha-amylase activity may be determined using potato starch as substrate. This method is based on the degradation of modified potato starch by the enzyme, and the reaction is followed by mixing samples of the starch/enzyme solution with an iodine solution. Initially, a blackish-blue color is formed, but during the degradation of the starch the blue color gets weaker and gradually turns into a reddish-brown, which is compared to a colored glass standard.

One Kilo Novo alpha-amylase Unit (KNU) is defined as the amount of enzyme which, under standard conditions (*i.e.,* at 37°C +/- 0.05; 0.0003 M $Ca^{2+}$; and pH 5.6) dextrinizes 5260 mg starch dry substance Merck Amylum soluble.

Acid alpha-amylase activity

When used according to the present invention the activity of any acid alpha-amylase may be measured in AFAU (Acid Fungal Alpha-amylase Units). Alternatively the activity of an acid alpha-amylase may be measured in AAU (Acid Alpha-amylase Units).

Acid alpha-amylase units (AAU)

The acid alpha-amylase activity can be measured in AAU (Acid Alpha-amylase Units), which is an absolute method. One Acid Amylase Unit (AAU) is the quantity of enzyme converting 1 g of starch (100% of dry matter) per hour under standardized conditions into a product having a transmission at 620 nm after reaction with an iodine solution of known strength equal to the one of a color reference.

Standard conditions/reaction conditions:

| | |
|---|---|
| Substrate: | Soluble starch. Concentration approx. 20 g DS/L |
| Buffer: | Citrate, approx. 0.13 M, pH=4.2 |
| Iodine solution: | 40.176 g potassium iodide + 0.088 g iodine/L |
| City water | 15-20°dH (German degree hardness) |
| pH: | 4.2 |
| Incubation temperature: | 30°C |
| Reaction time: | 11 minutes |
| Wavelength: | 620 nm |
| Enzyme concentration: | 0.13-0.19 AAU/mL |
| Enzyme working range: | 0.13-0.19 AAU/mL |

The starch should be Lintner starch. Further details can be found in EP 0140410.

Acid alpha-amylase activity (AFAU)

Acid alpha-amylase activity may be measured in AFAU (Acid Fungal Alpha-amylase Units), which is determined relative to an enzyme standard. One AFAU is defined as the amount of enzyme which degrades 5.260 mg starch dry matter per hour under the below mentioned standard conditions.

Acid alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions.

$$\text{Starch + Iodine} \quad \xrightarrow[\text{40°C, pH 2.5}]{\text{Alpha-amylase}} \quad \text{Dextrins + Oligosaccharides}$$

Blue/violet                    T = 23 seconds Decoloration

Standard conditions/reaction conditions:

| | |
|---|---|
| Substrate: | Soluble starch, approx. 0.17 g/l |
| Buffer: | Citrate, approx. 0.03 M |
| Iodine (12): | 0.03 g/l |
| $CaCl_2$: | 1.85 mM |
| pH: | $2.50 \pm 0.05$ |
| Incubation temperature: | 40°C |
| Reaction time: | 23 seconds |
| Wavelength: | 590 nm |
| Enzyme concentration: | 0.025 AFAU/ml |
| Enzyme working range: | 0.01-0.04 AFAU/ml |

Xylose/glucose isomerase assay (IGIU)

One IGIU is the amount of enzyme which converts glucose to fructose at an initial rate of 1 micromole per minute at standard analytical conditions.

Standard Conditions:

|  |  |
|---|---|
| Glucose concentration: | 45% w/w |
| pH: | 7.5 |
| Temperature: | 60°C |
| $Mg^{2+}$ concentration: | 99 mg/l (1.0 g/l $MgSO_4$ * 7 $H_2O$) |
| $Ca^{2+}$ concentration | < 2 ppm |
| Activator, $SO_2$ concentration: | 100 ppm (0.18 g/l $Na_2S_2O_5$) |
| Buffer, $Na_2CO_3$, concentration: | 2 mM $Na_2CO_3$ |

Protease activity

Protease assay method (LAPU)

One Leucine Amino Peptidase Unit (LAPU) is the amount of enzyme which decomposes 1 micromole substrate per minute at the following conditions: 26 mM of L-leucine-p-nitroanilide as substrate, 0.1 M Tris buffer (pH 8.0), 40°C, 10 minute reaction time.

Protease assay method AU(RH)

The proteolytic activity may be determined with denatured hemoglobin as substrate. In the Anson-Hemoglobin method for the determination of proteolytic activity denatured hemoglobin is digested, and the undigested hemoglobin is precipitated with trichloroacetic acid (TCA). The amount of TCA soluble product is determined with phenol reagent, which gives a blue color with tyrosine and tryptophan.

One Anson Unit (AU(RH)) is defined as the amount of enzyme which under standard conditions (*i.e.,* 25°C, pH 5.5 and 10 minute reaction time) digests hemoglobin at an initial rate such that there is liberated per minute an amount of TCA soluble product which gives the same color with phenol reagent as one milliequivalent of tyrosine.

Determination of maltogenic amylase activity (MANU)

One MANU (Maltogenic Amylase Novo Unit) may be defined as the amount of enzyme required to release one micromole of maltose per minute at a concentration of 10 mg of maltotriose (Sigma M 8378) substrate per ml of 0.1 M citrate buffer, pH 5.0 at 37°C for 30 minutes.

Materials

Spirizyme Excel - a *T. cingulata* and *T. emersonii* glucoamylase blend (Novozymes A/S, Denmark)) CELLIC™ CTec - a *Trichoderma reesei* cellulase composition supplemented with GH61 polypeptide and beta-glucosidase fusion protein (Novozymes A/S, Denmark).

CELLIC™ HTec - xylanase (Novozymes A/S, Denmark).

CELLIC™ CTec2 - a blend of (a) a *Trichoderma reesei* cellulase composition supplemented with GH61 polypeptide and beta-glucosidase and (b) CELLIC™ HTec2 (Novozymes A/S, Denmark). CELLIC™ HTec2 - xylanase (Novozymes A/S, Denmark).

Glucoamylase A (AMG A): Glucoamylase derived from *Trametes cingulata* disclosed in SEQ ID NO: 2 in WO 2006/069289 (Novozymes A/S).

Alpha-Amylase A (AAA): a hybrid alpha-amylase consisting of *Rhizomucor pusillus* alpha-amylase with *Aspergillus niger* glucoamylase linker and SBD disclosed as V039 in Table 5 in WO 2006/069290 (Novozymes A/S).

Celluclast 1.5L - a *Trichoderma reesei* cellulase composition with xylanase activity.

Ultraflo - *Humicola insolens* beta-glucanase with ferulic acid esterase and xylanase activity. Shearzyme 2x - *Aspergillus aculeatus* xylanase.

Shearzyme Plus - *Aspergillus aculeatus* xylanase.

## EXAMPLES

### Example 1

Unfermented corn mash was used to prepare fermentation samples. Urea and penicillin were added to a final concentration of 1000 ppm and 3 mg/L respectively. The pH of the mash was adjusted to pH 5. Approximately 620 g mash was placed into LR-2.ST 1 L reactor vessels (IKA, Wilmington, NC) which were held in a 32°C water bath with constant mixing throughout the 54 hour fermentation.

Spirizyme Excel was dosed at a final concentration of 0.06% (% w/w of corn). CELLIC™ CTec, CELLIC™ HTec and *Aspergillus fumigatus* GH61 polypeptide were each equally dosed as 33.3 micrograms enzyme protein/g DS. Fermentations were initiated by adding 0.68 g of dry Ethanol Red yeast (RedStar Yeast Co., Milwaukee, WI) directly to the reactor.

After 54 hours, the fermentations were stopped by adding 10 microliters of 40% (v/v) $H_2SO_4$/g mash and stored in the glass reactors overnight at 4°C. Each treatment was then transferred to three 250 ml Nalgene bottles, capped, and centrifuged for 15 minutes at 3500 x g at approximately 30°C in a F14BCl-6x250cy rotor in an Avanti J-E centrifuge (Beckman Coulter, Brea, CA). The supernatants from each treatment were recombined, allowed to cool to room temperature, and the oil was recovered according to the following method:

Supernatant from each treatment was combined in a 250 ml separatory funnel. The Nalgene bottles in which the samples were centrifuged were rinsed with a small amount of hexane (<5 ml), followed by extraction of the supernatant (three extractions, 40 ml hexane per extraction). The organic phase was collected in a pre-weighed round-bottomed flask, transferred to 50 ml tubes for centrifugation at 11,000 x g for 15 minutes then transferred back to the round-bottomed flask. The organic layer was distilled off under vacuum using a R-205 rotary evaporator (Buchi, Flawil, Switzerland) at 240 rpm with a water bath set to 85°C and the round-bottomed flask was reweighed to obtain the final oil weight.

In order to accurately compare oil yields, oil recovered was reported as a mass percent of the initial mash used in the treatment. For SSF treatments, the recorded initial mash weight was used for the calculation. To calculate the initial mash used for each sample, the amount of mash aliquoted into the large-scale fermentation was measured. The oil yield was calculated based on a percent weight basis of oil obtained to starting corn mash used (Table 1).

Table 1: Oil Yield

| Treatment | % oil (w/w) |
|---|---|
| Spirizyme Excel | 0.60% |
| Spirizyme Excel, CELLIC™ CTec, CELLIC™ HTec and GH61 polypeptide | 0.86% |

The results show that the addition of a blend of CELLIC™ CTec, CELLIC™ HTec, and GH61 polypeptide significantly increased the amount of corn oil extracted by 43% compared to the control sample.

### Example 2

Unfermented industrially produced mash was blended in a blender for 5 minutes. This served to increase available surface area for enzymatic activity and possibly to further disrupt the oil-containing germ. A 100 g sample of this corn mash was aliquoted into plastic 250 mL Erlenmeyer® flasks with filter-equipped caps. Saccharification was performed using AAA + AMG A (10.5 AGU/FAU-F) at a dose of 0.04 wt. %. CELLIC™ CTec2 was dosed at 12 mg product/g DS.

Rehydrated RED STAR™ yeast (Fermentis/Lesaffre, USA) was used in the fermentation, 3.3 g of yeast suspended in 50 mL of 32°C tap water for 30 minutes before pitching 2 mL of this culture into each mash. SSF was allowed to proceed for 72 hours in a reciprocal/orbital shaking air incubator at 32°C. After 72 hours, the fermentations were stopped by adding 10 microliters of 40% (v/v) $H_2SO_4$/g mash.

In order to measure the amount of oil captured in the aqueous and oil phases of the samples, a hexanes-based extractive assay was developed. The fermented samples were decanted into two 50 mL conical centrifuge tubes. The wall of the fermentation flask was rinsed well with deionized water and decanted into a third 50 ml centrifuge tube. All tubes were centrifuged in an Allegra 6R benchtop centrifuge with rotor GH-3.8 (Beckmen Coulter, Brea, CA) at 1462 x g for 10 minutes. The supernatant was decanted into a fresh 250 mL volumetric flask. Using a small amount (<5 ml) of hexane, residual oil was rinsed from the centrifuge tube walls and added to the volumetric flask. Deionized water was added to resuspend the pellet in the two centrifuge tubes and combined into one tube and centrifuged again. The supernatant was poured into the volumetric flask and the sides of the tubes were rinsed again using a small amount of hexane. The supernatant in the flask was poured into a 250 ml separatory funnel, followed by 2 g of NaCl, and inverted several times to mix. Then, 15 ml of hexane were added to the funnel and mixed thoroughly with venting, and then allowed to separate in order to decant the aqueous layer into a beaker. The hexane layer was transferred into a suitable vessel. The aqueous layer was added back to the funnel and washed with hexane and collected a second time. A third wash was performed in the funnel with hexane leaving the aqueous layer out and only washing the walls of the funnel. All of the hexane layers were poured back into the funnel for a fourth time and rinsed with 100 ml of hot tap water. After the phases separated, the aqueous phase was decanted and the hexane layer was transferred into a preweighed round bottom flask. The organic layer was distilled off under vacuum at 240 rpm with a water bath set to 85°C using an R-205 rotary evaporator (Buchi, Flawil, Switzerland) and the round bottom flask was re-weighed to obtain the final oil weight.

In order to accurately compare oil yields, oil recovered was reported as a mass percent of the initial mash used in the treatment. For SSF treatments, the recorded initial mash weight was used for the calculation. To calculate the initial mash used for each sample, the amount of mash aliquoted into the large-scale fermentation was measured. The oil yield was calculated based on a percent weight basis of oil obtained to starting corn mash used (Table 2).

Table 2: Oil Yield

| Treatment | % oil (w/w) |
|---|---|
| 1. AAA + AMG A (10.5 AGU/FAU-F) | 0.48% |
| 2. AAA + AMG A (10.5 AGU/FAU-F) + CELLIC™ CTec2 | 1.48% |

The results show that the addition of CELLIC™ CTec2 significantly increased the amount of corn oil extracted by 208% compared to the control sample.

**Example 3**

Industrially produced corn mash was used for all assay validation experiments. A 5 gallon container containing the liquefied mash was thawed, partitioned into smaller quantities (1 liter screw top Nalgene bottles), and refrozen. One liter of this mash was thawed for approximately 4 hours prior to starting this study. The mash was supplemented with 3 ppm penicillin and 1000 ppm urea using solutions of 1 g/l penicillin and 200 g/l urea, and adjusted to pH 5.0 with 40% (v/v) $H_2SO_4$. The dry solids content of the mash was measured on a halogen lamp moisture balance model HB43-S (Mettler-Toledo, Toledo, Ohio) with a resulting value of approximately 30% DS. Approximately 25 g of the industrial mash were added to 50 ml conical centrifuge tubes with screw caps. A total of 24 fermentations were run.

Spirizyme Ultra was dosed at a final concentration of 0.056% (% w/w of corn). Celluclast 1.5L, Shearzyme 2x, CELLIC™ Ctec2, CELLIC™ Htec2, and Shearzyme Plus were each dosed as 10 or 100 micrograms enzyme protein/g DS.

Water was dosed into each 25 g sample such that the total added volume of enzyme and water was normalized to each sample in the set. A total of 5.5 g of Fermentis Ethanol Red yeast was rehydrated in 100 mL of 35°C tap water by incubating at 32°C for 30 minutes. Each 25 g sample was then dosed with a 250 microliter aliquot of the rehydrated yeast solution. Tubes were weighed at time 0 and then fermented for 70 hours at 32°C at 150 rpm in an incubated orbital shaker model SHKE4000-5 (ThermoScientific, Waltham, MA). After 52 or 70 hours, tubes were weighed and ethanol concentration was calculated using the equation:

$$\text{g ethanol/g DS} = \frac{\text{g CO}_2 \text{ weight loss} \times \dfrac{1\,\text{mol CO}_2}{44.0098\,\text{g CO}_2} \times \dfrac{1\,\text{mol ethanol}}{1\,\text{mol CO}_2} \times \dfrac{46.094\,\text{g ethanol}}{1\,\text{mol ethanol}}}{\text{g mash in tube} \quad \times \quad \%DS}$$

*Distillation*

A Büchi Multivapor evaporation system was used for all distillations (Buchi, Flawil, Switzerland). The unit distilled 12 samples at a time. The parameters tested for wet cake dewatering processing are shown in Table 3 and the parameters for corn oil extraction processing are shown in Table 4:

**Table 3. Distillation parameters for wet cake dewatering**

| Time | 54 min |
|---|---|
| **Temperature** | 75°C |

(continued)

| Time | 54 min |
|------|--------|
| Vacuum | 275 - 185 mBar (35 min) |
| | 185 - 175 mBar (15 min) |
| | 175 mBar (14 min) |
| RPM | 8 |

**Table 4. Distillation parameters for corn oil extraction**

| Time | 45 min |
|------|--------|
| Temperature | 75°C |
| Vacuum | 450 - 200 mBar |
| RPM | 8 |

*Backend Processing (Wet cake dewatering)*

After distillation, the whole stillage samples were centrifuged at 1,400 x g for 10 minutes in an Avanti J-E series centrifuge with a JS-5.3 rotor (Beckman Coulter, Brea, CA). Following centrifugation the samples were immediately decanted into pre-weighed 15 mL conical tubes. Weights of the resulting wet cake and thin stillage were recorded. Wet cake was scraped out of the tube and placed in pre-weighed drying pans and incubated at 55°C overnight and transferred to 105°C for 2 hours in incubation ovens (VWR Models: 1545 and 1370FM, respectively). Weights were recorded before and after each incubation. These values were used to calculate dry solids values using the following formula:

$$\%DS = \frac{g\ \mathrm{d}rysample}{g\ \mathrm{wet\ sample}} * 100\%$$

*Backend Processing (Corn Oil Extraction)*

Hexane was added to each whole stillage sample at a dose of 0.125 mL hexane/g starting material. Each tube was sealed to prevent sample leakage, and mixed thoroughly. Tubes were centrifuged at 3,000 x g for 10 minutes in an Avanti JE Series centrifuge with a JS-5.3 rotor. After centrifugation, the oil/hexane layer (supernatant) was removed using a positive displacement pipette, transferred to a pre-weighed 5 ml flip-top tube, and reweighed. The density of the sample was measured using a research analytical density meter model DDM 2911 (Rudolph Research Analytical, Hackettstown, NJ). The density of the supernatant was then inserted into the standard curve equation to find the percent oil in the supernatant. From this value the total percent oil in the starting material was derived.

The results are shown below in Tables 5-13.

Table 5

| *T. aurantiacus* GH61A | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | n/a | -2.0 | 1.5 | 2.5 | 1.8 |
| | 100 | n/a | -1.4 | 1.1 | 1.7 | 2.7 |

Table 6

| CELLIC™ CTec | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.0578 EGU | -2.4 | 1.5 | 2.5 | 1.8 |
| | 100 | 0.578 EGU | 1.9 | -1.4 | 1.3 | 7.6 |

Table 7

| Celluclast 1.5L | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.04 EGU | -3.9 | 2.7 | 1.3 | -1.2 |
| | 100 | 0.4 EGU | -4.8 | 3.5 | 3.8 | 2.3 |

Table 8

| Shearzyme 2X | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.3 FXU | -1.6 | 1.2 | 0.3 | 1.6 |
| | 100 | 3.0 FXU | -2.4 | 1.4 | 0.04 | 8.9 |

Table 9

| CELLIC™ HTec2 | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.011 EGU, 0.2173 FXU | -2.5 | 1.9 | 0.5 | -1.8 |
| | 100 | 0.11 EGU, 2.173 FXU | -2.3 | 1.7 | 0.6 | 3.0 |

Table 10

| Shearzyme Plus | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.0365 EGU, 0.026 FXU | -5.5 | 4.0 | 4.3 | 4.7 |
| | 100 | 0.365 EGU, 0.26 FXU | -11.9 | 8.5 | 5.6 | 18.7 |

Table 11

| CELLIC™ CTec2 | Dose (micrograms EP/g DS) | Dose (Activity/g DS) | % Change relative to control | | | |
|---|---|---|---|---|---|---|
| | | | Wet cake | Thin Stillage | Dry Solids | Oil |
| | 10 | 0.076 EGU, 0.0147 FXU | -3.1 | 2.2 | 2.3 | 9.7 |
| | 100 | 0.76 EGU, 0.147 FXU | -9.6 | 6.6 | 4.8 | 7.1 |

The results indicate that the combination of cellulase, xylanase, and GH61 activities increased the efficacy of oil extraction and dewatering in a synergistic fashion. Furthermore, the greater the ratio between cellulase activity and xylanase activity when these activities were combined, led to a greater enhancement of the corn oil extraction and dewatering effects. On their own, none of the activities (cellulase, xylanase and GH61) contributed significantly to enhancing oil extraction or dewatering, unless they were used in uneconomically high doses.

**Example 4**

Industrially produced corn mash was used for all samples. A 5 gallon container containing the liquefied mash was thawed, partitioned into smaller quantities (1 liter screw top Nalgene bottles) and refrozen. One liter of this mash was thawed for about 4 hours prior to starting this study. The mash was supplemented with 3 ppm penicillin and 1000 ppm urea using solutions of 1 g/l penicillin and 200 g/L urea, respectively, and adjusted to pH 5.0 with 40% $H_2SO_4$. The dry solids content of the mash was measured on a Mettler-Toledo moisture balance (HB43-S halogen), with a resulting value of 30.00% DS. Approximately 5 g of the industrial mash was added to 15 ml conical centrifuge tubes with flip tops (NUNC #362694). A total of 126 fermentations were run. Enzymes were dosed according to the product specifications in the following table and the volume of stock solution to add to fermentation was determined using the equation:

$$\text{Enz. dose (ml)} = \frac{\text{Final enz. dose (mg EP/g DS)} \times \text{Mash weight (g)} \times \text{Solid content (\%DS)}}{\text{Conc. enzyme (mg EP/ml)}}$$

| | Enzyme | Dose | Units |
|---|---|---|---|
| **1** | Spirizyme Ultra (Control) | 0.500 | AGU/g DS |
| **2** | CELLIC™ CTEC2 | 100.000 | micrograms EP/g DS |
| **3** | CELLIC™ CTEC2 | 1000.000 | micrograms EP/g DS |
| **4** | Celluclast 1.5L | 100.000 | micrograms EP/g DS |
| **5** | Shearzyme Plus | 10.000 | micrograms EP/g |
| | | | DS |
| **6** | Shearzyme Plus | 100.000 | micrograms EP/g DS |

Water was dosed into each sample such that the total added volume of enzyme and water was approximately 70 microliters/25 g sample. Rehydrated yeast (5.5 g Fermentis Ethanol Red yeast in 100 mL 35°C tap water incubated at 32°C for 30 minutes) was dosed at 50 microliters of yeast solution in each 5 g sample. Tubes were weighed at time 0 and then fermented for 52 or 70 hours at 32°C, monitoring weight loss throughout fermentation and back calculating to determine the amount of ethanol produced according to the following equation:

$$\text{g ethanol/g DS} = \frac{\text{g CO}_2 \text{ weight loss} \times \dfrac{1 \text{ mol CO}_2}{44.0098 \text{ g CO}_2} \times \dfrac{1 \text{ mol ethanol}}{1 \text{ mol CO}_2} \times \dfrac{46.094 \text{ g ethanol}}{1 \text{ mol ethanol}}}{\text{g mash in tube} \times \%DS}$$

*HPLC analysis*

At the end of the fermentation time point, 50 microliters of 40% $H_2SO_4$ was added to each sample and vortexed to ensure mixing. Samples were centrifuged at 1570 x g (3000 rpm) for 10 minutes in a Beckman Coulture benchtop centrifuge (Allegra 6R) with rotor GH3.8 and then filtered into HPLC vials through 0.45 micron syringe filters (Whatman) prior to submission for HPLC analysis.

**HPLC system:** Agilent's 1100/1200 series with Chem station software

    Degasser
    Quadratic Pump
    Auto-Sampler

Column Compartment /w Heater
Refractive Index Detector (RI)

**Column:** Bio-Rad HPX- 87H Ion Exclusion Column 300 mm x 7.8 mm parts# 125-0140
Bio-Rad guard cartridge cation H parts# 125-0129, Holder parts# 125-0131
**Method:** 0.005 M $H_2SO_4$ mobile phase

Flow rate of 0.6 ml/min
Column temperature - 65°C
RI detector temperature - 55°C

The method quantifies several analytes using calibration standards for dextrins (DP4+), maltotriose, maltose, glucose, fructose, acetic acid, lactic acid, glycerol and ethanol. A 4 point calibration including the origin is used.

The results of ethanol yield and increases over the control at 52 and 70 hours of fermentation are summarized in Table 12.

**Table 12 - Comparison of ethanol yields after 52 and 70 hour fermentation time**

| Sample | 52 hour fermentation | | 70 hour fermentation | |
|---|---|---|---|---|
| | Ethanol (g/L) | % Increase | Ethanol (g/L) | % Increase |
| Control | 133.30 | | 135.29 | |
| CELLIC™ CTec2 (100 micrograms EP/g DS) | 134.64 | 1.01% | 136.88 | 1.17% |
| CELLIC™ CTec2 (1 mg EP/g DS) | 137.26 | 2.97% | 138.74 | 2.55% |
| Celluclast 1.5L (100 micrograms EP/g DS) | 134.42 | 0.84% | 135.89 | 0.44% |
| Shearzyme Plus (10 micrograms EP/g DS) | 133.90 | 0.45% | 135.51 | 0.17% |
| Shearzyme Plus (100 micrograms EP/g DS) | 133.50 | 0.15% | 135.76 | 0.35% |

The results show that after 52 hours, there are two samples that have significant increases in ethanol, CELLIC™ CTec2 dosed at 100 micrograms EP/g DS (1.01% increase) and at 1 mg EP/g DS (2.97% increase). After 70 hours, the same results were observed with CELLIC™ CTec2 the only sample having a significant increase over the control when dosed at 100 micrograms EP/g DS (1.17% increase) and 1 mg EP/g DS (2.55% increase). No other sample indicated a significant increase over the control even though these too are cellulose and hemicellulose degrading enzymes (cellulases and xylanase). This suggests that a combination of cellulase and xylanase degrading enzymes is needed in order to enhance ethanol yield in the ratio contained in CELLIC™ CTec2. This optimal ratio is supported by the fact that Shearzyme Plus also contains cellulases and xylanase degrading enzymes but at a much higher amount of xylanase the results in yield are not as high as CELLIC™ CTec2.

**Example 5**

A portion of corn was milled dry using a Fitzpatrick Hammer mill with a screen no. 7. The dry matter content of the milled product was measured gravimetrically to 88.0% w/w. A mash containing 30% dry matter was made using 681.8 g of milled corn and 1318.2 g of city water. 0.11 g of Termamyl SC-L (alpha-amylase) was added in a dosage that was equivalent to 20 KNU/kg milled corn. The mash was heated from 55°C to 90°C during 60 minutes under stirring and then cooled to 32°C and divided in 240 g portions for the simultaneous saccharification and fermentation trials that were carried in 500 ml blue cap flask with an air lock placed in the rubber stopper.

After the liquefaction of the corn mash using Termamyl SC-L the enzyme classes and products shown in the Table 13 were used:

Table 13

| Enzyme classes | Enzyme products and dosages (% of corn dry matter) |
|---|---|
| Glucoamylase | Spirizyme Plus 0.1 % |

(continued)

| Enzyme classes | Enzyme products and dosages (% of corn dry matter) |
|---|---|
| Glucoamylase + cellulase complex with xylanase activity (family GH3) | Spirizyme Plus 0.1% + Celluclast 1.5L 1% |
| Glucoamylase + cellulase complex with xylanase activity (family GH3) + ferulic acid esterase and beta-glucanase with xylanase activity (family GH3) | Spirizyme Plus 0.1 % + Celluclast 1.5L 0.5% + Ultraflo L 0.5% |
| Glucoamylase + ferulic acid esterase and beta-glucanase with xylanase activity (family GH3) | Spirizyme Plus 0.1% + Ultraflo L 1% |

After 30 minutes conditioning with the enzymes and dosages provided in Table 13, 0.20 g of fresh Baker's yeast (Danish Distillers A/S) was added to each flask. The simultaneous saccharification and fermentation trials were carried out in a thermostatted chamber with shaking table at 32°C at 150 rpm in an Innova® 44 shaker. The fermentation time was 67 hours and at the end of the fermentation period all samples were analyzed for content of ethanol and residual soluble sugars on the HPLC.

For fat/oil determination all the samples were centrifuged and the supernatants and the sediment were separated and freeze dried in aluminum trays. The oil content in the sediment was analyzed on an ASE 300 accelerated solvent extractor (Dionex Corporation).

The oil from the supernatant was diluted in hexane and filtrated. The hexane was evaporated in a fume cupboard and the oil was weighed.

The results from the ethanol determinations after 67 hours were in the interval of 11-13% w/w resulting in alcohol yields in the interval of 27-34 kg ethanol/100 kg dry matter based on milled corn. These yields were in the normal range for laboratory trials.

Table 14 shows the actual oil recovery data and average yields of oil to the supernatant (stillage) is calculated as well as the extra yields obtained. The yield calculation was based on an oil content of 10.50% w/w found in the milled corn product.

Table 14 - Oil Recovery

| Enzyme products and dosages (% of corn dry matter) | Mass of DDG, g | Mass of oil in DDG, g | Yield of oil (to supernatant),% | Avergage Yield of oil (to supernatant),% | % Extra yield |
|---|---|---|---|---|---|
| Spirizyme Plus 0.1% | 30.86 | 3.76 | 52.8 | 59.5 | 0 |
| | 31.35 | 2.68 | 66.3 | | |
| Spirizyme Plus 0.1% + Celluclast 1.5 L1% | 27.98 | 2.04 | 74.3 | 74.0 | 24 |
| | 28.59 | 2.10 | 73.6 | | |
| Spirizyme Plus 0.1% + Celluclast 1.5 L 0.5% + Ultraflo L 0.5% | 24.50 | 1.90 | 76.1 | 80.1 | 35 |
| | 23.54 | 1.26 | 84.2 | | |
| Spirizyme Plus 0.1% + Ultraflo L 1% | 22.84 | 2.45 | 69.2 | 69.1 | 16 |
| | 23.40 | 2.46 | 69.1 | | |

The results show that there is a synergistic effect of cellulases, xylanases and ferulic esterase resulted in an extra yield of 35% oil. Almost the double mass of oil could be extracted by applying the synergistically working cell wall degrading enzymes (Celluclast + Ultraflo). It is believed that the higher the concentration of ethanol in the thin stillage, the greater the amount of oil (including phospholipids) that can be extracted.

No special pre-treatment of the corn meal other than milling was made.

A fine milling is recommended, either dry or wet at a suitable step in the process to futher improve the yields.

**Claims**

1.  A process of dewatering a whole stillage, comprising

    (a) converting a starch-containing material into dextrins with an alpha-amylase;

(b) saccharifying the dextrins using a carbohydrate source generating enzyme to form a sugar;

(c) fermenting the sugar in a fermentation medium into a fermentation product using a fermenting organism, wherein the fermentation medium comprises a hemicellulase(s), an endoglucanase(s), and a GH61 polypeptide(s);

(d) distilling the fermentation product to form the whole stillage; and

(e) separating the whole stillage into thin stillage and wet cake, whereby more liquid phase is transferred to the thin stillage.

2. The process of claim 1, wherein the hemicellulase(s) is selected from the group consisting of acetylxylan esterase, arabinofuranosidase, feruloyl esterase, glucuronidase, xylanase, and xylosidase.

3. The process of claims 2, wherein the hemicellulase(s) is a xylanase.

4. The process of any of claims 1-3, wherein the fermentation medium further comprises a cellobiohydrolase(s).

5. The process of any of claims 1-4, wherein the fermentation medium further comprises a beta-glucosidase(s).

6. The process of any of claims 1-5, wherein the fermentation medium further comprises a protease(s).

7. The process of any of claims 1-6, wherein the fermentation medium further comprises a beta-glucanase(s).

8. The process of any of claims 1-7, wherein the fermentation medium further comprises a ferulic acid esterase(s).

**Patentansprüche**

1. Verfahren zum Entwässrung eine Rohschlempe, umfassend

(a) Umwandeln eines Stärke enthaltenden Materials in Dextrine mit einer alpha-Amylase;

(b) Verzuckern der Dextrine unter Verwendung eines eine Kohlenhydratquelle erzeugenden Enzyms, um einen Zucker zu bilden;

(c) Fermentieren des Zuckers in einem Fermentationsmedium zu einem Fermentationsprodukt unter Verwendung eines Fermentationsorganismus, wobei das Fermentationsmedium eine Hemicellulase(n), eine Endoglucanase(n), und ein GH61-Polypeptid umfasst;

(d) Destillieren des Fermentationsprodukts, um die Rohschlempe zu bilden;

(e) Trennen der Rohschlempe in Dünnschlempe und Dickschlempe; wobei mehr flüssige Phase auf die Dünnschlempe übertragen wird.

2. Verfahren nach Anspruch 1, wobei die Hemicellulase(n) ausgewählt ist aus der Gruppe bestehend aus Acetylxylanesterase, Arabinofuranosidase, Feruloylesterase, Glucuronidase, Xylanase und Xylosidase.

3. Verfahren nach Anspruch 2, wobei die Hemicellulase(n) eine Xylanase ist.

4. Verfahren Anspruch 3, wobei das Fermentationsmedium weiterhin eine Cellobiohydrolase(n) umfasst.

5. Verfahren nach einem beliebigen der Ansprüche 1-4, wobei das Fermentationsmedium weiterhin eine beta-Glucosidase(n) umfasst.

6. Verfahren nach einem beliebigen der Ansprüche 1-5, wobei das Fermentationsmedium weiterhin eine Protease(n) umfasst.

7. Verfahren nach einem beliebigen der Ansprüche 1-6, wobei das Fermentationsmedium weiterhin eine beta-Glucanase(n) umfasst.

8. Verfahren nach einem beliebigen der Ansprüche 1-7, wobei das Fermentationsmedium weiterhin eine Ferulasäureesterase(n) umfasst.

**Revendications**

1. Procédé de déshydratation du résidu de distillation entier comprenant

   (a) la conversion d'un matériau contenant de l'amidon en dextrines avec une alpha-amylase ;
   (b) la saccharification des dextrines en utilisant une enzyme générant une source de glucide pour former un sucre ;
   (c) la fermentation du sucre dans un milieu de fermentation en un produit de fermentation en utilisant un organisme de fermentation, dans laquelle le milieu de fermentation comprend une ou plusieurs hémicellulases, une ou plusieurs endoglucanases, et un polypeptide GH61 ;
   (d) la distillation du produit de fermentation pour former de résidu de distillation entier ;
   (e) la séparation du résidu de distillation entier en résidu de distillation soluble et gâteau humide, et par lequel plus de phase liquide est transféré au résidu de distillation soluble.

2. Procédé selon la revendication 1, dans lequel la ou les hémicellulases sont choisies dans le groupe constitué d'acétylxylane estérase, d'arabinofuranosidase, de féruloyl-estérase, de glucuronidase, de xylanase, et de xylosidase.

3. Procédé selon la revendication 2, dans lequel la ou les hémicellulases sont une xylanase.

4. Procédé selon la revendication 3, dans lequel le milieu de fermentation comprend en outre une ou plusieurs cellobiohydrolases.

5. Procédé selon l'une quelconque des revendications 1 à 4, dans lequel le milieu de fermentation comprend en outre une ou plusieurs bêta-glucosidases.

6. Procédé selon l'une quelconque des revendications 1 à 5, dans lequel le milieu de fermentation comprend en outre une ou plusieurs protéases.

7. Procédé selon l'une quelconque des revendications 1 à 6, dans lequel le milieu de fermentation comprend en outre une ou plusieurs bêta-glucanases.

8. Procédé selon l'une quelconque des revendications 1 à 7, dans lequel le milieu de fermentation comprend en outre une ou plusieurs acide férulique estérases.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6433146 B
- US 7601858 B
- US 7608729 B
- US 20100058649 A
- WO 2007056321 A1
- WO 02095014 A
- WO 2002095014 A
- WO 2006110891 A
- WO 2006110899 A
- WO 2006110900 A
- WO 2006110901 A
- US 20020164730 A
- WO 9421785 A
- WO 2006078256 A
- WO 2009079210 A
- WO 2005001036 A
- WO 2009042846 A
- WO 2009073709 A
- WO 2009076122 A
- WO 2009073383 A
- US 2008065417 W
- WO 2005074656 A
- WO 2008057637 A
- EP 562003 A
- US 4687742 A
- US 3616221 A
- US 3622463 A
- US 4351903 A
- US 4137126 A
- US 3625828 A
- HU 12415
- DE 2417642
- JP 6928473 B
- WO 2004044129 A
- WO 9517413 A
- WO 9522625 A
- US 5223409 A
- WO 9206204 A
- WO 2008151043 A
- WO 9919467 A
- WO 9623873 A
- WO 9623874 A
- WO 9741213 A
- WO 0060059 A
- WO 0210355 A
- US 6093562 A
- US 6297038 B
- US 6187576 B
- WO 199919467 A
- WO 8901969 A
- WO 2004055178 A
- WO 2005003311 A
- US 20050054071 A
- US 638614 P
- US 60638614 B
- US 316535 A
- WO 2006069290 A
- WO 9949740 A
- WO 2006043178 A
- US 714487 A
- US 263886 A
- WO 9200381 A
- WO 2000104136 A
- WO 200104273 A
- WO 8402921 A
- US 4727026 A
- WO 9928448 A
- US RE32153 E
- US 4587215 A
- EP 135138 A
- WO 8601831 A
- WO 2006069289 A
- WO 2005045018 A
- US 4598048 A
- US 4604355 A
- US 6162628 A
- WO 9502044 A
- WO 2003048353 A
- EP 0140410 A
- US 61318787 B

**Non-patent literature cited in the description**

- **WANG.** *Lipid Technology,* 2008, vol. 20 (9), 203-207
- **WANG et al.** *J. Agric. Food Chem.,* 2009, vol. 57, 2302-2307
- **DE VRIES.** *J. Bacteriol.,* 1998, vol. 180, 243-249

- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **ZHANG et al.** *Biotechnology Advances,* 2006, vol. 24, 452-481
- **GHOSE.** *Pure and Appl. Chem.,* 1987, vol. 59, 257-268
- **HENRISSAT.** A classification of glycosyl hydrolases based on amino-acid sequence similarities. *Biochem. J.,* 1991, vol. 280, 309-316
- **HENRISSAT ; BAIROCH.** Updating the sequence-based classification of glycosyl hydrolases. *Biochem. J.,* 1996, vol. 316, 695-696
- **SHALLOM ; SHOHAM.** Microbial hemicellulases. *Current Opinion In Microbiology,* 2003, vol. 6 (3), 219-228
- **GHOSE ; BISARIA.** *Pure & Appl. Chem.,* 1987, vol. 59, 1739-1752
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **EBRINGEROVA et al.** *Adv. Polym. Sci.,* 2005, vol. 186, 1-67
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN et al.** The beta-D-xylosidase of Trichoderma reesei is a multifunctional beta-D-xylan xylohydrolase. *Biochemical Journal,* 1997, vol. 321, 375-381
- **BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- **GORINSTEIN ; LII.** *Starch/Starke,* 1992, vol. 44 (12), 461-466
- **MOSIER et al.** *Bioresource Technology,* 2005, vol. 96, 673-686
- **SCHELL et al.** *Appl. Biochem and Biotechn.,* 2003, vol. 105-108, 69-85
- Pretreatment of biomass. **HSU, T.A.** Handbook on Bioethanol: Production and Utilization. Taylor & Francis, 1996, 179-212
- **GHOSH ; SINGH.** Physicochemical and biological treatments for enzymatic/microbial conversion of lignocellulosic biomass. *Adv. Appl. Microbiol.,* 1993, vol. 39, 295-333
- Pretreating lignocellulosic biomass: a review, in Enzymatic Conversion of Biomass for Fuels Production. **MCMILLAN.** ACS Symposium Series. American Chemical Society, 1994, vol. 566
- Ethanol production from renewable resources. **GONG, C. S. ; CAO, N. J. ; DU, J. ; TSAO, G. T.** Advances in Biochemical Engineering/Biotechnology. Springer-Verlag, 1999, vol. 65, 207-241
- **OLSSON ; HAHN-HAGERDAL.** Fermentation of lignocellulosic hydrolysates for ethanol production. *Enz. Microb. Tech.,* 1996, vol. 18, 312-331
- **VALLANDER ; ERIKSSON.** Production of ethanol from lignocellulosic materials: State of the art. *Adv. Biochem. Eng./Biotechnol.,* 1990, vol. 42, 63-95
- *Appl. Microbiol. Biotech.,* vol. 77, 61-86
- **HO et al.** *Applied and Environmental Microbiology,* 1998, vol. 64, 1852-1859
- **KARHUMAA et al.** *Microbial Cell Factories,* 2006, vol. 5, 18
- **KUYPER et al.** *FEMS Yeast Research,* 2005, vol. 5, 925-934
- **VENTURI et al.** *J. Basic Microbiol.,* 2002, vol. 42, 55-66
- *J. Appl.,* 1981, vol. 3, 157-163
- **VIEILLE et al.** *Appl. Environ. Microbiol.,* 1995, vol. 61 (5), 1867-1875

- **VONGSUVANLERT et al.** *Agric. Biol. Chem.,* 1988, vol. 52 (7), 1817-1824
- **OGATA et al.** *Agric. Biol. Chem.,* vol. 33, 1519-1520
- **VONGSUVANLERT et al.** *Agric. Biol. Chem.,* 1988, vol. 52 (2), 1519-1520
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **KANEKO et al.** *J. Ferment. Bioeng.,* 1996, vol. 81, 292-298
- **ENGELEN et al.** *Journal of AOAC International,* 1994, vol. 77, 760-764
- **BOEL et al.** *EMBO J.,* 1984, vol. 3 (5), 1097-1102
- *Agric. Biol. Chem.,* 1991, vol. 55 (4), 941-949
- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA et al.** Purification and properties of the raw-starch-degrading glucoamylases from Corticium rolfsii. *Appl. Microbiol. Biotechnol.,* 1998, vol. 50, 323-330
- **FOGARTY ; KELLY.** *Progress in Industrial Microbiology,* 1979, vol. 15, 112-115
- **KOAZE et al.** *Agr. Biol. Chem. Japan,* 1964, vol. 28, 216
- **YOSHIDA.** *J. Agr. Chem. Soc. Japan,* 1954, vol. 28, 66
- **HAYASHIDA et al.** *Agric. Biol. Chem.,* 1977, vol. 42 (5), 927-933
- Handbook of Proteolytic Enzymes. Academic Press, 1998
- **BERKA et al.** *Gene,* 1990, vol. 96, 313
- **BERKA et al.** *Gene,* 1993, vol. 125, 195-198
- **GOMI et al.** *Biosci. Biotech. Biochem.,* 1993, vol. 57, 1095-1100
- Measurement of Cellulase Activities. **ADNEY ; BAKER.** Laboratory Analytical Procedure, LAP-006. National Renewable Energy Laboratory (NREL), 1996
- **GHOSE.** Measurement of Cellulase Activities. *Pure & Appl. Chem.,* 1987, vol. 59, 257-268
- Approved methods of the American Association of Cereal Chemists. American Association of Cereal Chemists, 2000, vol. 1-2